# EUROPEAN PATENT APPLICATION

(11) **EP 4 834 372 A1**
(43) Date of publication of application: **04.11.2026**
(21) Application number: 24911468.7
(22) Date of filing: 27.12.2024
(51) Int. Cl.: A61K 47/68, C07K 16/28, A61P 35/00

(54) **IMMUNOCONJUGATE AND USE THEREOF**

(30) Priority: 29.12.2023 CN 202311862752
(71) Applicant: Hangzhou Sumgen Biotech Co., Ltd., Hangzhou, Zhejiang 310056 (CN); Sumgen Mab (Beijing) Biotech Co., Ltd., Beijing 100176 (CN)
(72) Inventor: DING, Xiaoran, Hangzhou, Zhejiang 310056 (CN); MIAO, Shiwei, Hangzhou, Zhejiang 310056 (CN); TAO, Jun, Hangzhou, Zhejiang 310056 (CN); CUI, Hang, Hangzhou, Zhejiang 310056 (CN); LV, Ming, Hangzhou, Zhejiang 310056 (CN)
(74) Representative: Murgitroyd & Company
(86) International application number: PCT/CN2024/143177
(87) International publication number: WO 2025/140548

(57) **Abstract**

The present application relates to an immunoconjugate, comprising an antigen-binding protein targeting LILRB4, an optional linking moiety, and a drug molecule. The present application further provides a pharmaceutical composition comprising the immunoconjugate, a pharmaceutical combination comprising the immunoconjugate, a preparation method for the immunoconjugate, and use of the immunoconjugate in the preparation of a drug.

## Description

### Technical Field

The present application relates to the field of biomedicine, and specifically to an immunoconjugate comprising an antigen-binding protein targeting LILRB4 and a drug molecule.

### Background Art

Leukocyte immunoglobulin-like receptor B4 (LILRB4) is a member of the leukocyte Ig-like receptors (LILRs). Under physiological conditions, LILRB4 plays a very important role in the function of the immune system through its expression on various immune cells such as T cells and plasma cells. Under pathological conditions, LILRB4 influences the progression of various diseases, such as tumor and leukemia transformation and infiltration, through multiple signaling pathways. Differential expression of LILRB4 occurs in various immune system diseases, such as Kawasaki disease, systemic lupus erythematosus (SLE), and sepsis. Recent studies have shown that LILRB4 also plays a role in psychiatric disorders. Given the critical role of LILRB4 in the immune system and its differential expression in various diseases, it has become a potential therapeutic target for multiple diseases.

Based on the therapeutic potential of LILRB4, there is a need to develop more diverse drug formats targeting LILRB4 to fully exploit the characteristics of this target and improve therapeutic efficacy.

### Summary of the Invention

The present application provides an immunoconjugate comprising an antigen-binding protein targeting LILRB4 and a drug molecule. The immunoconjugate of the present invention can specifically kill target cells that highly express LILRB4, thereby achieving targeted therapy. In the present application, said antigen-binding protein targeting LILRB4 exhibits one or more of the following properties: 1) capable of specifically recognizing leukocyte immunoglobulin-like receptor B4 (LILRB4); 2) capable of binding to LILRB4 protein with a K_{D} value of 2×10⁻⁷ M or lower; 3) capable of blocking the activity of LILRB4/APOE binding; 4) capable of stimulating activation of CD8+ T cells; 5) capable of inhibiting migration of human monocytic leukemia cell THP-1; 6) capable of inhibiting tumor cell growth and/or metastasis; 7) capable of blocking the binding of LILRB4 to Fibronectin.

In certain embodiments, said immunoconjugate has the following general formula:

Ab-(Q)ₙ (Formula 1)

wherein Ab represents the antigen-binding protein targeting LILRB4, Q represents the moiety linked to Ab and containing the drug molecule, n is an integer from 1 to 10, and each Q may be the same or different.

In certain embodiments, said LILRB4 is human LILRB4.

In certain embodiments, said antigen-binding protein targeting LILRB4 comprises at least one CDR from the antibody heavy chain variable region VH, wherein said VH comprises an amino acid sequence selected from any one of SEQ ID NO: 81, SEQ ID NO: 33, and SEQ ID NO: 22.

In certain embodiments, said antigen-binding protein targeting LILRB4 comprises HCDR1, HCDR2, and HCDR3, wherein said HCDR1 comprises an amino acid sequence as shown in SEQ ID NO: 69 (X₁X₂WMX₃, wherein X₁ is D or S; X₂ is A or Y; X₃ is D or H), said HCDR2 comprises an amino acid sequence as shown in SEQ ID NO: 2 or SEQ ID NO: 28, and said HCDR3 comprises an amino acid sequence as shown in SEQ ID NO: 83 or SEQ ID NO: 29.

In certain embodiments, said antigen-binding protein targeting LILRB4 comprises HCDR1, HCDR2, and HCDR3, wherein said HCDR1 comprises an amino acid sequence as shown in SEQ ID NO: 1 or SEQ ID NO: 27, said HCDR2 comprises an amino acid sequence as shown in SEQ ID NO: 2 or SEQ ID NO: 28, and said HCDR3 comprises an amino acid sequence as shown in any one of SEQ ID NO: 3, SEQ ID NO: 20, and SEQ ID NO: 29.

In certain embodiments, said antigen-binding protein targeting LILRB4 comprises HCDR1, HCDR2, and HCDR3, wherein said HCDR1, HCDR2, and HCDR3 comprise amino acid sequences selected from the group consisting of:
(1) said HCDR1 comprises an amino acid sequence as shown in SEQ ID NO: 1, said HCDR2 comprises an amino acid sequence as shown in SEQ ID NO: 2, and said HCDR3 comprises an amino acid sequence as shown in SEQ ID NO: 3;
(2) said HCDR1 comprises an amino acid sequence as shown in SEQ ID NO: 1, said HCDR2 comprises an amino acid sequence as shown in SEQ ID NO: 2, and said HCDR3 comprises an amino acid sequence as shown in SEQ ID NO: 20; and
(3) said HCDR1 comprises an amino acid sequence as shown in SEQ ID NO: 27, said HCDR2 comprises an amino acid sequence as shown in SEQ ID NO: 28, and said HCDR3 comprises an amino acid sequence as shown in SEQ ID NO: 29.

In certain embodiments, said antigen-binding protein targeting LILRB4 comprises a VH, wherein said VH comprises an amino acid sequence as shown in any one of SEQ ID NO: 81, SEQ ID NO: 33, and SEQ ID NO: 22.

In certain embodiments, said antigen-binding protein targeting LILRB4 comprises a VH, wherein said VH comprises an amino acid sequence as shown in any one of SEQ ID NO: 8, SEQ ID NO: 19, SEQ ID NO: 22, SEQ ID NO: 33, SEQ ID NO: 45, SEQ ID NO: 55, and SEQ ID NO: 58.

In certain embodiments, said antigen-binding protein targeting LILRB4 comprises at least one CDR from the antibody light chain variable region VL, wherein said VL comprises an amino acid sequence as shown in SEQ ID NO: 82 or SEQ ID NO: 40.

In certain embodiments, the antigen-binding protein targeting LILRB4 comprises LCDR1, LCDR2, and LCDR3, wherein the LCDR1 comprises an amino acid sequence as shown in SEQ ID NO: 70 or SEQ ID NO: 34, the LCDR2 comprises an amino acid sequence as shown in SEQ ID NO: 71, and the LCDR3 comprises an amino acid sequence as shown in SEQ ID NO: 72 (QX₁X₂X₃X₄X₅PX₆T, wherein X₁ is H or Q; X₂ is G or S; X₃ is D, N, or W; X₄ is E or T; X₅ is I or L; X₆ is P or R).

In certain embodiments, said antigen-binding protein targeting LILRB4 comprises LCDR1, LCDR2, and LCDR3, wherein said LCDR1 comprises an amino acid sequence as shown in any one of SEQ ID NO: 9, SEQ ID NO: 23, and SEQ ID NO: 34, said LCDR2 comprises an amino acid sequence as shown in SEQ ID NO: 10 or SEQ ID NO: 35, and said LCDR3 comprises an amino acid sequence as shown in any one of SEQ ID NO: 11, SEQ ID NO: 24, and SEQ ID NO: 36.

In certain embodiments, said antigen-binding protein targeting LILRB4 comprises LCDR1, LCDR2, and LCDR3, wherein said LCDR1, LCDR2, and LCDR3 comprise amino acid sequences selected from the group consisting of:
(1) said LCDR1 comprises an amino acid sequence as shown in SEQ ID NO: 9, said LCDR2 comprises an amino acid sequence as shown in SEQ ID NO: 10, and said LCDR3 comprises an amino acid sequence as shown in SEQ ID NO: 11;
(2) said LCDR1 comprises an amino acid sequence as shown in SEQ ID NO: 23, said LCDR2 comprises an amino acid sequence as shown in SEQ ID NO: 10, and said LCDR3 comprises an amino acid sequence as shown in SEQ ID NO: 24; and
(3) said LCDR1 comprises an amino acid sequence as shown in SEQ ID NO: 34, said LCDR2 comprises an amino acid sequence as shown in SEQ ID NO: 35, and said LCDR3 comprises an amino acid sequence as shown in SEQ ID NO: 36.

In certain embodiments, said antigen-binding protein targeting LILRB4 comprises a VL, wherein said VL comprises an amino acid sequence as shown in SEQ ID NO: 82 or SEQ ID NO: 40.

In certain embodiments, said antigen-binding protein targeting LILRB4 comprises a VL, wherein said VL comprises an amino acid sequence as shown in any one of SEQ ID NO: 16, SEQ ID NO: 26, SEQ ID NO: 40, SEQ ID NO: 51, and SEQ ID NO: 61.

In certain embodiments, said antigen-binding protein targeting LILRB4 comprises HCDR1, HCDR2, HCDR3, LCDR1, LCDR2, and LCDR3, wherein said HCDR1, HCDR2, HCDR3, LCDR1, LCDR2, and LCDR3 comprise amino acid sequences selected from the group consisting of:
(1) said HCDR1 comprises an amino acid sequence as shown in SEQ ID NO: 1, said HCDR2 comprises an amino acid sequence as shown in SEQ ID NO: 2, said HCDR3 comprises an amino acid sequence as shown in SEQ ID NO: 3, said LCDR1 comprises an amino acid sequence as shown in SEQ ID NO: 9, said LCDR2 comprises an amino acid sequence as shown in SEQ ID NO: 10, and said LCDR3 comprises an amino acid sequence as shown in SEQ ID NO: 11;
(2) said HCDR1 comprises an amino acid sequence as shown in SEQ ID NO: 1, said HCDR2 comprises an amino acid sequence as shown in SEQ ID NO: 2, said HCDR3 comprises an amino acid sequence as shown in SEQ ID NO: 20, said LCDR1 comprises an amino acid sequence as shown in SEQ ID NO: 23, said LCDR2 comprises an amino acid sequence as shown in SEQ ID NO: 10, and said LCDR3 comprises an amino acid sequence as shown in SEQ ID NO: 24; and
(3) said HCDR1 comprises an amino acid sequence as shown in SEQ ID NO: 27, said HCDR2 comprises an amino acid sequence as shown in SEQ ID NO: 28, said HCDR3 comprises an amino acid sequence as shown in SEQ ID NO: 29, said LCDR1 comprises an amino acid sequence as shown in SEQ ID NO: 34, said LCDR2 comprises an amino acid sequence as shown in SEQ ID NO: 35, and said LCDR3 comprises an amino acid sequence as shown in SEQ ID NO: 36.

In certain embodiments, said antigen-binding protein targeting LILRB4 comprises a VH and a VL, wherein said VH and VL comprise amino acid sequences selected from the group consisting of:
(1) said VH comprises an amino acid sequence as shown in SEQ ID NO: 8, and said VL comprises an amino acid sequence as shown in SEQ ID NO: 16;
(2) said VH comprises an amino acid sequence as shown in SEQ ID NO: 19, and said VL comprises an amino acid sequence as shown in SEQ ID NO: 16;
(3) said VH comprises an amino acid sequence as shown in SEQ ID NO: 22, and said VL comprises an amino acid sequence as shown in SEQ ID NO: 26;
(4) said VH comprises an amino acid sequence as shown in SEQ ID NO: 33, and said VL comprises an amino acid sequence as shown in SEQ ID NO: 40;
(5) said VH comprises an amino acid sequence as shown in SEQ ID NO: 45, and said VL comprises an amino acid sequence as shown in SEQ ID NO: 51;
(6) said VH comprises an amino acid sequence as shown in SEQ ID NO: 55, and said VL comprises an amino acid sequence as shown in SEQ ID NO: 51;
(7) said VH comprises an amino acid sequence as shown in SEQ ID NO: 58, and said VL comprises an amino acid sequence as shown in SEQ ID NO: 51; and
(8) said VH comprises an amino acid sequence as shown in SEQ ID NO: 45, and said VL comprises an amino acid sequence as shown in SEQ ID NO: 61.

In certain embodiments, said antigen-binding protein targeting LILRB4 comprises an antibody heavy chain constant region, wherein said antibody heavy chain constant region is derived from an IgG constant region.

In certain embodiments, said antibody heavy chain constant region comprises an amino acid sequence as shown in SEQ ID NO: 63.

In certain embodiments, said antigen-binding protein targeting LILRB4 comprises an antibody light chain constant region, wherein said antibody light chain constant region is derived from a human Igκ constant region.

In certain embodiments, said antibody light chain constant region comprises an amino acid sequence as shown in SEQ ID NO: 64.

In certain embodiments, said antigen-binding protein targeting LILRB4 comprises an antibody heavy chain and an antibody light chain, wherein said antibody heavy chain and antibody light chain comprise amino acid sequences selected from the group consisting of:
(1) said antibody heavy chain comprises an amino acid sequence as shown in SEQ ID NO: 86, and said antibody light chain comprises an amino acid sequence as shown in SEQ ID NO: 87;
(2) said antibody heavy chain comprises an amino acid sequence as shown in SEQ ID NO: 88, and said antibody light chain comprises an amino acid sequence as shown in SEQ ID NO: 87;
(3) said antibody heavy chain comprises an amino acid sequence as shown in SEQ ID NO: 89, and said antibody light chain comprises an amino acid sequence as shown in SEQ ID NO: 90;
(4) said antibody heavy chain comprises an amino acid sequence as shown in SEQ ID NO: 91, and said antibody light chain comprises an amino acid sequence as shown in SEQ ID NO: 92;
(5) said antibody heavy chain comprises an amino acid sequence as shown in SEQ ID NO: 46, and said antibody light chain comprises an amino acid sequence as shown in SEQ ID NO: 52;
(6) said antibody heavy chain comprises an amino acid sequence as shown in SEQ ID NO: 56, and said antibody light chain comprises an amino acid sequence as shown in SEQ ID NO: 52;
(7) said antibody heavy chain comprises an amino acid sequence as shown in SEQ ID NO: 59, and said antibody light chain comprises an amino acid sequence as shown in SEQ ID NO: 52; and
(8) said antibody heavy chain comprises an amino acid sequence as shown in SEQ ID NO: 46, and said antibody light chain comprises an amino acid sequence as shown in SEQ ID NO: 62.

In certain embodiments, said drug molecule in the immunoconjugate comprises a macromolecular drug and/or a small molecule drug.

In certain embodiments, said macromolecular drug includes other antibody drugs, nucleic acid drugs, molecular glues, and PROTACs; said other antibody drugs include antibodies binding to tumor therapy-related antigens; and said nucleic acid drugs include sgRNA, mRNA, siRNA, shRNA, and antisense RNA.

In certain embodiments, said drug molecule in the immunoconjugate is selected from chemotherapeutic agents, radioactive elements, cell growth inhibitors, and cytotoxic agents.

In certain embodiments, said radioactive element comprises one or more of ¹⁷⁷Lu, ⁶⁸Ga, ⁸⁹Zr, ²²⁵Ac, ¹¹¹In, ⁹⁰Y, ²¹³Bi, ¹³¹I, ¹²⁵I, ⁹⁹mTc, ³H, ¹⁴C, ¹⁵N, ³⁵S, ¹⁸F, and ⁶⁴Cu.

In certain embodiments, said drug molecule is selected from the group consisting of: V-ATPase inhibitor, Bcl2 inhibitor, MCL1 inhibitor, HSP90 inhibitor, IAP inhibitor, mTOR inhibitor, microtubule stabilizer, microtubule destabilizer, auristatin, dolastatin, maytansinoid, MetAP (methionine aminopeptidase), nuclear export inhibitor of protein CRM1, DPPIV inhibitor, proteasome inhibitor, mitochondrial phosphate transfer reaction inhibitor, protein synthesis inhibitor, CDK2 inhibitor, CDK9 inhibitor, kinesin inhibitor, HDAC inhibitor, DNA damaging agent, DNA alkylating agent, DNA intercalating agent, DNA minor groove binder, DHFR inhibitor, nucleoside analog, HDAC inhibitor, anthracycline, NAMPT inhibitor, SN-38 or its derivatives, etoposide phosphate, nitrogen mustard, proteasome inhibitor, cytokine, Tubulysin B analog, and Toll-like receptor agonist.

In certain embodiments, said drug molecule is selected from the group consisting of: antibiotic, microtubule disrupting agent, DNA damaging drug, apoptosis inducer, telomestatin and its analogs, amatoxin, nicotinamide phosphoribosyltransferase, and calicheamicin.

In certain embodiments, said microtubule disrupting agent is selected from: auristatin, maytansine derivative, tubulysin, cryptophycin, and antimitotic EG5 inhibitor, wherein preferably, said auristatin comprises MMAE and/or MMAF, and the maytansine derivative comprises DM1, DM2, DM3, and/or DM4.

In certain embodiments, said DNA damaging drugs are selected from: pyrrolobenzodiazepine drugs, indolinobenzodiazepine drugs, duocarmycins, camptothecin and its derivatives, and calicheamicin; wherein preferably, said pyrrolobenzodiazepine drugs comprise pyrrolo[2,1-c][1,4]benzodiazepine (PBD), said camptothecin and its derivatives comprise SN-38 and/or DXd/DX8951, and said apoptosis inhibitors comprise a Bcl-xL inhibitor.

In certain embodiments, said immunoconjugate further comprises a linker, said linker is capable of connecting said antigen-binding protein targeting LILRB4 and said drug molecule.

In certain embodiments, said linker is selected from: hydrophilic linker, hydrophobic linker, charged linker, uncharged linker, and dicarboxylic acid-based linker.

In certain embodiments, said linker is selected from: non-cleavable linker and cleavable linker.

In certain embodiments, said cleavable linker comprises a chemically reactive linker, acid-cleavable linker, reduction-cleavable linker, and/or enzyme-cleavable linker.

In certain embodiments, said linker is connected to said LILRB4 antigen-binding protein via a thiol group, azide group, or amide group on the LILRB4 antigen-binding protein.

In certain embodiments, said linker is selected from one or more of the following: Acetyl Butyrate, MC-Val-Cit-PABC, SMCC, maleimide, CL2A, MC, MC-VC-PAB, PY-VC-PAB, PEG8-VA-PABC, MC-GGFG, MC-Val-Ala-PAB, MC-Val-Lys(Ac)-PAB, MC-Phe-Lys-PAB, D-Val-Leu-Lys, Gly-Gly-Arg, Ala-Ala-Asn-PAB, Ala-PAB, disulfide bond, and acid-labile hydrazone bond.

In certain embodiments, said immunoconjugate comprises the structure as shown in Formula 2:

Ab-(L1)ₐ-(L2)_{b}-D (Formula 2),

wherein Ab represents an antigen-binding protein targeting LILRB4;
L1 represents a linker connected to Ab, and L2 represents a linker connected to D,
a, b is independently selected from 0-10,
D represents a drug molecule,
wherein preferably L1 is a group or molecule capable of reacting with a thiol group, azide group, or amide group;
L2 comprises one or more of the following: C1-C9 alkyl, C2-C9 alkenyl, C2-C9 alkynyl, C6-C24 aryl, C6-C15 heteroaryl, C3-C9 cycloalkyl, C3-C9 heterocyclyl, polyethylene glycol, O, S, alkylamino, carbonyl, carboxyl, sulfonic acid group, Val-Cit-PABC, VA-PABC, GGFG, Val-Lys(Ac)-PAB, Phe-Lys-PAB, D-Val-Leu-Lys, Gly-Gly-Arg, Ala-Ala-Asn-PAB, and Ala-PAB.

In certain embodiments, Q in Formula 1 of said immunoconjugate is selected from one or more combinations of the following: 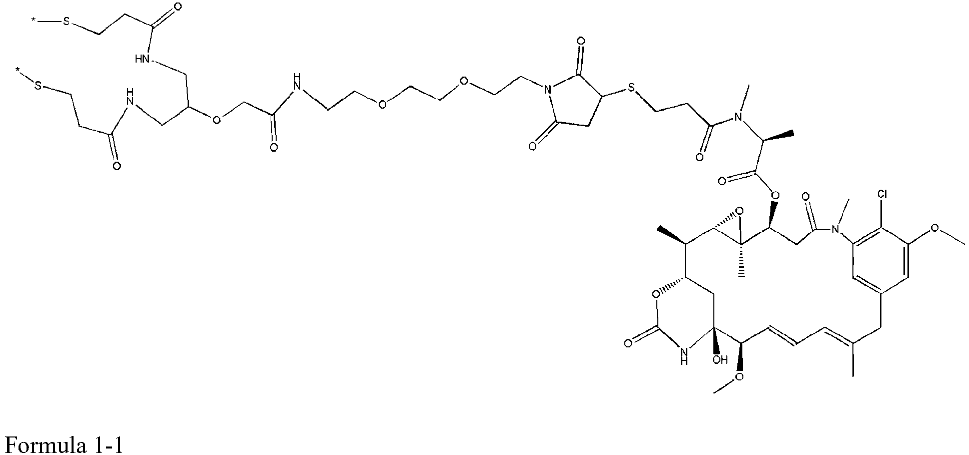 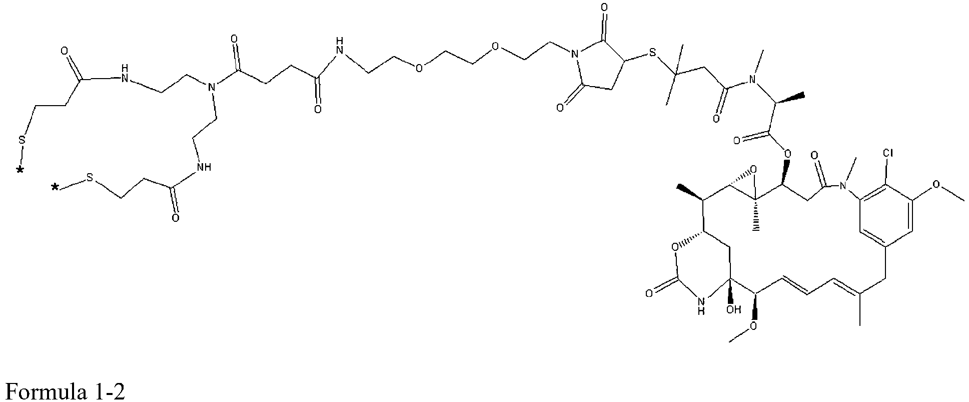 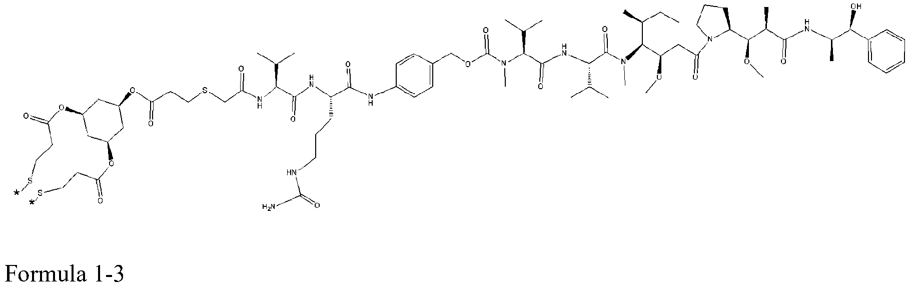 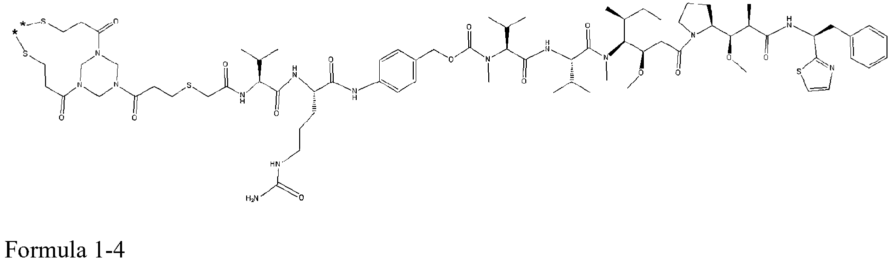 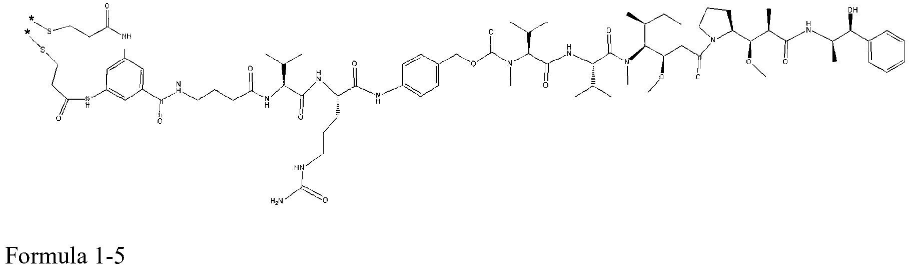 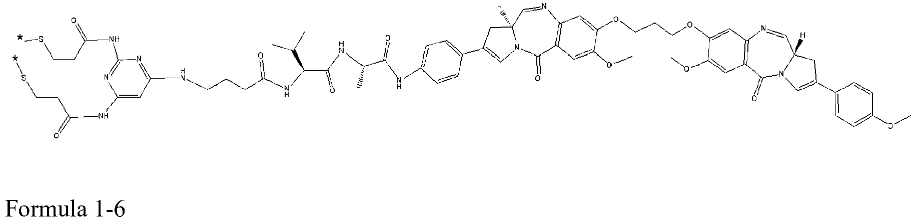 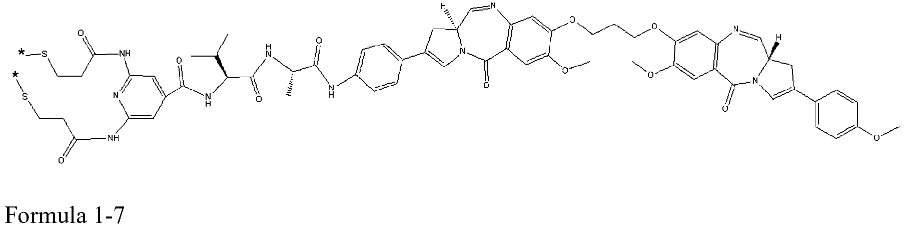 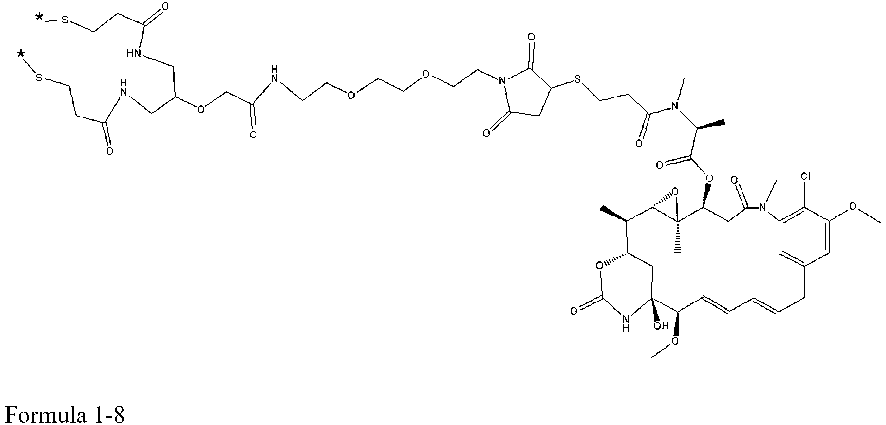 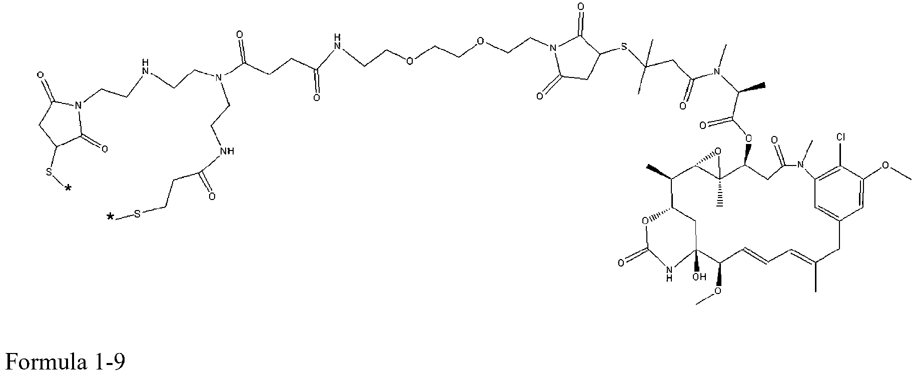 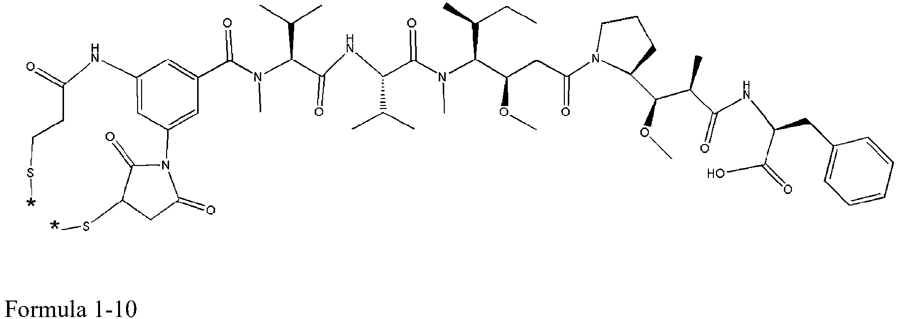 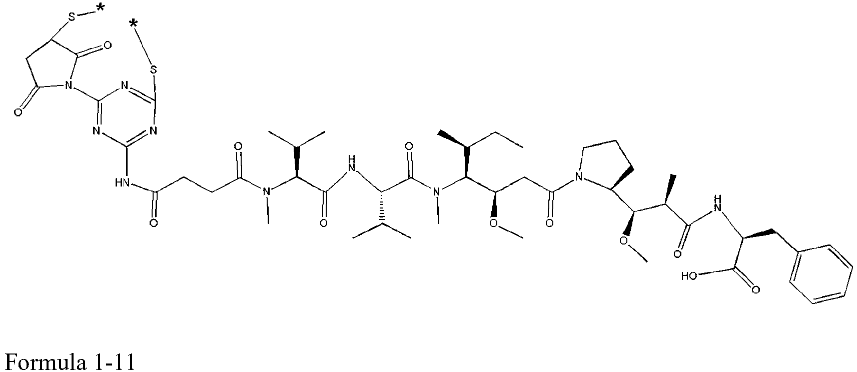 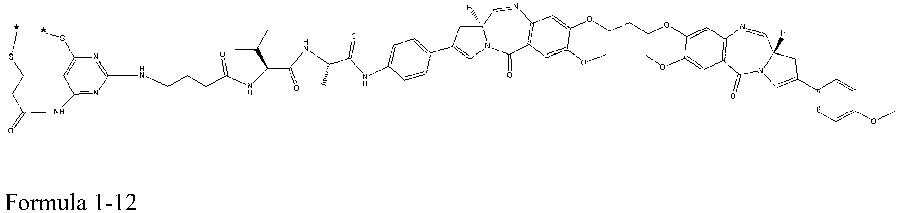 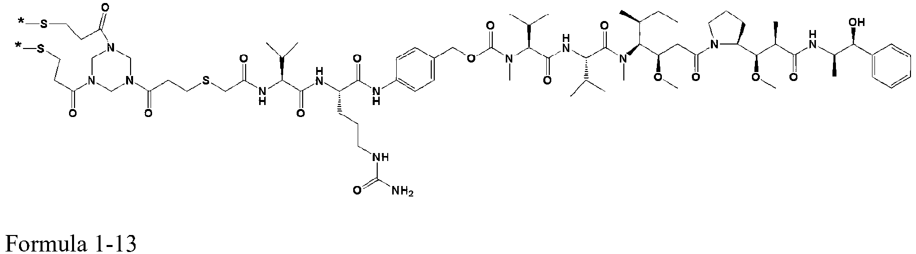 wherein * is the attachment site of Q to the antibody molecule.

In certain embodiments, Q in Formula 1 is selected from the group consisting of: -MC-MK4827, -MC-MK4827&MMAF, -MC-MMAF, -MC-MMAF, -MC-MMAF &SN38, -MC-SN38, -MC-2MMAF, -MC-MC-VC-PAB-MMAE, and -PY-VC-PAB-MMAE.

In certain embodiments, said immunoconjugate has the following molecular formula: 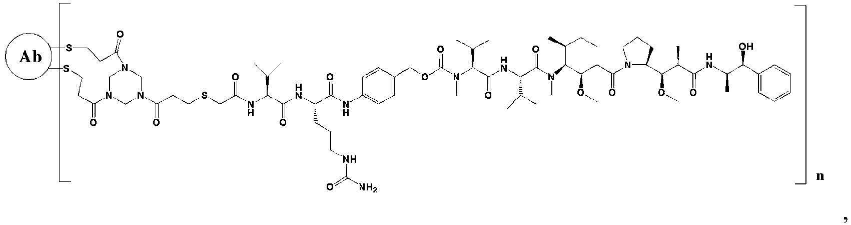 wherein n is selected from 1-8.

In another aspect, the present application provides a pharmaceutical composition comprising said immunoconjugate and, optionally, a pharmaceutically acceptable carrier.

In another aspect, the present application provides a pharmaceutical combination and an immune checkpoint inhibitor. In certain embodiments, said immune checkpoint comprises PD-1, PD-L1, GITR, and/or CTLA-4. Preferably, said immune checkpoint inhibitor comprises one or more selected from the group consisting of: PD-1 antibody, PD-L1 antibody, CTLA-4 antibody, and GITR antibody.

In another aspect, the present application provides a method for preparing said immunoconjugate, comprising conjugating said LILRB4-targeting antigen-binding protein with said drug molecule under suitable conditions.

In another aspect, the present application provides a method for preventing, diagnosing, and/or treating a disease and/or condition, comprising administering to a subject in need thereof said immunoconjugate, said pharmaceutical composition, or said drug combination.

In another aspect, the present application provides the use of said immunoconjugate, said pharmaceutical composition, or said drug combination in the preparation of a medicament for preventing, diagnosing, and/or treating a disease and/or condition.

In another aspect, the present application provides said immunoconjugate, said pharmaceutical composition, or said drug combination for use in preventing, diagnosing, and/or treating a disease and/or condition.

In certain embodiments, said disease and/or condition comprises a disease or condition associated with LILRB4 signal transduction and/or LILRB4 expression. In certain embodiments, said diseases and/or disorders comprise tumors; in certain embodiments, said diseases and/or disorders are selected from hematological tumors and/or solid tumors.

In certain embodiments, said solid tumor is selected from one or more of the following: breast cancer, melanoma, colon cancer, lung cancer, renal cancer, head and neck cancer, gastric cancer, pancreatic cancer, lymphoma, non-small cell lung cancer, leukemia, ovarian cancer, nasopharyngeal carcinoma, breast cancer, endometrial cancer, colon cancer, rectal cancer, gastric cancer, bladder cancer, lung cancer, bronchial cancer, bone cancer, prostate cancer, pancreatic cancer, liver and bile duct cancer, esophageal cancer, renal cancer, thyroid cancer, head and neck cancer, testicular cancer, glioblastoma, astrocytoma, melanoma, myelodysplastic syndrome, and sarcoma; wherein said hematological tumors are selected from multiple myeloma, leukemia, lymphoma, myelodysplastic syndrome, myelodysplastic syndrome, and myeloproliferative neoplasm.

Said leukemia is selected from acute lymphoblastic (lymphoblastic) leukemia, acute myeloid leukemia (AML), myeloid leukemia, chronic lymphocytic leukemia, multiple myeloma, plasma cell leukemia, chronic myeloid leukemia, acute promyelocytic leukemia (APL) or M3 AML, acute myelomonocytic leukemia or M4 AML, and acute monocytic leukemia or M5 AML; wherein preferably, said acute myeloid leukemia is M4 or M5 type acute myeloid leukemia; and preferably, said chronic myeloid leukemia is chronic myelomonocytic leukemia. Said lymphoma is selected from Hodgkin lymphoma and non-Hodgkin lymphoma, including B-cell lymphoma, diffuse large B-cell lymphoma, follicular lymphoma, mantle cell lymphoma, marginal zone B-cell lymphoma, T-cell lymphoma, and Waldenström macroglobulinemia. Said sarcoma is selected from osteosarcoma, Ewing sarcoma, leiomyosarcoma, synovial sarcoma, soft tissue sarcoma, angiosarcoma, liposarcoma, fibrosarcoma, rhabdomyosarcoma, and chondrosarcoma.

Other aspects and advantages of the present application will be readily apparent to those skilled in the art from the detailed description below. Only exemplary embodiments of the present application are shown and described in the detailed description below. As will be recognized by those skilled in the art, the content of the present application enables those skilled in the art to make modifications to the disclosed specific embodiments without departing from the spirit and scope of the invention described herein. Accordingly, the description in the drawings and specification of the present application is merely illustrative and not restrictive.

### Brief Description of the Drawings

The specific features of the invention described herein are set forth in the appended claims. The features and advantages of the invention described herein can be better understood by reference to the exemplary embodiments and drawings described in detail below. The drawings are briefly described as follows:
Figure 1 shows the binding of LILRB4 hybridoma antibodies to LILRB4-overexpressing cells.
Figure 2 shows the binding of LILRB4 hybridoma antibodies to LILRB family proteins.
Figure 3A shows the internalization activity of LILRB4 hybridoma antibodies on LILRB4-positive cells.
Figure 3B shows the inhibitory effect of LILRB4 hybridoma antibodies on migration of THP-1 cells.
Figure 4 shows the binding of the 3A2-18A humanized antibody to recombinant human LILRB4 protein.
Figure 5 shows the binding of the 3A2-18A humanized antibody to LILRB family proteins.
Figure 6 shows the internalization of the 3A2-18A humanized antibody on LILRB4-positive cells.
Figure 7 shows the inhibitory effect of the LILRB4 humanized antibody on migration of THP-1 cells.
Figure 8 shows the activity assay of the LILRB4 humanized antibody in blocking LILRB4/APOE binding.
Figure 9 shows the assay results of the LILRB4 humanized antibody in activating CD8+ T cells.
Figure 10A shows MicroPET/CT scan data of the test antibodies 89Zr-SG2919 and 89Zr-HEL-G1.
Figure 10B shows SUVmean data at 48 h in different tissues of LILRB4-humanized MC38-LILRB4 tumor-bearing and non-tumor-bearing mice for 89Zr-SG2919 and 89Zr-HEL-G1.
Figure 11 shows the cytotoxicity of SG2919-ADC conjugated with different toxins against THP-1 cells.
Figure 12A shows flow cytometry results of LILRB4 expression levels on the surface of various tumor cells.
Figure 12B shows the cytotoxicity of SG2918-ADC against THP-1 cells.
Figure 12C shows the cytotoxicity of SG2918-ADC against MOLM-13 cells.
Figure 12D shows the cytotoxicity of SG2918-ADC against MOLM-16 cells.
Figure 13A shows flow cytometry results of LILRB4 expression levels on the surface of various multiple myeloma (MM) cells.
Figure 13B shows the cytotoxicity of SG2918-PYAE against U-266 cells.
Figure 13C shows the cytotoxicity of SG2918-PYAE against MM.1S cells.
Figure 13D shows the cytotoxicity of SG2918-PYAE against ALMC-1 cells.
Figure 14A shows in vivo drug efficacy data (survival) of SG2918-ADC in a THP-1 systemic tumor model.
Figure 14B shows in vivo drug efficacy data (fluorescence intensity) of SG2918-ADC in a THP-1 systemic tumor model.
Figure 15 shows in vivo drug efficacy of SG2918-PYAE in a multiple myeloma OPM-2 model.
Figure 16 shows the drug efficacy curve of SG2918-PYAE in an LLC1 model.
Figure 17 shows the trend graph of tumor volume change after dosing.

### Detailed Description

The following specific examples illustrate the embodiments of the invention described herein; persons skilled in this art can readily understand other advantages and effects of the invention from the disclosure herein.

### Definition of Terms

In the present application, the term "LILRB4 protein" generally refers to leukocyte immunoglobulin-like receptor B4 (LILRB4). In the present application, this term comprises full-length LILRB4 as well as its functionally active fragments, variants, homologs, analogs, and derivatives.

In the present application, the term "antigen-binding protein" generally refers to a protein having antigen-binding capability. Such an "antigen-binding protein" may comprise an antigen-binding portion and, optionally, a scaffold or framework portion that allows the antigen-binding portion to adopt a conformation facilitating binding of the antigen-binding portion to the antigen. The antigen-binding protein may comprise, for example, protein scaffolds derived from antibodies or alternative protein scaffolds with transplanted CDRs or CDR derivatives, or artificial scaffolds. Such scaffolds include, but are not limited to, antibody-derived scaffolds containing mutations introduced, for example, to stabilize the three-dimensional structure of the antigen-binding protein, and fully synthetic scaffolds comprising, for example, biocompatible polymers. See, for example, Korndorfer et al., 2003, Proteins: Structure, Function, and Bioinformatics, 53(1):121-129 (2003); Roque et al., Biotechnol. Prog. 20:639-654 (2004). Additionally, peptide antibody mimetics ("PAMs") and antibody mimetic scaffolds based on fibronectin domains can be used as scaffolds.

In the present application, the term "antibody" generally refers to an immunoglobulin or fragment or derivative thereof, comprising any polypeptide containing an antigen-binding site, regardless of whether it is produced in vitro or in vivo. This term includes, but is not limited to, polyclonal, monoclonal, monospecific, multispecific, non-specific, humanized, single-chain, chimeric, synthetic, recombinant, hybrid, mutated, and transplanted antibodies. Unless otherwise modified by the term "intact," as in "intact antibody," for purposes of the present invention, the term "antibody" also includes antibody fragments such as Fab, F(ab')2, Fv, scFv, Fd, dAb, and other antibody fragments retaining antigen-binding function. Typically, such fragments should include an antigen-binding domain. The basic four-chain antibody unit is a heterotetrameric glycoprotein composed of two identical light (L) chains and two identical heavy (H) chains. IgM antibodies consist of five basic heterotetrameric units plus an additional polypeptide called the J chain, and contain ten antigen-binding sites, whereas IgA antibodies comprise 2-5 basic four-chain units that can polymerize with the J chain to form multivalent assemblies. For IgG, the four-chain unit has a molecular weight of approximately 150,000 daltons. Each L chain is linked to an H chain via a covalent disulfide bond, while the two H chains are interconnected by one or more disulfide bonds depending on the H chain isotype. Each H and L chain also contains regularly spaced intrachain disulfide bridges. Each H chain has a variable domain (VH) at its N-terminus, followed by three constant domains (CH) for α and γ chains, or four CH domains for µ and ε isotypes. Each L chain has a variable domain (VL) at its N-terminus and a constant domain at its other end. The VL corresponds to the VH, and the CL corresponds to the first constant domain of the heavy chain (CH1). Specific amino acid residues are considered to form the interface between the light and heavy chain variable domains. The VH and VL pair together to form a single antigen-binding site. For the structure and properties of antibodies of different classes, see, for example, Basic and Clinical Immunology, 8th Edition, Daniel P. Stites, Abba I. Terr and Tristram G. Parslow (eds.), Appleton & Lange, Norwalk, Conn., 1994, page 71 and Chapter 6. L chains from any vertebrate species can be classified into one of two distinct types, κ and λ, based on the amino acid sequence of their constant domains. Immunoglobulins can be divided into different classes or isotypes based on the amino acid sequence of their heavy chain (CH) constant domains. There are five classes of immunoglobulins: IgA, IgD, IgE, IgG, and IgM, with heavy chains designated α, δ, ε, γ, and µ, respectively. Based on relatively minor differences in CH sequences and functional characteristics, the γ and α classes are further subdivided into subclasses; for example, humans express the following subclasses: IgG1, IgG2A, IgG2B, IgG3, IgG4, IgA1, and IgK1.

In the present application, the term "CDR" generally refers to regions of the antibody variable domain whose sequences are highly variable and/or form structurally defined loops. Typically, an antibody includes six CDRs: three in the VH (HCDR1, HCDR2, HCDR3) and three in the VL (LCDR1, LCDR2, LCDR3). In natural antibodies, HCDR3 and LCDR3 exhibit the greatest diversity among the six CDRs, and HCDR3 in particular is considered to play a unique role in conferring fine specificity to the antibody. See, for example, Xu et al., Immunity 13:37-45 (2000); Johnson and Wu, in Methods in Molecular Biology 248:1-25 (Lo, ed., Human Press, Totowa, N.J., 2003). Naturally occurring camelid antibodies composed solely of heavy chains function normally and are stable even in the absence of light chains. See, for example, Hamers-Casterman et al., Nature 363:446-448 (1993); Sheriff et al., Nature Struct. Biol. 3:733-736 (1996).

In the present application, the term "FR" generally refers to the more highly conserved portions of the antibody variable domain, known as framework regions. Typically, the variable domains of natural heavy and light chains each contain four FR regions: four in the VH (H-FR1, H-FR2, H-FR3, and H-FR4) and four in the VL (L-FR1, L-FR2, L-FR3, and L-FR4). For example, the VL of the isolated antigen-binding protein described herein may include framework regions L-FR1, L-FR2, L-FR3, and L-FR4. The VH of the isolated antigen-binding protein described herein may include framework regions H-FR1, H-FR2, H-FR3, and H-FR4.

In the art, CDRs of antibodies can be delineated by several methods, for example: (1) the Kabat definition based on sequence variability (Wu and Kabat, J Exp Med 132:211-50, 1970; Kabat et al., Sequences of Proteins of Immunological Interest, 5th edition, Public Health Service, National Institutes of Health, Bethesda, Md., 1991); (2) the Chothia definition based on structural loop positions (Al-Lazikani et al., J Mol Biol 273:927-48, 1997); (3) the AbM definition, which balances the above two rules using Oxford Molecular's AbM antibody modeling software; and (4) the Contact definition based on crystal structure analysis of the resulting complex.

In the present application, the term "antigen-binding fragment" generally refers to one or more fragments of an antibody that mediate specific antigen binding. The antigen-binding function of an antibody can be achieved through a full-length fragment of the antibody. The antigen-binding function of an antibody can also be achieved through fragments comprising Fv, scFv, dsFv, Fab, Fab', or F(ab')₂ of the heavy chain, or fragments comprising Fv, scFv, dsFv, Fab, Fab', or F(ab')₂ of the light chain. (1) Fab fragment, a monovalent fragment consisting of VL, VH, CL, and CH domains; (2) F(ab')₂ fragment, a bivalent fragment comprising two Fab fragments linked by disulfide bonds in the hinge region; (3) Fd fragment consisting of VH and CH domains; (4) Fv fragment consisting of VL and VH domains of one arm of the antibody; (5) dAb fragment consisting of the VH domain (Ward et al., (1989) Nature 341:544-546); (6) isolated complementarity-determining regions (CDRs); and (7) combinations of two or more isolated CDRs optionally linked by a linker. Additionally, it may also include a monovalent single-chain Fv (scFv) molecule formed by pairing VL and VH (see Bird et al. (1988) Science 242:423-426; and Huston et al. (1988) Proc. Natl. Acad. Sci. 85:5879-5883). Said "antigen-binding portion" may also include fusion proteins comprising immunoglobulin. For example, said fusion protein may comprise a binding domain selected from: (1) a binding domain polypeptide fused to an immunoglobulin hinge region polypeptide; (2) an immunoglobulin heavy chain CH2 constant region fused to the hinge region; and/or (3) an immunoglobulin heavy chain CH3 constant region fused to the CH2 constant region.

In the present application, the term "*K_{D}*" may be used interchangeably with "KD" and generally refers to the dissociation equilibrium constant of a specific antibody-antigen interaction, expressed in units of M (mol/L). KD can be calculated from the concentrations of substance AB and its dissociated substances A and B: KD = c(A) * c(B) / c(AB). From this equation, it follows that a larger KD value indicates greater dissociation and thus weaker affinity between substances A and B; conversely, a smaller KD value indicates less dissociation and thus stronger affinity between substances A and B.

In the present application, the term "tumor" generally refers to a physiological condition typically characterized by dysregulation of cell proliferation or survival. The tumor may include all known cancers and tumor conditions, whether characterized as malignant, benign, soft tissue or solid, and cancers at all stages and grades, including pre-metastatic and post-metastatic cancers. The tumor may also include one or more tumor cells.

In the present application, the term "monoclonal antibody" generally refers to a population of substantially homogeneous antibodies, i.e., individual antibodies within the population are identical except for possible naturally occurring mutations present in trace amounts. Monoclonal antibodies can be highly specific, targeting directly a single antigenic site. The monoclonal antibodies may be prepared by hybridoma technology or produced using recombinant DNA methods in bacterial, eukaryotic animal, or plant cells. Monoclonal antibodies may also be derived from phage antibody libraries using techniques described, for example, by Clackson et al., Nature, 352:624-628 (1991) and Marks et al., Mol. Biol., 222:581-597 (1991).

In the present application, the term "single-chain antibody" (scFv) generally refers to a molecule comprising an antibody heavy chain variable region and a light chain variable region. For example, the scFv may be formed by linking the antibody heavy chain variable region and the light chain variable region via a linker molecule (e.g., a linker peptide).

In the present application, the term "chimeric antibody" generally refers to an antibody in which a portion of the amino acid sequence of the heavy or light chain is homologous to the corresponding amino acid sequence of an antibody from a particular species or belongs to a certain class, while the remaining portion of the chain is homologous to the corresponding sequence from another species. For example, the variable regions of both the light and heavy chains may originate from the variable regions of antibodies from one animal species (e.g., mouse, rat, etc.), while the constant regions are homologous to antibody sequences from another species (e.g., human). For example, to obtain a chimeric antibody, the variable region may be generated using non-human B cells or hybridoma cells, while the constant region combined therewith is derived from humans. Since the constant region of a chimeric antibody can be derived from humans, the likelihood of eliciting an immune response upon injection is lower compared to antibodies with constant regions derived from non-human sources.

In the present application, the term "humanized antibody" generally refers to an antibody containing reduced sequences derived from non-human immunoglobulins, thereby reducing immunogenicity when xenogeneic antibodies are introduced into humans. For example, CDR grafting (Jones et al., Nature 321:522 (1986)) and its variants-including "reshaping" (Verhoeyen et al., 1988 Science 239:1534-1536; Riechmann et al., 1988 Nature 332:323-337; Tempest et al., Bio/Technol 1991 9:266-271), "hyperchimerization" (Queen et al., 1989 Proc Natl Acad Sci USA 86:10029-10033; Co et al., 1991 Proc Natl Acad Sci USA 88:2869-2873; Co et al., 1992 J Immunol 148:1149-1154), and "veneering" (Mark et al., "Derivation of therapeutically active humanized and veneered anti-CD18 antibodies." In: Metcalf B W, Dalton B J, eds. Cellular adhesion: molecular definition to therapeutic potential. New York: Plenum Press, 1994: 291-312)-as well as surface remodeling (U.S. Patent US5639641), may be used to humanize non-human binding domains. If other regions, such as the hinge region and constant domain regions, are also derived from non-human sources, these regions may also be humanized.

In the present application, the term "fully human antibody" generally refers to a fully human antibody, i.e., an antibody whose constant and variable regions are both derived from humans. The fully human antibody may be generated using technologies such as phage antibody library technology, transgenic mice producing human antibodies, ribosome display technology, EBV-transformed B-cell cloning technology, or single B-cell cloning.

In the present application, the term "multispecific antibody" generally refers to an antibody molecule capable of simultaneously recognizing two or more antigens or epitopes. The multispecific antibody may be obtained in eukaryotic or prokaryotic expression systems using methods such as chemical conjugation, hybrid-hybridoma, or genetic engineering-based antibody preparation.

In the present application, the term "immunoconjugate" generally refers to a conjugate formed by linking another agent/drug molecule (e.g., chemotherapeutic agent, radioactive element, cell growth inhibitor, and cytotoxic agent) to the antibody or its antigen-binding fragment. This linkage may occur via covalent bonds or non-covalent interactions and may include chelation. The immunoconjugate may deliver the other agent to target cells (e.g., tumor cells) through the specific binding of the antibody or its antigen-binding fragment to the antigen on the target cell surface. Subsequently, the immunoconjugate undergoes internalization and ultimately enters the interior of the target cell (e.g., into lysosomes or other vesicular compartments), where the linker molecule in the immunoconjugate may cleave, releasing the other agent to exert its cytotoxic effect. Additionally, the antigen may also be secreted by the target cell and located in the extracellular space surrounding the target cell. Various linker molecules known in the art may be used to form immunoconjugates. In the present application, the term "linker" generally refers to a functional molecule that connects or joins two molecules. For example, the linker molecule may connect one molecule to another molecule (e.g., one molecule being a protein molecule and the other molecule also being a protein molecule or a small molecule drug). The linker molecule may be used in the construction of the immunoconjugate. In the immunoconjugate, the linker molecule may possess two functional characteristics: 1) stability in the circulatory system-prior to the immunoconjugate reaching the target cell, the linker must not cleave and release the other agent in the circulation to avoid toxic effects; 2) after entering the target cell, the linker molecule must rapidly and effectively cleave to allow effective release of the other agent to exert its intended pharmacological activity. The linker molecule may consist of polar or non-polar amino acids. The linker molecule may also be a carbon chain containing heteroatoms (such as nitrogen atoms, sulfur atoms, etc.). The length of the linker molecule may range from 2 to 100 atoms, for example, between 2 and 50 atoms, or specifically 3, 5, 10, 15, 20, 25, 30, 35, 40, 45, or 50 atoms; alternatively, for example, the length of the linker may be 20 to 26 atoms (20, 21, 22, 23, 24, 25, or 26 atoms). The linker molecule may include substituents selected from the group consisting of: hydrogen atom, alkyl, alkenyl, alkynyl, amino, alkylamino, dialkylamino, trialkylamino, hydroxy, alkoxy, halogen, aryl, heterocycle, aromatic heterocycle, cyano, amide, carbamoyl, carboxylic acid, ester, thioether, alkylthioether, thiol group, and ureido. Furthermore, the linker molecule may be selected from the group consisting of: pH-sensitive linkers, protease-cleavable linkers, nuclease-sensitive linkers, lipase-sensitive linkers, glycosidase linkers, hypoxia-sensitive linkers, photolabile linkers, thermally labile linkers, ultrasound-sensitive linkers, and peptide linkers. Exemplary linker molecules include, but are not limited to, 1,4-bis(maleimido)butane, (1,4-bismaleimidino-2,3-dihydroxybutane), bis(maleimido)hexane, bis(maleimido)ethane, 1,4-di-[3'-(2'-pyridyldithio)propionamido]butane, 1,6-hexanediyl divinyl sulfone, dithiobismaleimidoethane, 1,8-bismaleimidodiethylene glycol, and 1,11-bismaleimidotriethylene glycol. Common peptide linkers are well known in the art (see, e.g., Holliger et al. (1993) Proc. Natl. Acad. Sci. USA 90:6444-6448; Poljak et al. (1994) Structure 2:1121-1123).

In the present application, the term "covalent" generally refers to a covalent bond, i.e., a relatively stable chemical structure formed when two or more atoms share electron pairs to achieve electronic saturation. Covalent bond formation involves pairing of electrons with opposite spin directions between two adjacent atoms, resulting in overlapping atomic orbitals and increased electron cloud density between the two nuclei, thereby enhancing the attractive force toward both nuclei. Covalent bonds can exhibit saturation and directionality. Covalent bonds can be classified as nonpolar covalent bonds, polar covalent bonds, and coordinate covalent bonds. Compounds containing only covalent bonds may be referred to as covalent compounds.

In the present application, the term "drug molecule" generally refers to an active agent molecule conjugated to an antigen-binding protein. The drug molecule may be of various types. For example, the drug molecule may be a macromolecular drug or a small molecule drug. The macromolecular drug may include other antibody drugs, nucleic acid drugs, molecular glues, and PROTACs. The other antibody drugs may include antibodies targeting tumor therapy-related antigens other than LILRB4 antibodies, for example, PD-1 antibodies, PD-L1 antibodies, anti-CTLA-4 antibodies, LAG3 antibodies, ICOS antibodies, 4-1BB antibodies, TIGIT antibodies, TIM3 antibodies, CD47 antibodies, antibodies binding T lymphocyte surface markers, antibodies binding tumor stromal cells, or antibodies binding surface markers of myeloid-derived suppressor cells or NK cells. The nucleic acid drug antigens include, but are not limited to, sgRNA, mRNA, siRNA, shRNA, and antisense RNA. PROTAC and molecular glue (molecular glue) represent two primary modalities of targeted protein degradation technologies based on the ubiquitin-proteasome system. Specifically, PROTAC recruits E3 ubiquitin ligases to bring the target protein into proximity with the E3 ubiquitin ligase, leading to ubiquitination and degradation of the target protein. In contrast, molecular glue modifies the surface of ubiquitin ligases to promote or induce protein-protein interactions (PPI) between the E3 ubiquitin ligase and the target protein, resulting in ubiquitination and subsequent degradation of the target protein. Molecular glues include, but are not limited to, immunomodulatory drugs (IMiDs; thalidomide, lenalidomide, pomalidomide), CFT-7455, BAY-2666605, DKY-709, ICP-490, CC-9009, CC-92480, CC-99282, BTX-1188, and MRT-2359.

The small molecule drug may be a chemotherapeutic agent, radioactive element, cell growth inhibitor, and/or cytotoxic agent. In the present application, when referring to specific types of drug molecules, the meaning includes not only the drug molecule itself but also its analogs, derivatives, variants, and other forms. In certain circumstances, the drug molecule may possess therapeutic activity. In certain circumstances, the drug molecule may possess cytotoxicity. In the present application, the term "chemotherapeutic agent" generally refers to a chemotherapeutic reagent capable of inhibiting tumor and/or tumor cell proliferation. The chemotherapeutic agent may be selected from the group consisting of: mitotic inhibitors, kinase inhibitors, alkylating agents, antimetabolites, intercalating antibiotics, growth factor inhibitors, cell cycle inhibitors, enzymes, topoisomerase inhibitors, histone deacetylase inhibitors, anti-survival agents, biological response modifiers, anti-hormones such as anti-androgens, and anti-angiogenic agents. For example, the chemotherapeutic agent may be selected from the group consisting of: capecitabine, daunorubicin, daunomycin, actinomycin D, doxorubicin, epirubicin, idarubicin, esorubicin, bleomycin, mafosfamide, ifosfamide, cytarabine, chlorozotocin, busulfan, mitomycin C, actinomycin D, mithramycin, prednisone, hydroxyprogesterone, testosterone, tamoxifen, dacarbazine, procarbazine, altretamine, pentamustine, mitoxantrone, amsacrine, chlorambucil, methylcyclohexylnitrosourea, mechlorethamine, melphalan, cyclophosphamide, 6-mercaptopurine, 6-thioguanine, cytarabine (CA), 5-azacytidine, hydroxyurea, deoxycoformycin, 4-hydroperoxycyclophosphamide, 5-fluorouracil (5-FU), 5-fluoro-2'-deoxyuridine (5-FUdR), methotrexate (MTX), colchicine, paclitaxel (taxol), vincristine, vinblastine, etoposide, trimetrexate, teniposide, and/or diethylstilbestrol (DES).

In the present application, the term "radioactive element" generally refers to an element used in radiotherapy capable of inhibiting tumor and/or tumor cell proliferation. The radioactive element may be selected from the group consisting of: ¹⁷⁷Lu, ⁶⁸Ga, ⁸⁹Zr, ²²⁵Ac, ⁹⁰Y, ²¹³Bi, ⁹⁹mTc, ¹⁸F, ⁶⁴Cu, ³H, ¹⁴C, ¹⁵N, ³⁵S, ⁹⁹Tc, ¹¹¹In, ¹²⁵I, and/or ¹³¹I.

In the present application, the term "cell growth inhibitor" generally refers to an agent that inhibits tumors by inhibiting growth factors that promote tumor cell growth and replication. Upon binding to receptors on the cell surface, growth factors activate intracellular signaling pathways; complex pathways may cause uncontrolled cell growth, leading to excessive cell division and tumor development. The cell growth inhibitor can suppress the action of these growth factors. The cell growth inhibitor may be selected from the group consisting of: angiogenesis inhibitors, histone deacetylase (HDAC) inhibitors, Hedgehog signaling pathway inhibitors, mTOR inhibitors, p53/mdm2 inhibitors, PARP inhibitors, proteasome inhibitors, and/or tyrosine kinase inhibitors.

In the present application, the term "cytotoxic agent" generally refers to an agent that exerts toxicity on the cells it acts upon to inhibit tumor and/or tumor cell proliferation. The cytotoxic agent may be selected from the group consisting of: alkylating agents such as busulfan, altretamine, thiotepa, cyclophosphamide, mechlorethamine, uramustine, melphalan, chlorambucil, carmustine, streptozocin, dacarbazine, temozolomide, and ifosfamide; antitumor agents such as mitomycin C; antimetabolites such as methotrexate, azathioprine, mercaptopurine, fludarabine, and 5-fluorouracil; platinum-containing anticancer agents such as cisplatin and carboplatin; anthracyclines such as daunorubicin, doxorubicin, epirubicin, idarubicin, and mitoxantrone; plant alkaloids and terpenoids such as vincristine, vinblastine, vinorelbine, vindesine, podophyllotoxin, and docetaxel; topoisomerase inhibitors such as irinotecan, amsacrine, topotecan, etoposide, and teniposide; antibody-based agents such as rituximab, trastuzumab, bevacizumab, erlotinib, and dactinomycin; finasteride; aromatase inhibitors; tamoxifen; goserelin; paclitaxel; and/or imatinib mesylate. The cytotoxic agent may be administered orally, by injection, or by other routes.

In the present application, the term "pharmaceutical composition" generally refers to a composition suitable for administration to a patient, preferably a human patient. For example, the pharmaceutical composition described herein may comprise the immunoconjugate described herein and, optionally, a pharmaceutically acceptable adjuvant. Additionally, the pharmaceutical composition may further comprise a suitable formulation containing one or more (pharmaceutically effective) carriers, stabilizers, excipients, diluents, solubilizers, surfactants, emulsifiers, and/or preservatives. Acceptable components of the composition are preferably non-toxic to the recipient at the doses and concentrations employed. The pharmaceutical compositions of the present invention include, but are not limited to, liquid, frozen, and lyophilized compositions.

In the present application, the term "pharmaceutically acceptable carrier" generally refers to any and all solvents, dispersion media, coatings, isotonic agents, absorption delaying agents, etc., compatible with drug administration, which are generally safe, non-toxic, and neither biologically nor otherwise undesirable.

In the present application, the term "subject" generally refers to a human or non-human animal, including but not limited to cats, dogs, horses, pigs, cows, sheep, rabbits, mice, rats, or monkeys.

In the present application, the term "about" generally refers to a variation within ±0.5% to 10% of the specified value, for example, within ±0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% of the specified value.

In the present application, the term "comprising" generally carries the meaning of including, encompassing, containing, or incorporating. In certain contexts, it may also signify "being," or "consisting of."

### Detailed Description of the Invention

### LILRB4-targeting antigen-binding protein

Antibody CDRs, also known as complementarity-determining regions, are part of the variable region. Amino acid residues in this region can contact the antigen or antigenic epitope. Antibody CDRs can be defined using various numbering schemes, such as CCG, Kabat, Chothia, IMGT, AbM, or combinations considering both Kabat and Chothia. These numbering schemes are known in the art; see, for example, http://www.bioinf.org.uk/abs/index.html#kabatnum. Those skilled in the art can determine CDR regions using different numbering schemes based on the antibody sequence and structure. CDR regions may differ depending on the numbering scheme used. In the present application, the term CDR encompasses CDR sequences defined by any CDR delineation method, as well as variants thereof, wherein the variants include amino acid sequences of the CDRs with one or more amino acid substitutions, deletions, and/or insertions. For example, 1-30, 1-20, or 1-10, or specifically 1, 2, 3, 4, 5, 6, 7, 8, or 9 amino acid substitutions, deletions, and/or insertions; it also encompasses homologs thereof, wherein the homologs are amino acid sequences having at least about 85% (e.g., at least about 85%, about 90%, about 91%, about 92%, about 93%, about 94%, about 95%, about 96%, about 97%, about 98%, about 99%, or higher) sequence homology with the CDR amino acid sequence. For example, the CDRs of the isolated antigen-binding proteins listed in the sequence listing of the present application can be determined using the Kabat scheme.

In one aspect, the present application provides an isolated antigen-binding protein comprising at least one CDR from a VH as shown in any of SEQ ID NO: 81, SEQ ID NO: 33, and SEQ ID NO: 22; and/or comprising at least one CDR from a VL as shown in SEQ ID NO: 82 or SEQ ID NO: 40.

X₁VQLX₂QX₃GAEX₄X₅KPGASVKX₆SCKASGYTFTSYWMHWVX₇QX₈PGQGLEWX₉GEINPSNGR TNYNEKFKSX₁₀X₁₁TX₁₂TX₁₃DX₁₄SX₁₅STX₁₆YX₁₇X₁₈LX₁₉SLX₂₀SEDX₂₁AVYYCARDRYDEGNAM DYWGQGTX₂₂VTVSS (SEQ ID NO: 81). Wherein X₁ can be E or Q; X₂ can be Q or V; X₃ can be P or S; X₄ can be L or V; X₅ can be K or V; X₆ can be L or V; X₇ can be K or R; X₈ can be A or R; X₉ can be I or M; X₁₀ can be K or R; X₁₁ can be A or V; X₁₂ can be L or M; X₁₃ can be R or V; X₁₄ can be K or T; X₁₅ can be S or T; X₁₆ can be A or V; X₁₇ can be M or V; X₁₈ can be E or Q; X₁₉ can be N or S; X₂₀ can be R or T; X₂₁ can be S or T; X₂₂ can be S or T.

DIX₁MTX₂X₃X₄SSLSASX₅GDRVTIX₆CRASQDIX₇NYLNWYQQKPX₈X₉X₁₀X₁₁KLLIYYTSRLHSGV PSRFSGSGSGTX₁₂YX₁₃X₁₄TISX₁₅LX₁₆X₁₇X₁₈DX₁₉ATYX₂₀CQQGX₂₁TLPX₂₂TFGX₂₃GTX₂₄LEIK (SEQ ID NO: 82). Wherein X₁ can be Q or V; X₂ can be H or Q; X₃ can be S or T; X₄ can be P or T; X₅ can be L or V; X₆ can be S or T; X₇ can be S or T; X₈ can be D or G; X₉ can be G or K; X₁₀ can be A or T; X₁₁ can be P or V; X₁₂ can be D or E; X₁₃ can be S or T; X₁₄ can be F or L; X₁₅ can be N or S; X₁₆ can be E or Q; X₁₇ can be P or Q; X₁₈ can be D or E; X₁₉ can be F or I; X₂₀ can be F or Y; X₂₁ can be D or N; X₂₂ can be P or R; X₂₃ can be G or Q; X₂₄ can be K or R.

In the present application, the VH may comprise an amino acid sequence as shown in any of SEQ ID NO: 8, SEQ ID NO: 19, SEQ ID NO: 22, SEQ ID NO: 33, SEQ ID NO: 45, SEQ ID NO: 55, and SEQ ID NO: 58.

In the present application, the VL may comprise an amino acid sequence as shown in any of SEQ ID NO: 16, SEQ ID NO: 26, SEQ ID NO: 40, SEQ ID NO: 51, and SEQ ID NO: 61.

In the present application, the HCDR of the isolated antigen-binding protein may be delineated by any method, provided that the VH and/or VL amino acid sequence is identical to any of the VH and/or VL sequences disclosed herein; thus, any HCDR defined by any delineation method falls within the scope of protection of the present application.

For example, different CDR delineation methods applied to VH (SEQ ID NO: 8) and/or VL (SEQ ID NO: 16) are summarized in the following table.

| | Chothia | AbM | Kabat | Contact | IMGT |
|---|---|---|---|---|---|
| LCDR1 | L24-34 | L24-34 | L24-34 | L30-36 | L27-32 |
| LCDR2 | L50-56 | L50-56 | L50-56 | L46-55 | L50-51 |
| LCDR3 | L89-97 | L89-97 | L89-97 | L89-96 | L89-97 |
| HCDR1 | H26-32 | H26-35 | H31-35 | H30-35 | H26-33 |
| HCDR2 | H52-57 | H50-59 | H50-66 | H47-59 | H51-58 |
| HCDR3 | H99-109 | H99-109 | H99-109 | H97-108 | H97-109 |

For example, the delineation of VH (SEQ ID NO: 19) and/or VL (SEQ ID NO: 16) using different CDR numbering schemes is summarized in the following table.

| | Chothia | AbM | Kabat | Contact | IMGT |
|---|---|---|---|---|---|
| LCDR1 | L24-34 | L24-34 | L24-34 | L30-36 | L27-32 |
| LCDR2 | L50-56 | L50-56 | L50-56 | L46-55 | L50-51 |
| LCDR3 | L89-97 | L89-97 | L89-97 | L89-96 | L89-97 |
| HCDR1 | H26-32 | H26-35 | H31-35 | H30-35 | H26-33 |
| HCDR2 | H52-57 | H50-59 | H50-66 | H47-59 | H51-58 |
| HCDR3 | H99-109 | H99-109 | H99-109 | H97-108 | H97-109 |

For example, the delineation of VH (SEQ ID NO: 22) and/or VL (SEQ ID NO: 26) using different CDR numbering schemes is summarized in the following table.

| | Chothia | AbM | Kabat | Contact | IMGT |
|---|---|---|---|---|---|
| LCDR1 | L24-34 | L24-34 | L24-34 | L30-36 | L27-32 |
| LCDR2 | L50-56 | L50-56 | L50-56 | L46-55 | L50-51 |
| LCDR3 | L89-97 | L89-97 | L89-97 | L89-96 | L89-97 |
| HCDR1 | H26-32 | H26-35 | H31-35 | H30-35 | H26-33 |
| HCDR2 | H52-57 | H50-59 | H50-66 | H47-59 | H51-58 |
| HCDR3 | H99-110 | H99-110 | H99-110 | H97-109 | H97-110 |

For example, the division of VH (SEQ ID NO: 33) and/or VL (SEQ ID NO: 40) using different CDR annotation methods is summarized in the following table.

| | Chothia | AbM | Kabat | Contact | IMGT |
|---|---|---|---|---|---|
| LCDR1 | L24-38 | L24-38 | L24-38 | L30-40 | L27-36 |
| LCDR2 | L54-60 | L54-60 | L54-60 | L50-59 | L54-55 |
| LCDR3 | L93-101 | L93-101 | L93-101 | L93-100 | L93-101 |
| HCDR1 | H26-32 | H26-35 | H31-35 | H30-35 | H26-33 |
| HCDR2 | H52-59 | H50-61 | H50-68 | H47-61 | H51-60 |
| HCDR3 | H101-108 | H101-108 | H101-108 | H99-107 | H99-108 |

Herein, Laa-Lbb refers to the amino acid sequence from position aa to bb, counting from the N-terminus of the antibody light chain variable region; Haa-Hbb refers to the amino acid sequence from position aa to bb, counting from the N-terminus of the antibody heavy chain variable region. For example, L24-L34 refers to the amino acid sequence from position 24 to 34, counting from the N-terminus of the antibody light chain; H26-H35 refers to the amino acid sequence from position 26 to 35, counting from the N-terminus of the antibody heavy chain.

In one embodiment, in the sequence listing of the present application, the CDR sequences and framework region (FR) sequences of the isolated antigen-binding protein are defined according to the Kabat numbering scheme.

In the present application, the VH of the isolated antigen-binding protein may comprise HCDR1, HCDR2, and HCDR3.

In the present application, the HCDR1 may comprise an amino acid sequence shown as SEQ ID NO: 69.

X₁X₂WMX₃ (SEQ ID NO: 69). Wherein X₁ may be D or S; X₂ may be A or Y; X₃ may be D or H.

For example, the HCDR1 may comprise an amino acid sequence as shown in SEQ ID NO: 1 or SEQ ID NO: 27.

In the present application, the HCDR2 may comprise an amino acid sequence as shown in SEQ ID NO: 2 or SEQ ID NO: 28.

In the present application, the HCDR3 may comprise an amino acid sequence as shown in SEQ ID NO: 83 or SEQ ID NO: 29.

[-G][DI][RY]Y[DG][EY][DG][NY]AMDY (SEQ ID NO: 83). Wherein X₁ may be absent or G; X₂ may be D or I; X₃ may be R or Y; X₄ may be D or G; X₅ may be E or Y; X₆ may be D or G; X₇ may be N or Y.

In the present application, the HCDR1 of the isolated antigen-binding protein may comprise an amino acid sequence as shown in SEQ ID NO: 1 or SEQ ID NO: 27, the HCDR2 may comprise an amino acid sequence as shown in SEQ ID NO: 2 or SEQ ID NO: 28, and the HCDR3 may comprise an amino acid sequence as shown ins any one of SEQ ID NO: 3, SEQ ID NO: 20, and SEQ ID NO: 29.

In the present application, the HCDR1 of the isolated antigen-binding protein may comprise an amino acid sequence as shown in SEQ ID NO: 1, the HCDR2 may comprise an amino acid sequence as shown in SEQ ID NO: 2, and the HCDR3 may comprise an amino acid sequence as shown in SEQ ID NO: 3.

In the present application, the HCDR1 of the isolated antigen-binding protein may comprise an amino acid sequence as shown in SEQ ID NO: 1, the HCDR2 may comprise an amino acid sequence as shown in SEQ ID NO: 2, and the HCDR3 may comprise an amino acid sequence as shown in SEQ ID NO: 20.

In the present application, the HCDR1 of the isolated antigen-binding protein may comprise an amino acid sequence as shown in SEQ ID NO: 27, the HCDR2 may comprise an amino acid sequence as shown in SEQ ID NO: 28, and the HCDR3 may comprise an amino acid sequence as shown in SEQ ID NO: 29.

In the present application, the VL of the isolated antigen-binding protein may comprise LCDR1, LCDR2, and LCDR3.

In the present application, the LCDR1 may comprise an amino acid sequence as shown in SEQ ID NO: 70 or SEQ ID NO: 34.

RASQDIX₁NYLN (SEQ ID NO: 70). Wherein X₁ may be S or T.

For example, the LCDR1 may comprise an amino acid sequence as shown in any one of SEQ ID NO: 9, SEQ ID NO: 23, and SEQ ID NO: 34.

In the present application, the LCDR2 may comprise an amino acid sequence as shown in SEQ ID NO: 71.

YX₁SX₂LX₃S (SEQ ID NO: 71). Wherein X₁ may be A or T; X₂ may be N or R; X₃ may be H or Q.

For example, the LCDR2 may comprise an amino acid sequence as shown in SEQ ID NO: 10 or SEQ ID NO: 35.

In the present application, the LCDR3 may comprise an amino acid sequence as shown in SEQ ID NO: 72.

QX₁X₂X₃X₄X₅PX₆T (SEQ ID NO: 72). For example, X₁ may be H or Q; X₂ may be G or S; X₃ may be D, N, or W; X₄ may be E or T; X₅ may be I or L; X₆ may be P or R.

For example, the LCDR3 may comprise an amino acid sequence as shown in any one of SEQ ID NO: 11, SEQ ID NO: 24, and SEQ ID NO: 36.

In the present application, the LCDR1 of the isolated antigen-binding protein may comprise an amino acid sequence as shown in SEQ ID NO: 70 or SEQ ID NO: 34, the LCDR2 may comprise an amino acid sequence as shown in SEQ ID NO: 71, and the LCDR3 may comprise an amino acid sequence as shown in SEQ ID NO: 72 (QX₁X₂X₃X₄X₅PX₆T, wherein X₁ is H or Q; X₂ is G or S; X₃ is D, N, or W; X₄ is E or T; X₅ is I or L; X₆ is P or R).

In the present application, the isolated antigen-binding protein may comprise HCDR1, HCDR2, HCDR3, LCDR1, LCDR2, and LCDR3.

In the present application, the HCDR1 of the isolated antigen-binding protein may comprise an amino acid sequence as shown in SEQ ID NO: 1 or SEQ ID NO: 27, the HCDR2 may comprise an amino acid sequence as shown in SEQ ID NO: 2 or SEQ ID NO: 28, the HCDR3 may comprise an amino acid sequence as shown in any one of SEQ ID NO: 3, SEQ ID NO: 20, and SEQ ID NO: 29, the LCDR1 may comprise an amino acid sequence as shown in any one of SEQ ID NO: 9, SEQ ID NO: 23, and SEQ ID NO: 34, the LCDR2 may comprise an amino acid sequence as shown in SEQ ID NO: 10 or SEQ ID NO: 35, and the LCDR3 may comprise an amino acid sequence as shown in any one of SEQ ID NO: 11, SEQ ID NO: 24, and SEQ ID NO: 36.

In the present application, the HCDR1, HCDR2, HCDR3, LCDR1, LCDR2, and LCDR3 of the isolated antigen-binding protein may comprise an amino acid sequences selected from any one of the following groups:
(1) the HCDR1 may comprise an amino acid sequence as shown in SEQ ID NO: 1, the HCDR2 may comprise an amino acid sequence as shown in SEQ ID NO: 2, the HCDR3 may comprise an amino acid sequence as shown in SEQ ID NO: 3, the LCDR1 may comprise an amino acid sequence as shown in SEQ ID NO: 9, the LCDR2 may comprise an amino acid sequence as shown in SEQ ID NO: 10, and the LCDR3 may comprise an amino acid sequence as shown in SEQ ID NO: 11;
(2) the HCDR1 may comprise an amino acid sequence as shown in SEQ ID NO: 1, the HCDR2 may comprise an amino acid sequence as shown in SEQ ID NO: 2, the HCDR3 may comprise an amino acid sequence as shown in SEQ ID NO: 20, the LCDR1 may comprise an amino acid sequence as shown in SEQ ID NO: 23, the LCDR2 may comprise an amino acid sequence as shown in SEQ ID NO: 10, and the LCDR3 may comprise an amino acid sequence as shown in SEQ ID NO: 24; and
(3) the HCDR1 may comprise an amino acid sequence as shown in SEQ ID NO: 27, the HCDR2 may comprise an amino acid sequence as shown in SEQ ID NO: 28, the HCDR3 may comprise an amino acid sequence as shown in SEQ ID NO: 29, the LCDR1 may comprise an amino acid sequence as shown in SEQ ID NO: 34, the LCDR2 may comprise an amino acid sequence as shown in SEQ ID NO: 35, and the LCDR3 may comprise an amino acid sequence as shown in SEQ ID NO: 36.

In the present application, the VH of the isolated antigen-binding protein may comprise framework regions H-FR1, H-FR2, H-FR3, and H-FR4.

In the present application, the C-terminus of the H-FR1 may be directly or indirectly connected to the N-terminus of the HCDR1, and the H-FR1 may comprise an amino acid sequence as shown in SEQ ID NO: 73.

X₁VX₂LX₃X₄X₅GX₆X₇X₈X₉X₁₀PGX₁₁SX₁₂KX₁₃SCX₁₄ASGX₁₅TFX₁₆ (SEQ ID NO: 73). wherein X₁ may be E or Q; X₂ may be K or Q; X₃ may be E, V, or Q; X₄ may be E or Q; X₅ may be P or S; X₆ may be A or G; X₇ may be E or G; X₈ may be L or V; X₉ may be K or V; X₁₀ may be K or Q; X₁₁ may be A or G; X₁₂ may be M or V; X₁₃ may be L or V; X₁₄ may be A or K; X₁₅ may be F or Y; X₁₆ may be S or T.

For example, the H-FR1 may comprise an amino acid sequence as shown in any one of SEQ ID NO: 4, SEQ ID NO: 17, SEQ ID NO: 30, and SEQ ID NO: 41.

In the present application, the H-FR2 may be located between the HCDR1 and the HCDR2, and the H-FR2 may comprise an amino acid sequence as shown in SEQ ID NO: 74.

WVX₁QX₂PX₃X₄GLEWX₅X₆ (SEQ ID NO: 74). wherein X₁ may be K or R; X₂ may be A, R, or S; X₃ may be E or G; X₄ may be K or Q; X₅ may be I, M, or V; X₆ may be A or G.

For example, the H-FR2 may comprise an amino acid sequence as shown in any one of SEQ ID NO: 5, SEQ ID NO: 31, SEQ ID NO: 42, and SEQ ID NO: 53.

In the present application, the H-FR3 may be located between the HCDR2 and the HCDR3, and the H-FR3 may comprise an amino acid sequence as shown in SEQ ID NO: 75.

X₁X₂TX₃X₄X₅DX₆SX₇SX₈X₉YX₁₀X₁₁X₁₂X₁₃SLX₁₄X₁₅EDX₁₆X₁₇X₁₈YYCX₁₉R (SEQ ID NO: 75). wherein X₁ may be K or R; X₂ may be A, F, or V; X₃ may be I, L, or M; X₄ may be I, S, or T; X₅ may be R or V; X₆ may be D, K, or T; X₇ may be K, S, or T; X₈ may be S or T; X₉ may be A or V; X₁₀ may be L, M, or V; X₁₁ may be E or Q; X₁₂ may be L or M; X₁₃ may be N or S; X₁₄ may be R or T; X₁₅ may be A or S; X₁₆ may be S or T; X₁₇ may be A or G; X₁₈ may be I or V; X₁₉ may be A or T.

For example, the H-FR3 may comprise an amino acid sequence as shown in any one of SEQ ID NO: 6, SEQ ID NO: 18, SEQ ID NO: 21, SEQ ID NO: 32, SEQ ID NO: 43, SEQ ID NO: 54, and SEQ ID NO: 57.

In the present application, the N-terminus of the H-FR4 may be connected to the C-terminus of the HCDR3, and the H-FR4 may comprise an amino acid sequence as shown in SEQ ID NO: 76.

GQGTX₁VTVSS (SEQ ID NO: 76). wherein X₁ may be S or T.

For example, the H-FR4 may comprise an amino acid sequence as shown in SEQ ID NO: 7 or SEQ ID NO: 44.

For example, the H-FR1 of the isolated antigen-binding protein described in the present application may comprise an amino acid sequence as shown in SEQ ID NO: 73, the H-FR2 may comprise an amino acid sequence as shown in SEQ ID NO: 74, the H-FR3 may comprise an amino acid sequence as shown in SEQ ID NO: 75, and the H-FR4 may comprise an amino acid sequence as shown in SEQ ID NO: 76.

For example, the H-FR1, H-FR2, H-FR3, and H-FR4 of the isolated antigen-binding protein described in the present application may comprise an amino acid sequence as shown in any one of the following groups:
(1) the H-FR1 comprises an amino acid sequence as shown in SEQ ID NO: 4, the H-FR2 comprises an amino acid sequence as shown in SEQ ID NO: 5, the H-FR3 comprises an amino acid sequence as shown in SEQ ID NO: 6, and the H-FR4 comprises an amino acid sequence as shown in SEQ ID NO: 7;
(2) the H-FR1 comprises an amino acid sequence as shown in SEQ ID NO: 17, the H-FR2 comprises an amino acid sequence as shown in SEQ ID NO: 5, the H-FR3 comprises an amino acid sequence as shown in SEQ ID NO: 18, and the H-FR4 comprises an amino acid sequence as shown in SEQ ID NO: 7;
(3) the H-FR1 comprises an amino acid sequence as shown in SEQ ID NO: 4, the H-FR2 comprises an amino acid sequence as shown in SEQ ID NO: 5, the H-FR3 comprises an amino acid sequence as shown in SEQ ID NO: 21, and the H-FR4 comprises an amino acid sequence as shown in SEQ ID NO: 7;
(4) the H-FR1 comprises an amino acid sequence as shown in SEQ ID NO: 30, the H-FR2 comprises an amino acid sequence as shown in SEQ ID NO: 31, the H-FR3 comprises an amino acid sequence as shown in SEQ ID NO: 32, and the H-FR4 comprises an amino acid sequence as shown in SEQ ID NO: 7;
(5) the H-FR1 comprises an amino acid sequence as shown in SEQ ID NO: 41, the H-FR2 comprises an amino acid sequence as shown in SEQ ID NO: 42, the H-FR3 comprises an amino acid sequence as shown in SEQ ID NO: 43, and the H-FR4 comprises an amino acid sequence as shown in SEQ ID NO: 44;
(6) the H-FR1 comprises an amino acid sequence as shown in SEQ ID NO: 41, the H-FR2 comprises an amino acid sequence as shown in SEQ ID NO: 53, the H-FR3 comprises an amino acid sequence as shown in SEQ ID NO: 54, and the H-FR4 comprises an amino acid sequence as shown in SEQ ID NO: 44;
(7) the H-FR1 comprises an amino acid sequence as shown in SEQ ID NO: 41, the H-FR2 comprises an amino acid sequence as shown in SEQ ID NO: 53, the H-FR3 comprises an amino acid sequence as shown in SEQ ID NO: 57, and the H-FR4 comprises an amino acid sequence as shown in SEQ ID NO: 44.

In the present application, the VL of the isolated antigen-binding protein may comprise framework regions L-FR1, L-FR2, L-FR3, and L-FR4.

In the present application, the C-terminus of the L-FR1 may be directly or indirectly connected to the N-terminus of the LCDR1, and the L-FR1 may comprise an amino acid sequence as shown in SEQ ID NO: 77.

DIX₁MTX₂X₃X₄X₅SLX₆X₇SX₈GX₉RX₁₀TIX₁₁C (SEQ ID NO: 77). wherein X₁ may be Q or V; X₂ may be H or Q; X₃ may be S or T; X₄ may be P or T; X₅ may be A or S; X₆ may be A or S; X₇ may be A or V; X₈ may be L or V; X₉ may be D or Q; X₁₀ may be A or V; X₁₁ may be S or T.

For example, the L-FR1 may comprise an amino acid sequence as shown in any one of SEQ ID NO: 12, SEQ ID NO: 25, SEQ ID NO: 37, and SEQ ID NO: 47.

In the present application, the L-FR2 may be located between the LCDR1 and the LCDR2, and the L-FR2 may comprise an amino acid sequence as shown in SEQ ID NO: 78.

WYQQKPX₁X₂X₃X₄KLLIX₅ (SEQ ID NO: 78). wherein X₁ may be D or G; X₂ may be G, K, or Q; X₃ may be A, P, or T; X₄ may be P or V; X₅ may be K or Y.

For example, the L-FR2 may comprise an amino acid sequence as shown in any one of SEQ ID NO: 13, SEQ ID NO: 38, and SEQ ID NO: 48.

In the present application, the L-FR3 may be located between the LCDR2 and the LCDR3, and the L-FR3 may comprise an amino acid sequence as shown in SEQ ID NO: 79.

GVPX₁RFSGSGSGTX₂X₃X₄X₅X₆IX₇X₈X₉X₁₀X₁₁X₁₂DX₁₃ATYX₁₄C (SEQ ID NO: 79). wherein X₁ may be A or S; X₂ may be D or E; X₃ may be F or Y; X₄ may be S or T; X₅ may be F or L; X₆ may be N or T; X₇ may be H or S; X₈ may be N, P, or S; X₉ may be L or V; X₁₀ may be E or Q; X₁₁ may be E, P, or Q; X₁₂ may be D or E; X₁₃ may be F, I, or S; X₁₄ may be F or Y.

For example, the L-FR3 may comprise an amino acid sequence as shown in any one of SEQ ID NO: 14, SEQ ID NO: 39, SEQ ID NO: 49, and SEQ ID NO: 60.

In the present application, the N-terminus of the L-FR4 may be connected to the C-terminus of the HCDR3, and the L-FR4 may comprise an amino acid sequence as shown in SEQ ID NO: 80.

FGX₁GTX₂LEIK (SEQ ID NO: 80). wherein X₁ may be G or Q; X₂ may be K or R.

For example, the L-FR4 may comprise an amino acid sequence as shown in SEQ ID NO: 15 or SEQ ID NO: 50.

For example, the L-FR1 of the isolated antigen-binding protein described in the present application may comprise an amino acid sequence as shown in SEQ ID NO: 77, the L-FR2 may comprise an amino acid sequence as shown in SEQ ID NO: 78, the L-FR3 may comprise an amino acid sequence as shown in SEQ ID NO: 79, and the L-FR4 may comprise an amino acid sequence as shown in SEQ ID NO: 80.

For example, the H-FR1, H-FR2, H-FR3, and H-FR4 of the isolated antigen-binding protein described in the present application may comprise an amino acid sequence as shown in any one of the following groups:
(1) the L-FR1 comprises an amino acid sequence as shown in SEQ ID NO: 12, the L-FR2 comprises an amino acid sequence as shown in SEQ ID NO: 13, the L-FR3 comprises an amino acid sequence as shown in SEQ ID NO: 14, and the L-FR4 comprises an amino acid sequence as shown in SEQ ID NO: 15;
(2) the L-FR1 comprises an amino acid sequence as shown in SEQ ID NO: 25, the L-FR2 comprises an amino acid sequence as shown in SEQ ID NO: 13, the L-FR3 comprises an amino acid sequence as shown in SEQ ID NO: 14, and the L-FR4 comprises an amino acid sequence as shown in SEQ ID NO: 15;
(3) the L-FR1 comprises an amino acid sequence as shown in SEQ ID NO: 37, the L-FR2 comprises an amino acid sequence as shown in SEQ ID NO: 38, the L-FR3 comprises an amino acid sequence as shown in SEQ ID NO: 39, and the L-FR4 comprises an amino acid sequence as shown in SEQ ID NO: 15;
(4) the L-FR1 comprises an amino acid sequence as shown in SEQ ID NO: 47, the L-FR2 comprises an amino acid sequence as shown in SEQ ID NO: 48, the L-FR3 comprises an amino acid sequence as shown in SEQ ID NO: 49, and the L-FR4 comprises an amino acid sequence as shown in SEQ ID NO: 50; and
(5) the L-FR1 comprises an amino acid sequence as shown in SEQ ID NO: 47, the L-FR2 comprises an amino acid sequence as shown in SEQ ID NO: 48, the L-FR3 comprises an amino acid sequence as shown in SEQ ID NO: 60, and the L-FR4 comprises an amino acid sequence as shown in SEQ ID NO: 50.

The isolated antigen-binding protein described in the present application may comprise an antibody heavy chain variable region VH and an antibody light chain variable region VL.

In the present application, the VH may comprise an amino acid sequence as shown in any one of SEQ ID NO: 81, SEQ ID NO: 33, and SEQ ID NO: 22. For example, the VH may comprise an amino acid sequence as shown in any one of SEQ ID NO: 8, SEQ ID NO: 19, SEQ ID NO: 22, SEQ ID NO: 33, SEQ ID NO: 45, SEQ ID NO: 55, and SEQ ID NO: 58.

In the present application, the VL may comprise an amino acid sequence as shown in SEQ ID NO: 82 or SEQ ID NO: 40. For example, the VL may comprise an amino acid sequence as shown in any one of SEQ ID NO: 16, SEQ ID NO: 26, SEQ ID NO: 40, SEQ ID NO: 51, and SEQ ID NO: 61.

For example, the VH of the isolated antigen-binding protein may comprise an amino acid sequence as shown in SEQ ID NO: 8, and the VL may comprise an amino acid sequence as shown in SEQ ID NO: 16.

For example, the VH of the isolated antigen-binding protein may comprise an amino acid sequence as shown in SEQ ID NO: 19, and the VL may comprise an amino acid sequence as shown in SEQ ID NO: 16.

For example, the VH of the isolated antigen-binding protein may comprise an amino acid sequence as shown in SEQ ID NO: 22, and the VL may comprise an amino acid sequence as shown in SEQ ID NO: 26.

For example, the VH of the isolated antigen-binding protein may comprise an amino acid sequence as shown in SEQ ID NO: 33, and the VL may comprise an amino acid sequence as shown in SEQ ID NO: 40.

For example, the VH of the isolated antigen-binding protein may comprise an amino acid sequence as shown in SEQ ID NO: 45, and the VL may comprise an amino acid sequence as shown in SEQ ID NO: 51.

For example, the VH of the isolated antigen-binding protein may comprise an amino acid sequence as shown in SEQ ID NO: 55, and the VL may comprise an amino acid sequence as shown in SEQ ID NO: 51.

For example, the VH of the isolated antigen-binding protein may comprise an amino acid sequence as shown in SEQ ID NO: 58, and the VL may comprise an amino acid sequence as shown in SEQ ID NO: 51.

For example, the VH of the isolated antigen-binding protein may comprise an amino acid sequence as shown in SEQ ID NO: 45, and the VL may comprise an amino acid sequence as shown in SEQ ID NO: 61.

In the present application, the isolated antigen-binding protein may comprise an antibody heavy chain constant region, and the antibody heavy chain constant region may be derived from a human IgG heavy chain constant region.

In certain embodiments, the isolated antigen-binding protein may comprise an antibody heavy chain constant region, and the antibody heavy chain constant region may be derived from a human IgG1 heavy chain constant region. In other embodiments, the isolated antigen-binding protein may comprise an antibody heavy chain constant region, and the antibody heavy chain constant region may be derived from a human IgG4 heavy chain constant region. The isolated antigen-binding protein may comprise a native Fc and Fc mutants, wherein the Fc mutants include but are not limited to S239D/I332E and S239D/I332E/A330L, which significantly enhance binding and ADCC activity; S239D/I332E Fc variants that promote B-cell depletion; S239D/I332E mutations that enhance ADCC/ADCP activity; S298A/E333A/K334A and F243L/R292P/Y300L/V305I/P396L variants that enhance FcγRIIIa binding and ADCC activity; IgG1 antibodies lacking fucose at the N297-linked carbohydrate moiety, which markedly increase ADCC activity; L234A/L235A, D265A, and N297A, which reduce ADCC activity; S267E/H268F/S324T, which increases C1q affinity and enhances CDC activity; G236A/I332E, which improves undesirable ADCC activity; M252Y/S254T/T256E (Y/T/E), which increases IgG affinity for FcRn; and M428L/N434S.

For example, the antibody heavy chain constant region may comprise an amino acid sequence as shown in SEQ ID NO: 63.

In the present application, the isolated antigen-binding protein may comprise an antibody light chain constant region, and the antibody light chain constant region may comprise a human Igκ constant region. For example, the antibody light chain constant region may comprise an amino acid sequence as shown in SEQ ID NO: 64.

In the present application, the isolated antigen-binding protein may comprise an antibody heavy chain HC, and the HC may comprise an amino acid sequence as shown in any one of SEQ ID NO: 46, SEQ ID NO: 56, SEQ ID NO: 59, SEQ ID NO: 86, SEQ ID NO: 88, SEQ ID NO: 89, and SEQ ID NO: 91.

In the present application, the isolated antigen-binding protein may comprise an antibody light chain LC, and the LC may comprise an amino acid sequence as shown in any one of SEQ ID NO: 52, SEQ ID NO: 62, SEQ ID NO: 87, SEQ ID NO: 90, and SEQ ID NO: 92.

The isolated antigen-binding protein described in the present application may comprise an antibody heavy chain and an antibody light chain.

The isolated antigen-binding protein described in the present application may comprise an antibody heavy chain and an antibody light chain.

For example, the antibody heavy chain may comprise an amino acid sequence as shown in SEQ ID NO: 86, and the antibody light chain may comprise an amino acid sequence as shown in SEQ ID NO: 87.

For example, the antibody heavy chain may comprise an amino acid sequence as shown in SEQ ID NO: 88, and the antibody light chain may comprise an amino acid sequence as shown in SEQ ID NO: 87.

For example, the antibody heavy chain may comprise an amino acid sequence as shown in SEQ ID NO: 89, and the antibody light chain may comprise an amino acid sequence as shown in SEQ ID NO: 90.

For example, the antibody heavy chain may comprise an amino acid sequence as shown in SEQ ID NO: 91, and the antibody light chain may comprise an amino acid sequence as shown in SEQ ID NO: 92.

For example, the antibody heavy chain may comprise an amino acid sequence as shown in SEQ ID NO: 46, and the antibody light chain may comprise an amino acid sequence as shown in SEQ ID NO: 52.

For example, the antibody heavy chain may comprise an amino acid sequence as shown in SEQ ID NO: 56, and the antibody light chain may comprise an amino acid sequence as shown in SEQ ID NO: 52.

For example, the antibody heavy chain may comprise an amino acid sequence as shown in SEQ ID NO: 59, and the antibody light chain may comprise an amino acid sequence as shown in SEQ ID NO: 52.

For example, the antibody heavy chain may comprise an amino acid sequence as shown in SEQ ID NO: 46, and the antibody light chain may comprise an amino acid sequence as shown in SEQ ID NO: 62.

Furthermore, it should be noted that the isolated antigen-binding protein described in the present application may comprise heavy chain and/or light chain sequences having one or more conservative sequence modifications relative to the antigen-binding proteins described herein. The "conservative sequence modification" refers to amino acid modifications that do not significantly affect or alter the binding properties of the antibody. Such conservative modifications include amino acid substitutions, additions, and deletions. Modifications can be introduced into the isolated antigen-binding protein described herein using standard techniques known in the art, such as site-directed mutagenesis and PCR-mediated mutagenesis. Conservative amino acid substitutions involve replacing an amino acid residue with another amino acid residue having a similar side chain. Groups of amino acid residues having similar side chains are known in the art. These groups of amino acid residues include those having basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). In certain embodiments, one or more amino acid residues in the CDR regions of the isolated antigen-binding protein described herein may be replaced with other amino acid residues from the same side-chain group. Those skilled in the art understand that some conservative sequence modifications do not abolish antigen-binding activity; see, for example, Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem. 32:6862-35; Adib-Conquy et al., (1998) Int. Immunol. 10:341-6; and Beers et al., (2000) Clin. Can. Res. 6:2835-43.

Proteins, polypeptides, and/or amino acid sequences referred to in the present application should also be understood to encompass at least the following scope: variants or homologs possessing the same or similar functions as the stated proteins or polypeptides.

In the present application, the term "variant" refers to a protein or polypeptide in which one or more amino acids have been substituted, deleted, or added in the amino acid sequence of the protein and/or polypeptide (e.g., the isolated antigen-binding protein described herein). For example, the variant may comprise a protein or polypeptide having amino acid alterations through at least one substitution, deletion, and/or insertion, for instance, 1-30, 1-20, or 1-10, such as 1, 2, 3, 4, or 5 amino acid substitutions, deletions, and/or insertions. The functional variant may substantially retain the biological properties of the protein or polypeptide prior to the alteration (e.g., substitution, deletion, or addition). For example, the functional variant may retain at least 60%, 70%, 80%, 90%, or 100% of the biological activity (e.g., the ability to specifically bind LILRB4 protein) of the protein or polypeptide prior to the alteration.

In the present application, the term "homolog" refers to a protein or polypeptide having at least about 80% (e.g., at least about 85%, about 90%, about 91%, about 92%, about 93%, about 94%, about 95%, about 96%, about 97%, about 98%, about 99%, or higher) sequence homology with the amino acid sequence of the protein and/or polypeptide (e.g., the antibody or its antigen-binding fragment described herein).

In the present application, the term "homology" generally refers to the degree of similarity or relatedness between two or more sequences. The "percentage of sequence homology" can be calculated by comparing two sequences to be aligned within a comparison window, determining the number of positions at which the two sequences have identical nucleotide bases (e.g., A, T, C, G, I) or identical amino acid residues (e.g., Ala, Pro, Ser, Thr, Gly, Val, Leu, Ile, Phe, Tyr, Trp, Lys, Arg, His, Asp, Glu, Asn, Gln, Cys, and Met) to obtain the number of matching positions, dividing the number of matching positions by the total number of positions in the comparison window (i.e., window size), and multiplying the result by 100 to yield the percentage of sequence homology. Alignment for determining the percentage of sequence homology can be performed in various ways known in the art, for example, using publicly available computer software such as BLAST, BLAST-2, ALIGN, or Megalign (DNASTAR) software. Those skilled in the art can determine suitable parameters for sequence alignment, including any algorithm required to achieve maximal alignment over the full-length sequences being compared or within a region of interest. The homology can also be determined by methods such as FASTA and BLAST. Descriptions of the FASTA algorithm can be found in W. R. Pearson and D. J. Lipman, "Improved tools for biological sequence comparison," Proceedings of the National Academy of Sciences (Proc. Natl. Acad. Sci.), 85:2444-2448, 1988; and D. J. Lipman and W. R. Pearson, "Rapid and sensitive protein similarity searches," Science, 227:1435-1441, 1989. Descriptions of the BLAST algorithm can be found in S. Altschul, W. Gish, W. Miller, E. W. Myers, and D. Lipman, "Basic local alignment search tool," Journal of Molecular Biology, 215:403-410, 1990.

In this application, the isolated antigen-binding protein may include an antibody or an antigen-binding fragment thereof. For example, the isolated antigen-binding protein described herein may include, but is not limited to, recombinant antibodies, monoclonal antibodies, human antibodies, murine antibodies, humanized antibodies, chimeric antibodies, single-chain antibodies, multibodies, Fv fragments, scFv fragments, Fab fragments, Fab' fragments, F(ab')₂ fragments, and camelidized single-domain antibodies.

In this application, the antibody may be a humanized antibody. In other words, the isolated antigen-binding protein described herein may be an antibody or a variant, derivative, analog, or fragment thereof that immunospecifically binds to a relevant antigen (e.g., human LILRB4) and comprises framework (FR) regions having substantially the amino acid sequence of a human antibody and complementarity-determining regions (CDRs) having substantially the amino acid sequence of a non-human antibody. As used herein, "substantially" in the context of CDRs means that the amino acid sequence of the CDR is at least 80%, preferably at least 85%, at least 90%, at least 95%, at least 98%, or at least 99% identical to the amino acid sequence of the CDR of a non-human antibody. The humanized antibody substantially comprises all of at least one, and typically both, variable domains (Fab, Fab', F(ab')2, FabC, Fv), wherein all or substantially all CDR regions correspond to those of a non-human immunoglobulin (i.e., antibody), and all or substantially all framework regions are framework regions having the consensus sequence of a human immunoglobulin. Preferably, the humanized antibody further comprises at least a portion of an immunoglobulin constant region (e.g., Fc), typically the constant region of a human immunoglobulin. In some embodiments, the humanized antibody contains at least the light chain and the variable domain of the heavy chain. The antibody may also include the CH1, hinge, CH2, CH3, and CH4 regions of the heavy chain. In some embodiments, the humanized antibody contains only a humanized light chain. In some embodiments, the humanized antibody contains only a humanized heavy chain. In specific embodiments, the humanized antibody contains only the humanized variable domain(s) of the light chain and/or the humanized heavy chain.

In this application, the said antigen-binding fragment may include Fab, Fab', F(ab)₂, Fv fragments, F(ab')₂, scFv, di-scFv, and/or dAb.

In this application, the said isolated antigen-binding protein may possess one or more of the following properties: 1) capable of specifically recognizing leukocyte immunoglobulin-like receptor B4 (LILRB4); 2) capable of binding to LILRB4 protein with a K_{D} value of 2×10⁻⁷ M or lower; 3) capable of blocking the activity of LILRB4/APOE binding; 4) capable of stimulating activation of CD8+ T cells; 5) capable of inhibiting migration of human monocytic leukemia cells THP-1; 6) capable of inhibiting tumor cell growth and/or metastasis; 7) capable of blocking the binding of LILRB4 to Fibronectin.

In this application, the said isolated antigen-binding protein is capable of binding to LILRB4 protein with a K_{D} value of 2×10⁻⁷ M or lower. For example, the K_{D} value of the isolated antigen-binding protein described herein for binding to human LILRB4 protein may be ≤2×10⁻⁷ M, ≤1.5×10⁻⁷ M, ≤1×10⁻⁷ M, ≤9×10⁻⁸ M, ≤8×10⁻⁸ M, ≤7×10⁻⁸ M, ≤6×10⁻⁸ M, ≤5×10⁻⁸ M, ≤4×10⁻⁸ M, ≤3×10⁻⁸ M, ≤2×10⁻⁸ M, ≤1.5×10⁻⁸ M, ≤1.2×10⁻⁸ M, ≤1.15×10⁻⁸ M, ≤1.1×10⁻⁸ M, ≤1.05×10⁻⁸ M, ≤1×10⁻⁸ M, ≤5×10⁻⁹ M, or ≤1×10⁻⁹ M.

In this application, the KD value may also be determined by ELISA, competitive ELISA, BIACORE, or KINEXA.

In this application, the isolated antigen-binding protein is capable of specifically binding to human LILRB4 protein. The specific binding can be assessed by FACS assay.

In this application, the LILRB4 protein may be human LILRB4 protein (GenBank Accession No.: CAG46845.1).

The LILRB4 protein may include variants of LILRB4 protein. For example, the variant may be: 1) a protein or polypeptide in which one or more amino acids have been substituted, deleted, or added in the amino acid sequence of the LILRB4 protein; and 2) a protein or polypeptide having at least about 85% (e.g., at least about 85%, about 90%, about 91%, about 92%, about 93%, about 94%, about 95%, about 96%, about 97%, about 98%, about 99%, or higher) sequence homology with the LILRB4 protein.

The LILRB4 protein may also include functional active fragments of LILRB4 protein.

### linker

Linkers used in immunoconjugates can be classified into cleavable and non-cleavable types. Cleavable linkers release the drug at target cells under specific conditions. Cleavable linkers can be divided into chemically cleavable or enzymatically cleavable linkers. There are three main types of chemically induced cleavable linkers: acid-cleavable linkers, linkers cleaved under reducing conditions (e.g., disulfides), and linkers cleavable by exogenous stimuli.

Acid-cleavable linkers must maintain stability in circulation under physiological conditions (pH ≈ 7.4) and undergo acid-catalyzed hydrolysis upon entering the acidic environments of endosomes (pH ~5.5-6.2) and lysosomes (pH ~4.5-5.0).

Acid-cleavable linkers may also include carbonate-based linkers. In certain cases, simple carbonates exhibit limited stability in serum, and a para-aminobenzyl (PAB) group can be introduced to improve half-life. For example, the carbonate structure can hydrolyze under acidic conditions to release SN-38 drug and carbon dioxide.

Specific enzymes present both intracellularly in lysosomes and extracellularly in the tumor microenvironment can selectively cleave their corresponding substrates. Therefore, the linker used may be an enzyme-cleavable linker.

For example, cathepsin-cleavable dipeptides can be used as dipeptide-based linkers. For example, the said dipeptide may be Val-Cit, Val-Ala, or Phe-Lys. Additionally, a self-immolative spacer may be introduced to facilitate enzyme access. For example, the said self-immolative spacer may be PABC. In certain cases, tetrapeptide linkers may also be used. For example, the tetrapeptide linker may be Gly-Gly-Phe-Gly. For example, when using a tetrapeptide linker, the self-immolative spacer may be hemiaminal.

Like cathepsins, pyrophosphatases and phosphatases are also hydrolytic enzymes selectively expressed in lysosomes. For example, linkers containing phosphate or pyrophosphate groups can be combined with the cathepsin B-sensitive Val-Cit-PABA.

β-Glucuronidase is a glycosidase that catalyzes the hydrolysis of β-glucuronic acid residues and is highly expressed in lysosomes and tumor stroma. In certain cases, glucuronide-containing linkers may be used. Glucuronic acid can be attached to a self-immolative spacer. Glucuronide-containing linkers can also be applied to other amine-containing payloads, such as camptothecin analogs, SN38, duocarmycin, and matrine, via an additional dimethylethylenediamine (DMED) self-immolative spacer.

In certain embodiments, the said linker may also include a β-galactosidase-cleavable linker. The said β-galactosidase-cleavable linker may contain a PEG10 spacer. The spacer may be substituted with a nitro group to enhance the rate of self-immolation.

In certain embodiments, the said linker includes a sulfatase-cleavable linker.

In this application, the linker may include a charged linker or an uncharged linker.

In this application, the linker may include a dicarboxylic acid-based linker.

In this application, the linker may include hydrophilic linkers (e.g., PEG4Mal and sulfo-SPDB) and hydrophobic linkers. For example, the hydrophilic linker may reduce the extent to which the antibody-drug conjugate is pumped out of resistant cancer cells by MDR (multidrug resistance) or functionally similar transporter proteins.

In this application, the linker may also function to directly or indirectly inhibit cell growth and/or cell proliferation. For example, the linker may function as an intercalating agent upon cleavage. For example, the linker may inhibit macromolecular biosynthesis.

In this application, the linker may promote cellular uptake of the antibody-drug conjugate (e.g., promote 'internalization'). In this application, the linker may also be designed to enhance the stability of the antibody-drug conjugate.

For example, the linker may possess a functional group capable of reacting with free cysteine residues on the antibody to form a bond (e.g., a covalent bond). For example, the functional group may include: maleimide, haloacetamides, α-haloacetyl groups, active esters such as succinimidyl esters, 4-nitrophenyl esters, pentafluorophenyl esters, tetrafluorophenyl esters, acid anhydrides, acyl chlorides, sulfonyl chlorides, isocyanates, and/or isothiocyanates.

In certain cases, the linker may include a functional group capable of reacting with electrophilic groups present on the antibody. For example, the functional group may include: aldehydes, ketone carbonyls, hydrazides, oximes, amines, hydrazines, thiosemicarbazides, hydrazine carboxylates, and/or aryl hydrazides.

In this application, the linker may be connected to the LILRB4 antigen-binding protein via a thiol group, azide group, or amide group present on the LILRB4-targeting antigen-binding protein. For example, linked via a thiol group.

In this application, the linker may be selected from one or more of the following: Acetyl Butyrate, MC-Val-Cit-PABC, SMCC, maleimide, CL2A, MC, MC-VC-PAB, PY-VC-PAB, PEG8-VA-PABC, MC-GGFG, MC-Val-Ala-PAB, MC-Val-Lys(Ac)-PAB, MC-Phe-Lys-PAB, D-Val-Leu-Lys, Gly-Gly-Arg, Ala-Ala-Asn-PAB, Ala-PAB, disulfide bond, and acid-labile hydrazone bond.

In this application, the types of linker include, but are not limited to, linkers currently known and disclosed in patent documents such as CN107921030A, WO2023237050, and US20050238649A1. The linkers disclosed in these documents may be used individually or in combination. For example, a single immunoconjugate molecule may contain one linker, or two or more linkers.

### drug molecule

In this application, the immunoconjugate comprises a drug molecule. In this application, the drug molecule may comprise any known molecule possessing pharmaceutical activity. For example, the drug molecule may be of any molecular type. For example, the drug molecule may be selected from at least one of the following agents: chemotherapeutic agent, radioactive element, cell growth inhibitor, and cytotoxic agent.

In this application, the radioactive element may be selected from one or more of the following: ¹⁷⁷Lu, ⁶⁸Ga, ⁸⁹Zr, ²²⁵Ac, ¹¹¹In, ⁹⁰Y, ²¹³Bi, ¹³¹I, ⁹⁹mTc, ³H, ¹⁴C, ¹⁵N, ³⁵S, ¹⁸F, and ⁶⁴Cu.

In this application, the drug molecule may include macromolecular drug and/or small molecule drug. The macromolecular drug may be other antibody drugs, nucleic acid drugs, molecular glues, and PROTACs. The other antibody drugs may be antibodies targeting tumor therapy-related antigens other than LILRB4 antibodies, for example, PD-1 antibodies, PD-L1 antibodies, anti-CTLA-4 antibodies, LAG3 antibodies, ICOS antibodies, 4-1BB antibodies, TIGIT antibodies, TIM3 antibodies, CD47 antibodies, antibodies binding to T lymphocyte surface markers, antibodies binding to tumor stromal cells, antibodies binding to myeloid-derived suppressor cells, or antibodies binding to NK cell surface markers. The nucleic acid drug antigens include, but are not limited to, sgRNA, mRNA, siRNA, shRNA, and antisense RNA.

PROTAC and molecular glue are two primary modalities of targeted protein degradation technologies based on the ubiquitin-proteasome system. Specifically, PROTAC recruits an E3 ubiquitin ligase to bring the target protein into proximity with the E3 ubiquitin ligase, leading to ubiquitination and degradation of the target protein. In contrast, molecular glue modifies the surface of the ubiquitin ligase to promote or induce protein-protein interactions (PPI) between the E3 ubiquitin ligase and the target protein, resulting in ubiquitination and subsequent degradation of the target protein. Molecular glues include, but are not limited to, immunomodulatory drugs (IMiDs; thalidomide, lenalidomide, pomalidomide), CFT-7455, BAY-2666605, DKY-709, ICP-490, CC-9009, CC-92480, CC-99282, BTX-1188, and MRT-2359.

The small molecule drug may be a chemotherapeutic agent, radioactive element, cell growth inhibitor, and/or cytotoxic agent. In this application, when referring to specific types of drug molecules, the term encompasses not only the drug molecule itself but also its analogs, derivatives, variants, and other forms. In certain circumstances, the drug molecule may possess therapeutic activity. In certain circumstances, the drug molecule may possess cytotoxicity.

In this application, the drug molecule may be selected from the following group: V-ATPase inhibitor, Bcl2 inhibitor, MCL1 inhibitor, HSP90 inhibitor, IAP inhibitor, mTOR inhibitor, microtubule stabilizer, microtubule destabilizer, auristatin, dolastatin, maytansinoid, MetAP (methionine aminopeptidase), nuclear export inhibitor of protein CRM1, DPPIV inhibitor, proteasome inhibitor, mitochondrial phosphate transfer reaction inhibitor, protein synthesis inhibitor, CDK2 inhibitor, CDK9 inhibitor, kinesin inhibitor, HDAC inhibitor, DNA damaging agent, DNA alkylating agent, DNA intercalating agent, DNA minor groove binder, DHFR inhibitor, nucleoside analog, HDAC inhibitor, anthracycline, NAMPT inhibitor, SN-38 glucuronide, etoposide phosphate, nitrogen mustard, proteasome inhibitor, cytokine, Tubulysin B analog, and Toll-like receptor agonist.

In this application, the drug molecule may be a DNA topoisomerase I inhibitor. In this application, the drug molecule may be a camptothecin derivative.

In this application, Tubulysin B is a cytotoxic tubulysin that inhibits tubulin polymerization, leading to cell cycle arrest and apoptosis. The CAS number of Tubulysin B is 205304-87-6. In this application, MMAE, i.e., Monomethyl auristatin E, is a synthetic derivative of dolastatin 10 and acts as a potent mitotic inhibitor by inhibiting tubulin polymerization. The CAS number of MMAE is 474645-27-7. In this application, SN38 is the active metabolite of the topoisomerase I inhibitor irinotecan. Its CAS number is 119577-28-5. In the present application, the drug may be SN38 or MMAE.

In the present application, the drug molecule may be selected from microtubule disrupting agents, DNA damaging drugs, apoptosis inducers, telomestatin and its analogs, amatoxin, nicotinamide phosphoribosyltransferase, and calicheamicin. For example, the microtubule disrupting agent may be auristatin, maytansine derivative, tubulysin, cryptophycin, and/or antimitotic EG5 inhibitor. For example, the auristatin may comprise MMAE and/or MMAF. For example, the maytansine derivative may comprise DM1, DM2, DM3, and/or DM4. For example, the DNA damaging drug may be selected from: pyrrolobenzodiazepine drugs, indolinobenzodiazepine drugs, duocarmycin, camptothecin and its derivatives, and calicheamicin. For example, the pyrrolobenzodiazepine drug may comprise pyrrolo[2,1-c][1,4]benzodiazepine (PBD). For example, camptothecin and its derivatives include SN-38, and/or DXd/DX8951. For example, the apoptosis inhibitor may comprise a Bcl-xL inhibitor.

In the present application, each of the drug molecules may comprise analogs or derivatives of the drug molecule. The analogs or derivatives of the drug molecule can still retain the function of the drug molecule.

### Immunoconjugate

In another aspect, the present application provides an immunoconjugate comprising an antigen-binding protein targeting LILRB4 and a drug molecule. In the present application, the antigen-binding protein targeting LILRB4 and the drug molecule may be linked via a linker.

In the present application, the immunoconjugate may have the structure shown in Formula 1: Ab-(Q)ₙ (Formula 1), wherein Ab represents the antigen-binding protein targeting LILRB4, Q represents the moiety containing the drug molecule linked to Ab, and n is an integer from 1 to 10.

For example, in Formula 1, Q may be selected from one or more of the following group: 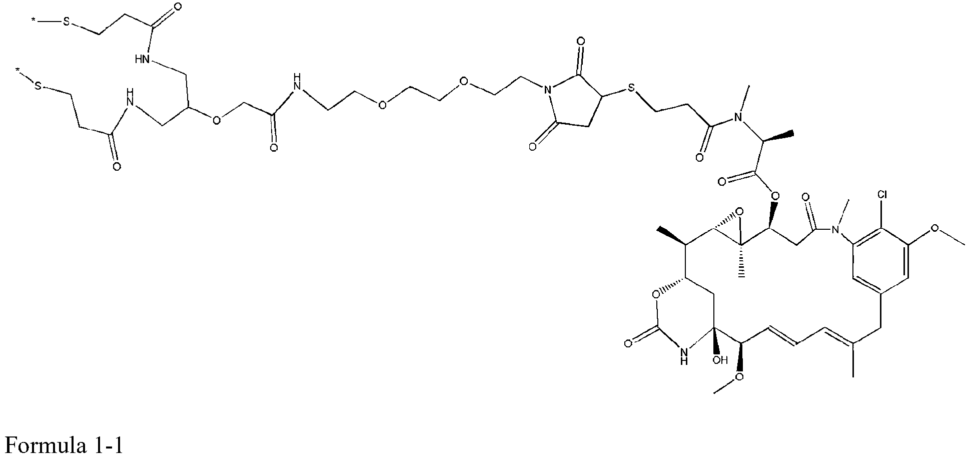 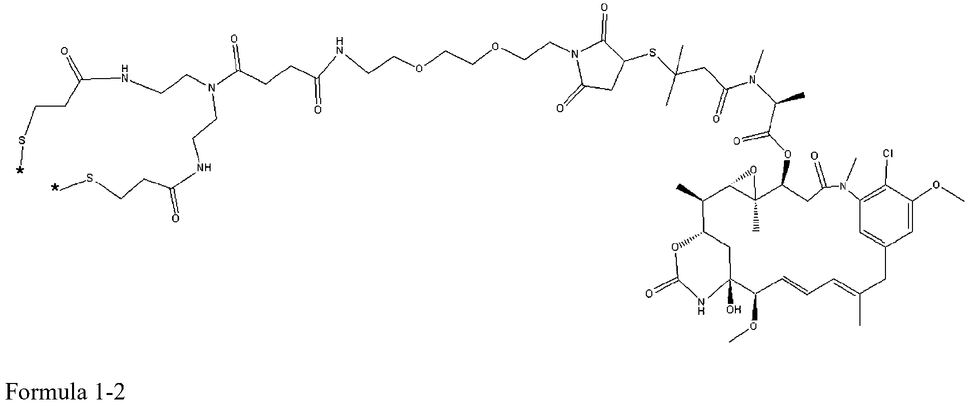 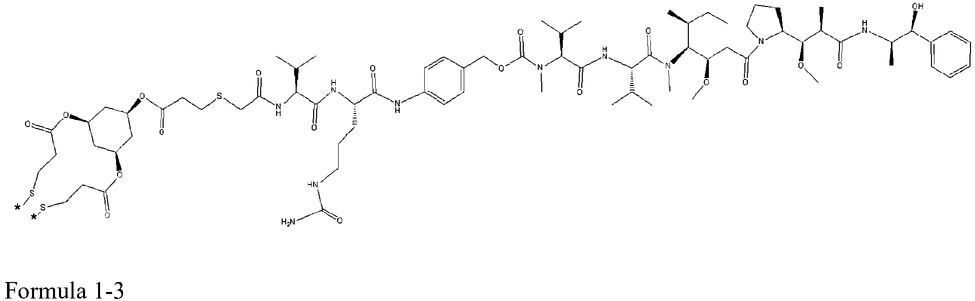 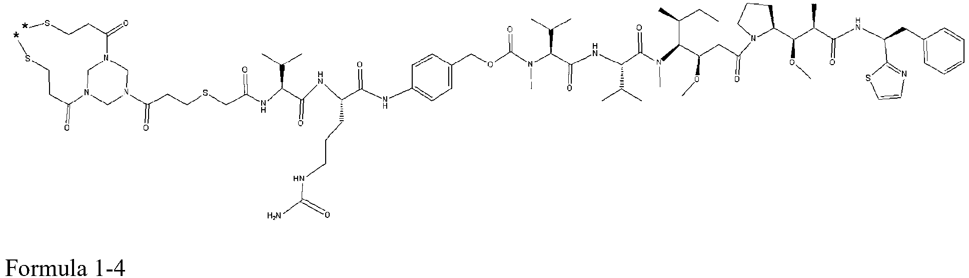 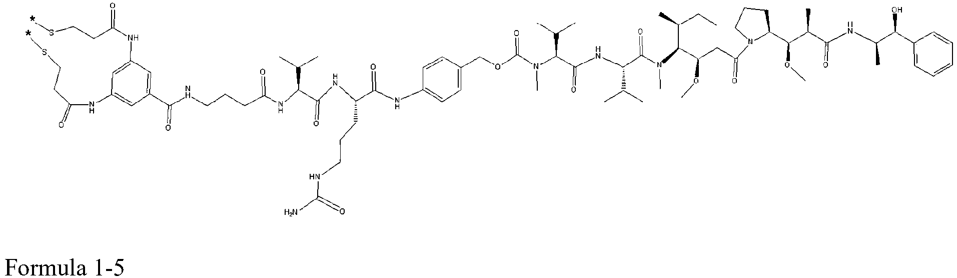 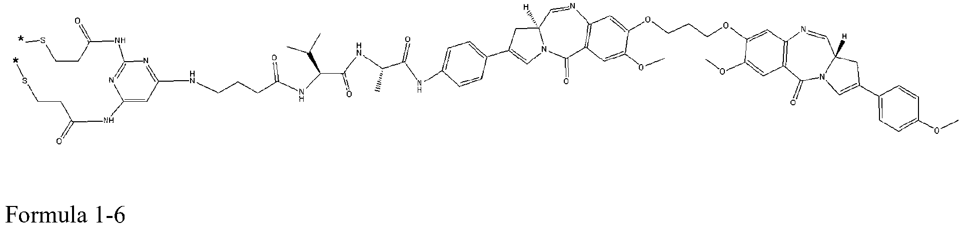 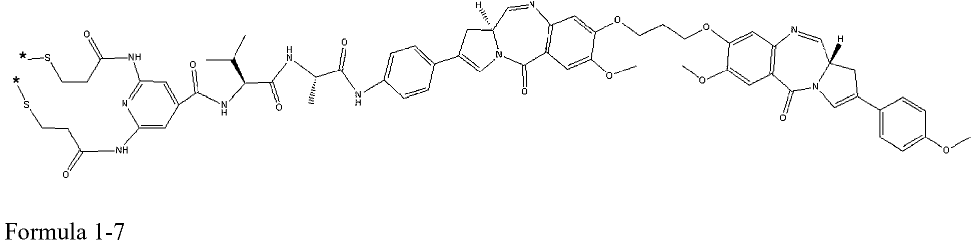 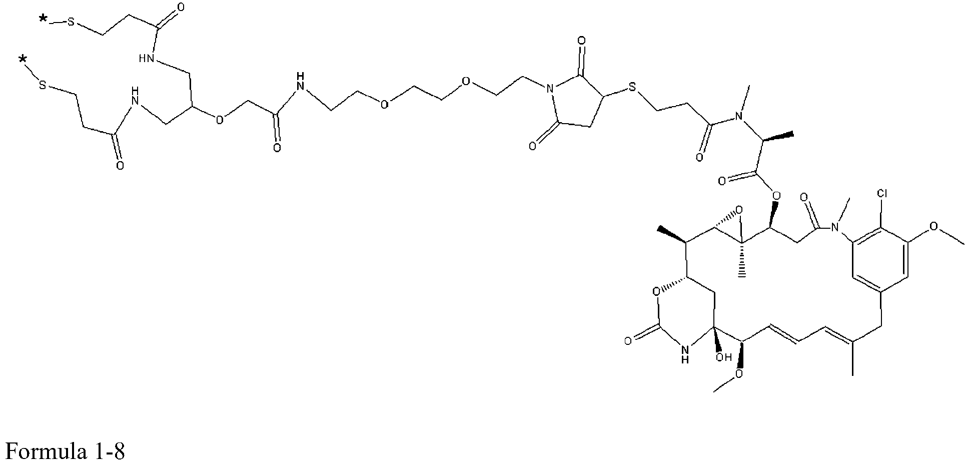 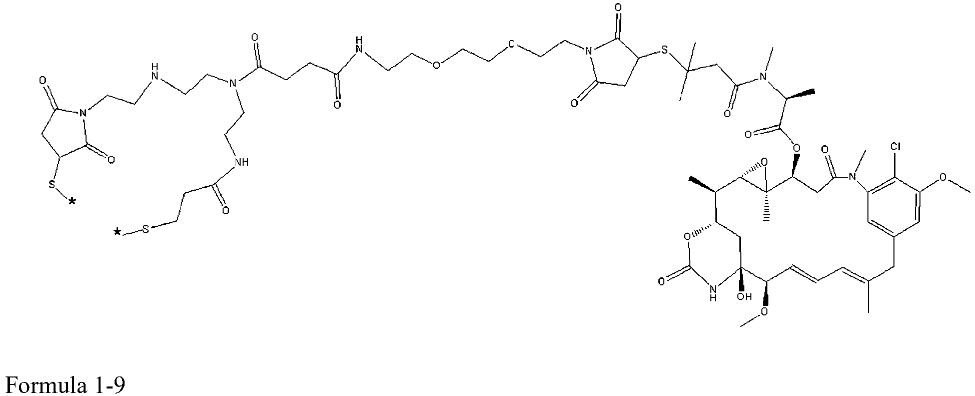 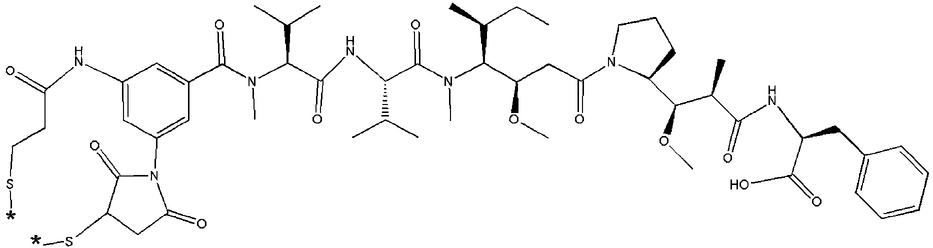 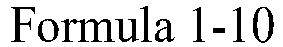 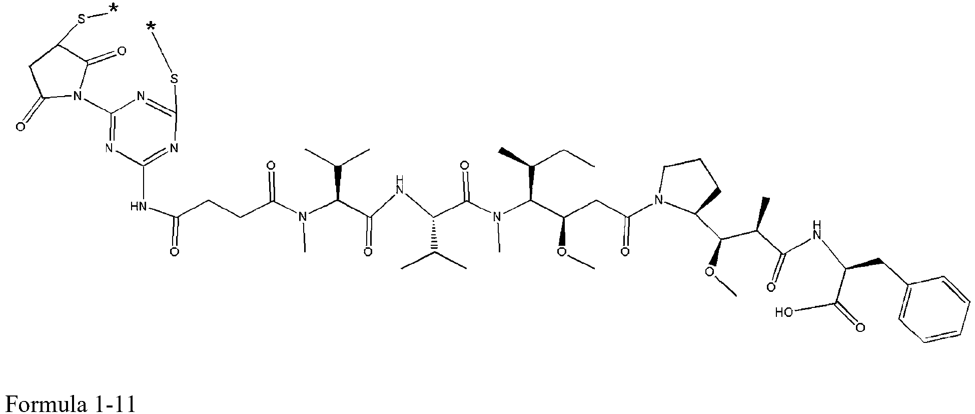 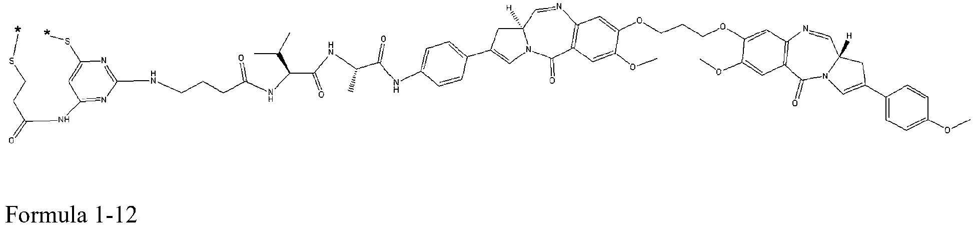 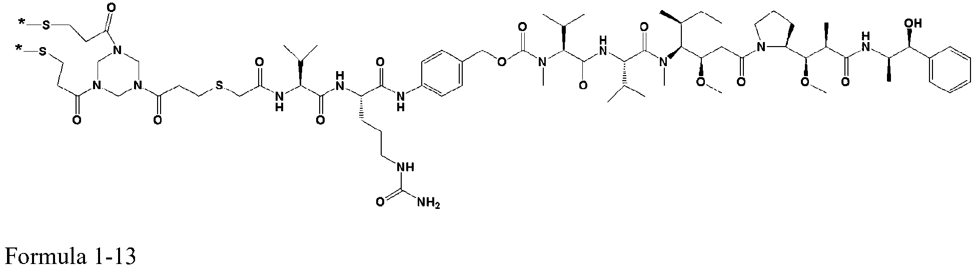 wherein * is the attachment site of Q to the antibody molecule.

In certain specific embodiments, Q in Formula 1 may be selected from the group consisting of: -MC-MK4827, -MC-MK4827&MMAF, -MC-MMAF, -MC-MMAF, -MC-MMAF&SN38, -MC-SN38, -MC-2MMAF, -MC-MC-VC-PAB-MMAE, and -PY-VC-PAB-MMAE.

In the present application, the antigen-binding protein targeting LILRB4 in the immunoconjugate may be linked directly or indirectly to the drug molecule. For example, they may be linked via the linker described herein.

In the present application, the immunoconjugate may have the structure shown in Formula 2: Ab-(L1)ₐ-(L2)_{b}-D (Formula 2), wherein Ab represents the antigen-binding protein targeting LILRB4 as described herein; L1 represents the linker attached to Ab, L2 represents the linker attached to D, a and b are each independently selected from 0-10, and D represents the drug molecule. For example, L1 may be a group or molecule capable of reacting with a thiol group, azide group, or amide group. For example, L2 may comprise one or more of the following: C1-C9 alkyl, C2-C9 alkenyl, C2-C9 alkynyl, C6-C24 aryl, C6-C15 heteroaryl, C3-C9 cycloalkyl, C3-C9 heterocyclyl, polyethylene glycol, O, S, alkylamino, carbonyl, carboxyl, sulfonic acid group, Val-Cit-PABC, VA-PABC, GGFG, Val-Lys(Ac)-PAB, Phe-Lys-PAB, D-Val-Leu-Lys, Gly-Gly-Arg, Ala-Ala-Asn-PAB, and Ala-PAB.

In the present application, Ab may be a complete antibody structure (e.g., a complete IgG antibody structure comprising two identical light chains and two identical heavy chains). In the present application, the constant region of the light chain, and/or the CH1 and/or hinge region of the heavy chain constant region of Ab, may contain attachment sites capable of linking to L1. For example, the constant region of the light chain and the CH1 and hinge regions of the heavy chain constant region of Ab may contain attachment sites capable of linking to L1.

In the present application, the attachment site may comprise cysteine. In the present application, the attachment site may possess a thiol group in a connectable state. In the present application, the number of attachment sites may be 8. In the present application, the attachment sites may include: 2 attachment sites located in the constant region of the light chain, 2 attachment sites located in the CH1 region of the heavy chain constant region, and 4 attachment sites located in the hinge region.

In the present application, the attachment site may comprise a group capable of linking to L1 after deglycosylation modification. In the present application, the attachment site may comprise a group capable of linking to L1 after glycosylation modification.

In the present application, a may be an integer selected from 0-10, for example, 0, 1, 2, 3, 4, 5, 6, 7, 8, 9, or 10.

In the present application, b may be an integer selected from 0-10, for example, 0, 1, 2, 3, 4, 5, 6, 7, 8, 9, or 10.

In certain cases, the linker in the immunoconjugate may, for example, maintain the specific binding properties of the antigen-binding protein targeting LILRB4; participate in the delivery of the drug molecule; and/or maintain the therapeutic effect of the drug (e.g., cytotoxic effect).

In the present application, the immunoconjugate may have the structure shown in Formula 1: Ab-(Q)ₙ (Formula 1), wherein Ab represents the antigen-binding protein targeting LILRB4, Q represents the moiety containing the drug molecule linked to Ab, and n is an integer from 1 to 10.

For example, in Formula 1, Q may be selected from one or more of the following group: 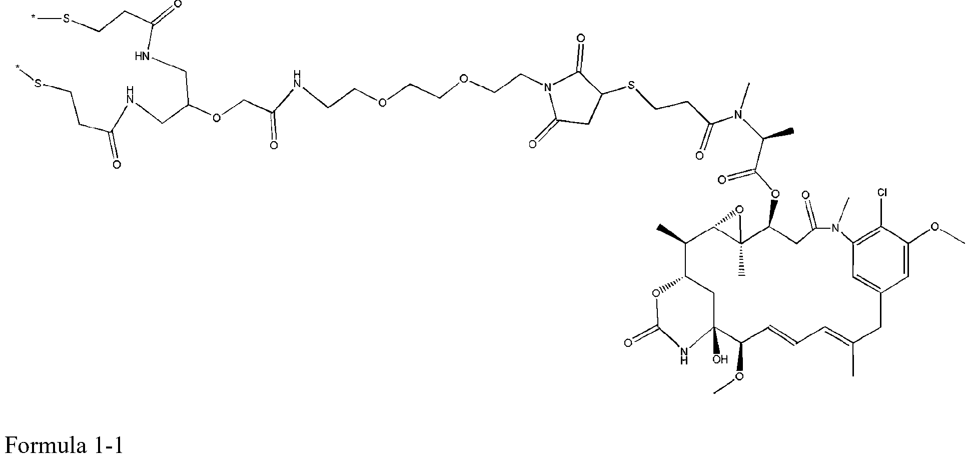 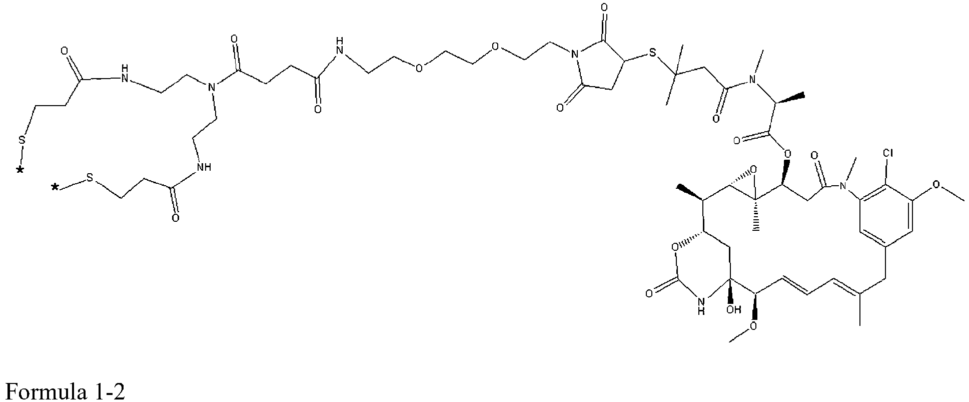 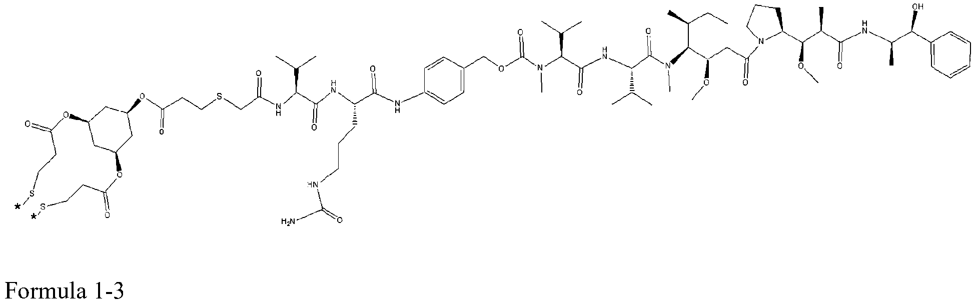 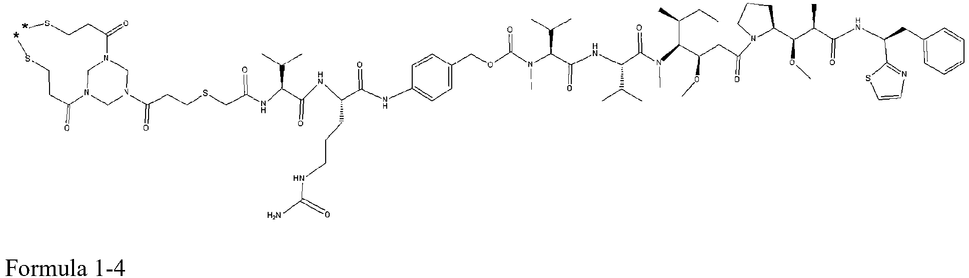 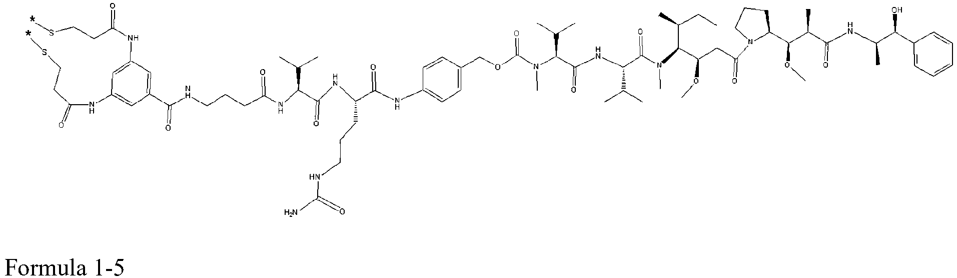 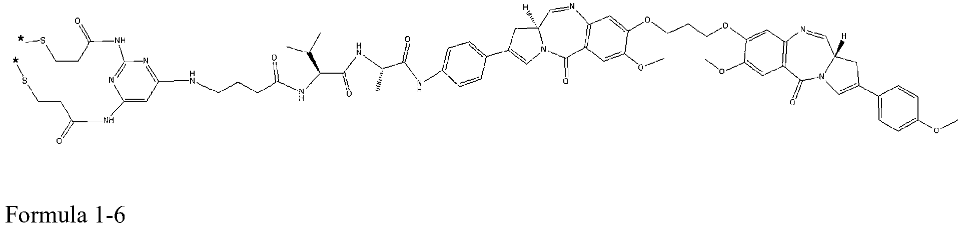 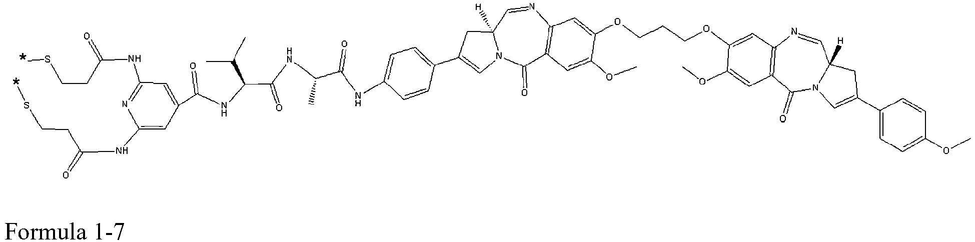 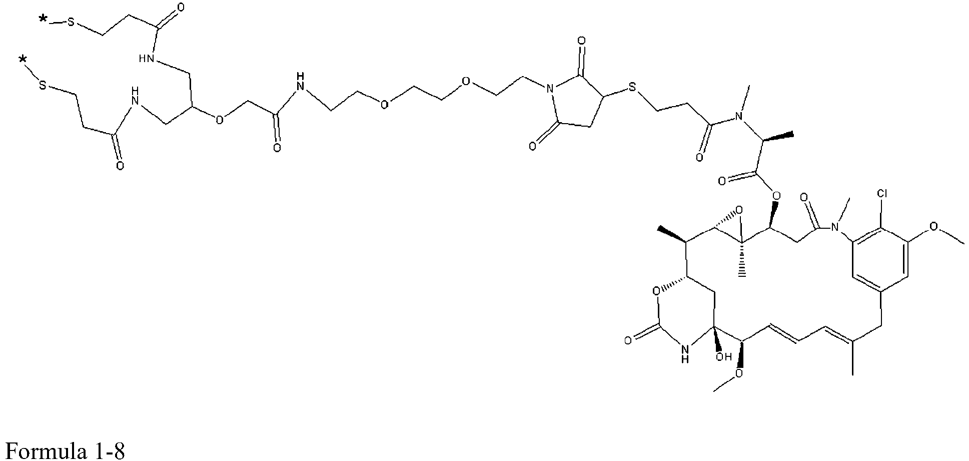 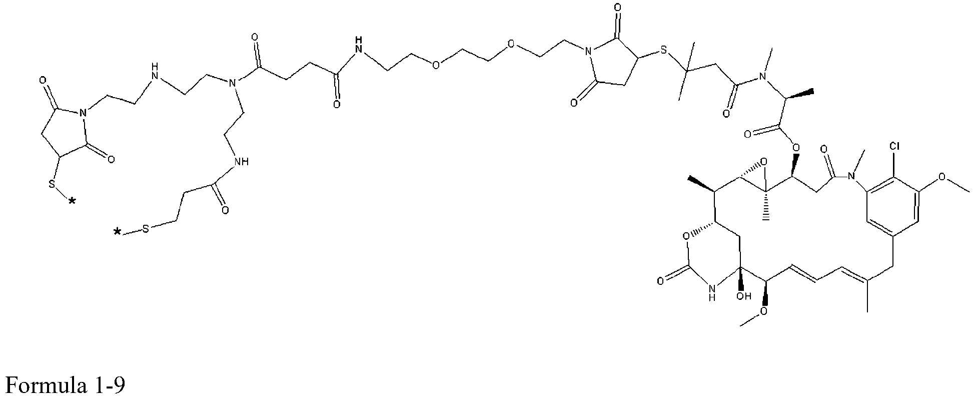 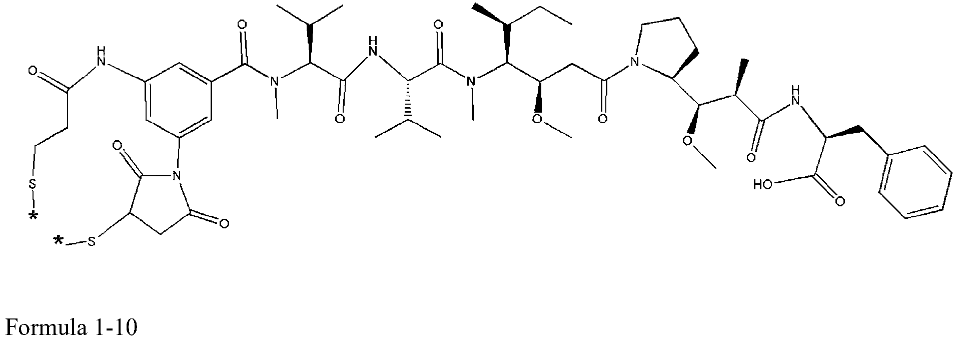 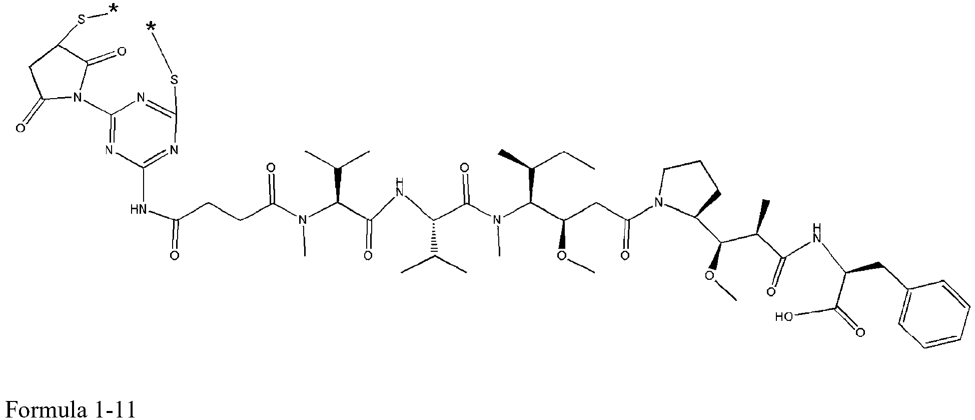 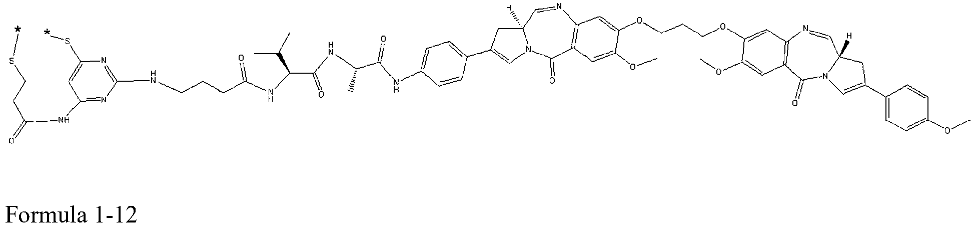 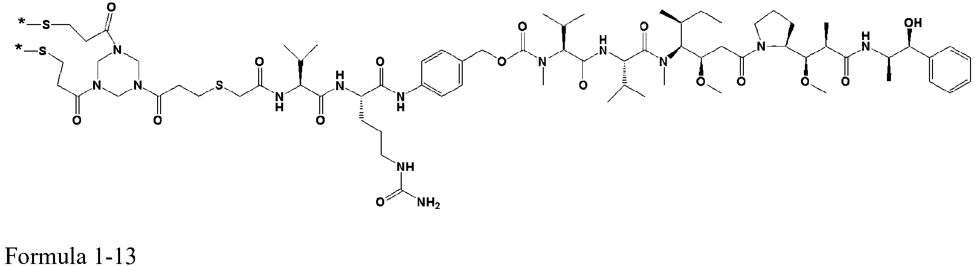 wherein * is the attachment site of Q to the antibody molecule.

In certain specific embodiments, Q in Formula 1 may be selected from the group consisting of: -MC-MK4827, -MC-MK4827&MMAF, -MC-MMAF, -MC-MMAF, -MC-MMAF&SN38, -MC-SN38, -MC-2MMAF, -MC-MC-VC-PAB-MMAE, and -PY-VC-PAB-MMAE.

In certain specific embodiments, the immunoconjugate may have the following molecular formula: 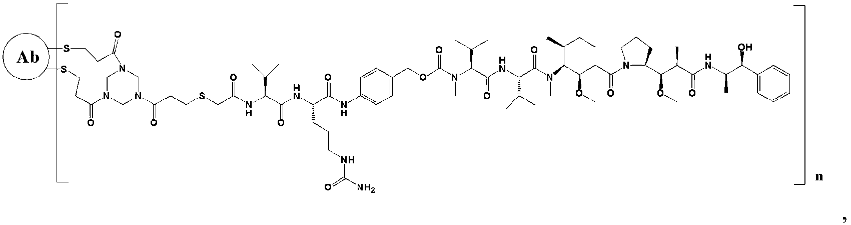 wherein n is selected from 1-8, preferably 1-6, for example, n is 1, 2, 3, 4, 5, 6, 7, or 8.

### Pharmaceutical composition, use

In another aspect, the present application provides a pharmaceutical composition comprising the immunoconjugate and, optionally, a pharmaceutically acceptable carrier.

In the present application, the pharmaceutically acceptable carrier may include buffers, antioxidants, preservatives, low molecular weight polypeptides, proteins, hydrophilic polymers, amino acids, sugars, chelating agents, counterions, metal complexes, and/or nonionic surfactants, etc.

In the present application, the pharmaceutical composition may be formulated for oral administration, intravenous administration, intramuscular administration, local administration at the tumor site, inhalation, rectal administration, vaginal administration, transdermal administration, or administration via a subcutaneous depot. For example, for injectable formulations, the pharmaceutical composition may be prepared as single-dose ampoules or multi-dose container unit dosage forms. The pharmaceutical composition may also be formulated as solutions, suspensions, tablets, pills, capsules, and long-acting formulations.

In the present application, the pharmaceutical composition may further include one or more drugs selected from the group consisting of: anthracycline topoisomerase inhibitors, daunorubicin, cytarabine nucleoside metabolism inhibitors, combination of daunorubicin and cytarabine, liposomal injection of daunorubicin and cytarabine, Vyxeos, all-trans retinoic acid (ATRA), arsenic, arsenic trioxide, histamine hydrochloride, dihydrochloride histamine (Ceplene), interleukin-2, gemtuzumab ozogamicin (Mylotarg), clofarabine, farnesyl transferase inhibitors, decitabine, IDH1 inhibitors, IDH2 inhibitors, enasidenib, Idhifa, IDO inhibitors, epacadostat, platinum complex derivatives, oxaliplatin, kinase inhibitors, tyrosine kinase inhibitors, PI3 kinase inhibitors, BTK inhibitors, antibodies binding tumor antigens such as PD-1 antibody, PD-L1 antibody, anti-CTLA-4 antibody, LAG3 antibody, ICOS antibody, 4-1BB antibody, TIGIT antibody, TIM3 antibody, and CD47 antibody, angiogenesis inhibitors, antibodies binding T lymphocyte surface markers, antibodies binding tumor stromal cells, antibodies binding myeloid-derived suppressor cells or NK cell surface markers, alkylating agents, nitrosourea agents, antimetabolites, antitumor antibiotics, plant-derived alkaloids, topoisomerase inhibitors, microtubule inhibitors, hormone therapy drugs, hormone antagonists, aromatase inhibitors, P-glycoprotein inhibitors, radiotherapeutic agents, and nucleic acid drugs.

In another aspect, the present application provides a drug combination comprising the immunoconjugate and an immune checkpoint inhibitor. The immune checkpoint includes PD-1, PD-L1, GITR, and/or CTLA-4. The immune checkpoint inhibitor includes one or more selected from the group consisting of: PD-1 antibody, PD-L1 antibody, CTLA-4 antibody, and GITR antibody.

In another aspect, the present application provides the immunoconjugate and/or the pharmaceutical composition for use in preventing and/or treating diseases and/or conditions.

In another aspect, the present application provides a method for preventing, diagnosing, and/or treating diseases and/or conditions, comprising administering an effective amount of the immunoconjugate and/or the pharmaceutical composition to a subject in need thereof.

In the present application, the administration may be performed by various routes, for example, intravenously, intratumorally, intraperitoneally, subcutaneously, intramuscularly, topically, or intradermally.

In the present application, the disease and/or condition may include diseases or conditions associated with LILRB4 signal transduction and/or LILRB4 expression.

In the present application, the disease and/or condition may include tumors.

In the present application, the disease and/or condition may include LILRB4-positive diseases and/or conditions.

In the present application, the tumor may include solid tumors and/or hematological tumors.

In the present application, the solid tumor may be selected from one or more of the following: breast cancer, melanoma, colon cancer, lung cancer, renal cancer, head and neck cancer, gastric cancer, pancreatic cancer, lymphoma, non-small cell lung cancer, leukemia, ovarian cancer, nasopharyngeal carcinoma, breast cancer, endometrial cancer, colon cancer, rectal cancer, gastric cancer, bladder cancer, lung cancer, bronchial cancer, bone cancer, prostate cancer, pancreatic cancer, liver and bile duct cancer, esophageal cancer, renal cancer, thyroid cancer, head and neck cancer, testicular cancer, glioblastoma, astrocytoma, melanoma, myelodysplastic syndrome, and sarcoma; wherein the hematological malignancy is selected from multiple myeloma, leukemia, lymphoma, myelodysplastic syndrome, myelodysplastic syndrome, and myeloproliferative neoplasm.

The leukemia is selected from acute lymphoblastic (lymphoblastic) leukemia, acute myeloid leukemia (AML), myeloid leukemia, chronic lymphocytic leukemia, multiple myeloma, plasma cell leukemia, chronic myeloid leukemia, acute promyelocytic leukemia (APL) or M3 AML, acute myelomonocytic leukemia or M4 AML, and acute monocytic leukemia or M5 AML, wherein preferably the acute myeloid leukemia is M4 or M5 type acute myeloid leukemia; and preferably the chronic myeloid leukemia is chronic myelomonocytic leukemia. The lymphoma is selected from Hodgkin lymphoma and non-Hodgkin lymphoma, including B-cell lymphoma, diffuse large B-cell lymphoma, follicular lymphoma, mantle cell lymphoma, marginal zone B-cell lymphoma, T-cell lymphoma, and Waldenström macroglobulinemia. The sarcoma is selected from osteosarcoma, Ewing sarcoma, leiomyosarcoma, synovial sarcoma, soft tissue sarcoma, angiosarcoma, liposarcoma, fibrosarcoma, rhabdomyosarcoma, and chondrosarcoma.

Without being bound by any theory, the examples below are provided solely to illustrate the immunoconjugates, preparation methods, and uses of the present application, and are not intended to limit the scope of the invention disclosed herein.

### Examples

### Example 1 Evaluation of binding activity of LILRB4 hybridoma antibodies

Recombinant LILRB4 protein (Human LILRB4/CD85k/ILT3 Protein (ECD, His Tag), Sino Biological) was coated onto ELISA plate wells and incubated overnight at 4°C. After washing with PBST, blocking buffer containing 10% fetal bovine serum was added and incubated at 37°C for 1 hour. Six anti-LILRB4 hybridoma antibodies at a starting concentration of 20 µg/mL and serial dilutions were added and incubated at 37°C for 1 hour. After washing with PBST, horseradish peroxidase (HRP)-conjugated goat anti-human IgG secondary antibody (Goat anti-Human IgG (H+L) Cross-Adsorbed Secondary Antibody, HRP, Invitrogen) was added and incubated at 37°C for 30 minutes. The plates were washed five times with PBST. Then, 100 µL of TMB substrate (eBioscience) was added to each well and incubated in the dark at room temperature (20±5°C) for 2-3 minutes. Subsequently, 100 µL of 2N H₂SO₄ stop solution was added to each well to terminate the reaction, and the OD values were read at 450 nm using a microplate reader to analyze the binding capacity of the anti-LILRB4 hybridoma antibodies to recombinant LILRB4 protein.

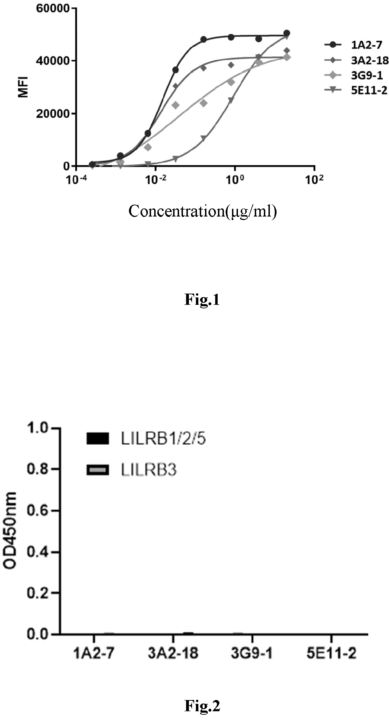

The results are shown in Figure 1. Figure 1 shows that among the four anti-LILRB4 hybridoma antibodies, 1A2-7 and 3A2-18 exhibit stronger binding affinity to LILRB4 than the other antibodies.

The amino acid sequences of 1A2-7, 3A2-18, 3G9-1, and 5E11-2 are listed in the table below, with CDR sequences defined according to the Kabat numbering scheme.

| | 1A2-7 | 3A2-18 | 3G9-1 | 5E11-2 |
|---|---|---|---|---|
| HCDR1 | SEQ ID NO: 1 | SEQ ID NO: 1 | SEQ ID NO: 1 | SEQ ID NO: 27 |
| HCDR2 | SEQ ID NO: 2 | SEQ ID NO: 2 | SEQ ID NO: 2 | SEQ ID NO: 28 |
| HCDR3 | SEQ ID NO: 3 | SEQ ID NO: 3 | SEQ ID NO: 20 | SEQ ID NO: 29 |
| VH | SEQ ID NO: 8 | SEQ ID NO: 19 | SEQ ID NO: 22 | SEQ ID NO: 33 |
| LCDR1 | SEQ ID NO: 9 | SEQ ID NO: 9 | SEQ ID NO: 23 | SEQ ID NO: 34 |
| LCDR2 | SEQ ID NO: 10 | SEQ ID NO: 10 | SEQ ID NO: 10 | SEQ ID NO: 35 |
| LCDR3 | SEQ ID NO: 11 | SEQ ID NO: 11 | SEQ ID NO: 24 | SEQ ID NO: 36 |
| VL | SEQ ID NO: 16 | SEQ ID NO: 16 | SEQ ID NO: 26 | SEQ ID NO: 40 |

### Example 2 Cross-binding activity assay of LILRB4 family proteins

Inhibitory leukocyte immunoglobulin-like receptor subfamily B (LILRB) proteins, including LILRB4 protein (Human LILRB4/CD85k/ILT3 Protein (ECD, His Tag), Sino Biological), LILRB1 protein (LILRB1 Protein, Human, Recombinant (ECD, His Tag), Sino Biological), LILRB2 protein (ILT4 Protein, Human, Recombinant (ECD, His Tag), Sino Biological), LILRB3 protein (LILRB3 Protein, Human, Recombinant (ECD, His Tag), Sino Biological), and LILRB5 protein (LILRB5/CD85c Protein, Human, Recombinant (ECD, His Tag), Sino Biological), were coated onto ELISA plates and incubated overnight at 4°C. After washing with PBST, 10% fetal bovine serum was added to each well and plates were blocked at 37°C for 1 hour. Anti-LILRB4 antibody at a concentration of 10 µg/mL (100 µL per well) was added and incubated at 37°C for 1 hour. Following PBST washes, horseradish peroxidase (HRP)-conjugated goat anti-human IgG secondary antibody (Goat anti-Human IgG (H+L) Cross-Adsorbed Secondary Antibody, HRP, Invitrogen) was added and incubated at 37°C for 30 minutes. Plates were washed five times with PBST, then 100 µL of TMB substrate (eBioscience) was added to each well and incubated in the dark at room temperature (20±5°C) for 2-3 minutes. The reaction was stopped by adding 100 µL of 2N H₂SO₄ stop solution per well, and absorbance was measured at 450 nm using a microplate reader to analyze the binding capability of the anti-LILRB4 antibody to other LILRB family proteins besides its specific antigen.

The results are shown in Figure 2. Figure 2 shows that none of the four tested antibodies bound to other LILRB family proteins.

### Example 3 Biological activity evaluation of LILRB4 antibodies

### Example 3-1 Evaluation of internalization activity of LILRB4 antibodies

Human monocytic leukemia cells THP-1 were harvested and aliquoted into 1.5 mL EP tubes at 1×10⁶ cells per tube. Different concentrations of hybridoma-derived anti-LILRB4 antibodies and isotype control antibody HG1k were added, and cells were incubated on ice in the dark for 30 minutes. After washing with FACS buffer, PE-conjugated goat anti-human IgG Fc secondary antibody (Goat Anti-Human IgG Fc Secondary Antibody, Invitrogen) was added, and cells were incubated on ice in the dark for another 30 minutes. Cells were washed twice with FACS buffer, resuspended in complete culture medium, and equally divided into two groups, which were incubated at either 4°C or 37°C for 2 hours, with gentle mixing every 30 minutes. After two additional washes with FACS buffer, 400 µL of 1% paraformaldehyde fixation solution (Solarbio) was added to each tube to fix the cells. Following thorough mixing, samples were analyzed by flow cytometry to measure the mean fluorescence intensity (MFI) of PE fluorescence in each group. Internalization efficiency (%) was calculated using the formula: [(Total surface MFI at 4°C - Total surface MFI at 37°C) / Total surface MFI at 4°C] × 100%, to evaluate the internalization efficiency of the antibodies after binding to LILRB4-positive tumor cells THP-1.

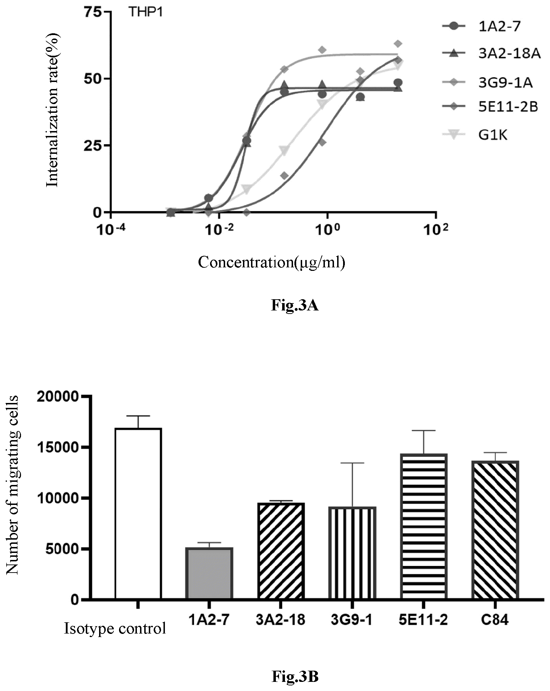

The results are shown in Figure 3A. Figure 3A shows that hybridoma-derived anti-LILRB4 antibodies 3G9-1 (i.e., 3G9-1A in the figure), 1A2-7, and 3A2-18 (i.e., 3A2-18A in the figure) exhibited high internalization efficiency after binding to LILRB4-positive tumor cells THP-1.

### Example 3-2 Effect of LILRB4 antibodies on migration of THP-1 cells

Human monocytic leukemia cells THP-1, serum-starved for 24 hours, were collected and seeded into 24-well cell culture plates. LILRB4 antibodies and positive control antibody C84 (disclosed in patent WO2016144728A3) were added to a final concentration of 10 µg/mL, and cells were incubated at 37°C in a 5% CO₂ incubator for 1 hour. A mixture of 100 µL of cells and antibody was loaded into the upper chamber of a Transwell insert (6.5 mm Transwell^{®} with 8.0 µm Pore Polycarbonate Membrane Insert, Corning), and 700 µL of complete culture medium was added to the lower chamber. Cells were incubated at 37°C in a 5% CO₂ incubator for 24 hours. Then, 100 µL of solution from the lower chamber was transferred to a white clear-bottom 96-well plate (96-well Black/Clear and White/Clear Bottom Polystyrene Microplates, Corning), followed by addition of 100 µL of CellTiter-Lumi^{™} Luminescent Cell Viability Assay Reagent (Beyotime). After incubation in the dark at room temperature for 10 minutes, luminescence was measured using a multi-mode microplate reader. A standard curve was generated using serially diluted THP-1 cells and their corresponding relative luminescence values to calculate the number of migrated cells in the lower chamber, thereby evaluating the inhibitory effect of LILRB4 antibodies on THP-1 cell migration.

As shown in Figure 3B, antibodies 1A2-7, 3A2-18, and 3G9-1 exhibited stronger inhibition of THP-1 cell migration than the positive control antibody C84, while the inhibitory effect of 5E11-2 was similar to that of the positive control antibody C84.

### Example 4-1 Antibody humanization

Antibodies were humanized using the standard CDR grafting method. BLAST searches were performed against human heavy and light chain variable region sequences, and 3 or 4 sequences were selected from each as acceptor frameworks for humanization. Heavy chain and light chain CDR1, CDR2, and CDR3 regions were grafted into three different heavy chain frameworks (H1-H4) and two different light chain frameworks (L1-L2). Subsequently, expression levels and antigen-binding capabilities of four different antibody combinations were evaluated in CHO-S cells.

| Humanized antibody name | Heavy chain | Light chain |
|---|---|---|
| RB4-G1-018 | SEQ ID NO: 46 | SEQ ID NO: 52 |
| RB4-G1-019 | SEQ ID NO: 56 | SEQ ID NO: 52 |
| RB4-G1-020 | SEQ ID NO: 59 | SEQ ID NO: 52 |
| RB4-G1-022 | SEQ ID NO: 46 | SEQ ID NO: 62 |

### Example 4-2 Evaluation of binding activity of humanized antibodies (ELISA)

LILRB4 protein (Human LILRB4/CD85k/ILT3 Protein (ECD, His Tag), Sino Biological) was coated onto ELISA plates and incubated overnight at 4°C. After washing with PBST, 10% fetal bovine serum was added to each well and plates were blocked at 37°C for 1 hour. Various concentrations of the 3A2-18A humanized antibody (corresponding to 3A2-18) and positive control antibody 128-3 were added and incubated at 37°C for 1 hour. Following PBST washes, horseradish peroxidase (HRP)-conjugated goat anti-human IgG secondary antibody (Goat anti-Human IgG (H+L) Cross-Adsorbed Secondary Antibody, HRP, Invitrogen) was added and incubated at 37°C for 30 minutes. Plates were washed five times with PBST, then 100 µL of TMB substrate (eBioscience) was added to each well and incubated in the dark at room temperature (20±5°C) for 2-3 minutes. The reaction was stopped by adding 100 µL of 2N H₂SO₄ stop solution per well, and absorbance was measured at 450 nm using a microplate reader to analyze the binding capability of the 3A2-18A humanized antibody to LILRB4.

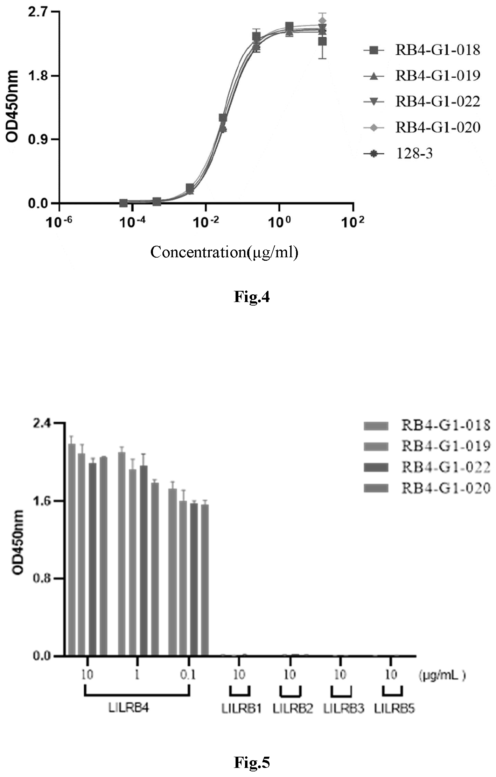

The results are shown in Figure 4. Figure 4 shows that the binding affinities of the four 3A2-18A humanized antibodies to LILRB4 were comparable to each other and similar to that of the positive control antibody 128-3 (whose heavy chain amino acid sequence is shown in SEQ ID NO: 65 and light chain amino acid sequence in SEQ ID NO: 66).

### Example 5 Affinity determination of humanized antibodies binding to LILRB4 protein

100 µL of NHS and 100 µL of EDC were mixed and centrifuged at 12,000 rpm for 5 minutes. A certain volume of antibody was taken and diluted according to the pre-determined coupling ratio to a final volume of 150 µL. Additionally, 100 µL of ethanolamine HCl was prepared. All reagents were equilibrated to room temperature (~25°C) and centrifuged at high speed for 5 minutes. The three reagents were placed on the sample rack, and the injection flow rate was set to 10 µL/min. NHS/EDC, sample, and ethanolamine-HCl were injected at volumes of 100 µL, 100 µL, and 60 µL, respectively. The antigen was diluted into seven concentrations using an appropriate buffer, centrifuged, and then injected to monitor the antigen-antibody binding process. The assay showed that the affinity of the 3A2-18A humanized antibody for human LILRB4 was in the 10⁻⁹ M range, slightly lower than that of the parental antibody but similar to that of the positive control antibody 128-3. The results are shown in Table 1.

**Table 1. Binding affinity of 3A2-18A humanized antibodies to LILRB4 protein determined by Biacore T200**

| Ligand | Analyte | ka (1/Ms) | kd (1/s) | KD (M) |
|---|---|---|---|---|
| 3A2-18A | Human LILRB4 | 6.95E+06 | 2.24E-03 | 3.22E-10 |
| RB4-G1-018 | Human LILRB4 | 1.54E+06 | 4.33E-03 | 2.81E-09 |
| RB4-G1-019 | Human LILRB4 | 1.24E+06 | 3.43E-03 | 2.77E-09 |
| RB4-G1-022 | Human LILRB4 | 1.80E+06 | 4.18E-03 | 2.33E-09 |
| RB4-G1-020 | Human LILRB4 | 2.53E+06 | 8.34E-03 | 3.29E-09 |
| 128-3 | Human LILRB4 | 1.72E+06 | 4.22E-03 | 2.46E-09 |

### Example 6 Cross-binding activity assay of humanized antibody family proteins

Inhibitory leukocyte immunoglobulin-like receptor subfamily B (LILRB) proteins, including LILRB4 protein (Human LILRB4/CD85k/ILT3 Protein (ECD, His Tag), Sino Biological), LILRB1 protein (LILRB1 Protein, Human, Recombinant (ECD, His Tag), Sino Biological), LILRB2 protein (ILT4 Protein, Human, Recombinant (ECD, His Tag), Sino Biological), LILRB3 protein (LILRB3 Protein, Human, Recombinant (ECD, His Tag), Sino Biological), and LILRB5 protein (LILRB5/CD85c Protein, Human, Recombinant (ECD, His Tag), Sino Biological), were coated onto ELISA plate strips and incubated overnight at 4°C. After washing with PBST, PBS containing 10% fetal bovine serum was added for blocking at 37°C for 1 hour. Then, 100 µL of anti-LILRB4 antibody at a concentration of 10 µg/mL was added to each well (for wells coated with LILRB4 antigen, additional test wells with antibody concentrations of 1 µg/mL and 0.1 µg/mL were included), followed by incubation at 37°C for 1 hour. After PBST washes, horseradish peroxidase (HRP)-conjugated goat anti-human IgG (H+L) cross-adsorbed secondary antibody (Goat anti-Human IgG (H+L) Cross-Adsorbed Secondary Antibody, HRP, Invitrogen) was added and incubated at 37°C for 30 minutes. The plates were washed five times with PBST, then 100 µL of TMB substrate (eBioscience) was added to each well and incubated in the dark at room temperature (20±5°C) for 2-3 minutes. Subsequently, 100 µL of 2N H₂SO₄ stop solution was added to terminate the reaction, and the OD values were measured at 450 nm using a microplate reader to analyze the binding capability of anti-LILRB4 antibodies to other LILRB subfamily proteins besides the specific antigen.

The results are shown in Figure 5. Figure 5 shows that the four tested anti-LILRB4 humanized antibodies derived from 3A2-18A bind only to LILRB4 protein among the LILRB subfamily proteins and do not bind to any other proteins within the same subfamily.

### Example 7 Evaluation of internalization activity of humanized antibodies

Human monocytic leukemia cells THP-1 were harvested and aliquoted into 1.5 mL EP tubes at 1×10⁶ cells per tube. Different concentrations of hybridoma-derived anti-LILRB4 antibodies and isotype control antibody HG1k were added, and the tubes were incubated on ice in the dark for 30 minutes. After washing with FACS buffer, PE-conjugated goat anti-human IgG Fc secondary antibody (Goat Anti-Human IgG Fc Secondary Antibody, Invitrogen) was added, followed by incubation on ice in the dark for another 30 minutes. Cells were washed twice with FACS buffer, then resuspended in complete culture medium and equally divided into two groups, which were incubated at either 4°C or 37°C for 2 hours, with gentle mixing every 30 minutes. After two additional washes with FACS buffer, 400 µL of 1% paraformaldehyde fixation solution (Solarbio) was added to each tube to fix the cells. Following thorough mixing, the mean fluorescence intensity (MFI) of PE fluorescence in each group was measured by flow cytometry. Internalization efficiency (%) was calculated using the formula: [Total surface MFI (4°C) - Total surface MFI (37°C)] / Total surface MFI (4°C) × 100%, to evaluate the internalization efficiency of antibodies upon binding to LILRB4-positive tumor cells THP-1.

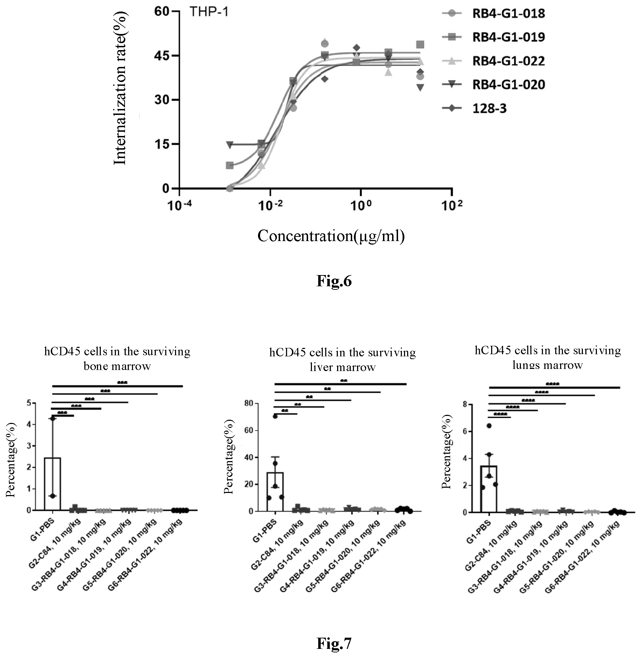

The results are shown in Figure 6. Figure 6 shows that the 3A2-18A humanized antibodies are internalized after binding to LILRB4-positive tumor cells THP-1, with internalization efficiencies comparable to those of the positive control antibody 128-3.

### Example 8 Inhibition of migration of LILRB4+ tumors by humanized antibodies

THP1-Luc human monocytic leukemia cells resuspended in PBS at 5×10⁶ cells/0.2 mL/mouse were injected intravenously via the tail vein into B-NDG mice. On the day of inoculation, mice were randomly assigned into six experimental groups (n=5 per group) based on their bioluminescence imaging signals (average signal: 3.6×10⁷ p/s) and body weights (average weight: 23 g). The groups were as follows: G1 (PBS), G2 (C84, 10 mg/kg), G3 (RB4-G1-018, 10 mg/kg), G4 (RB4-G1-019, 10 mg/kg), G5 (RB4-G1-020, 10 mg/kg), and G6 (RB4-G1-022, 10 mg/kg). All mice received intraperitoneal injections on the day of grouping (day 0) and again on day 3 post-grouping. Mice were imaged using an in vivo imaging system prior to dosing (day 0) and on days 7, 14, 21, 25, and at the end of the experiment (day 28) to obtain bioluminescence images and signal intensities for monitoring tumor growth in vivo. Animal body weights were measured twice weekly during the dosing and observation period, and the measurements were recorded.

At the experimental endpoint, mice were weighed and imaged before euthanasia, and survival curves were generated. Flow cytometry was used to analyze the proportion of hCD45⁺ cells (live/dead, hCD45⁺, mCD45⁺) in the liver, lungs, and bone marrow of mice from each group.

As shown in Figure 7, compared to the G1 control group, the percentages of hCD45⁺ cells in the liver, lungs, and bone marrow were significantly reduced in all treatment groups, indicating that test articles C84, RB4-G1-018, RB4-G1-019, RB4-G1-020, and RB4-G1-022 at a dose of 10 mg/kg markedly decreased tumor burden in these organs.

### Example 9 Blocking of LILRB4/APOE binding by humanized LILRB4 antibodies using a jurkat-LILRB4-CHO-CD3-APOE reporter cell line

APOE/TCR activator/CHO cells in logarithmic growth phase were seeded into 96-well plates and cultured overnight at 37°C. The next day, the supernatant was discarded, and the cells were washed twice with PBS. Antibody samples diluted in RPMI 1640 medium were added at 50 µL per well, followed by the addition of logarithmic-phase LILRB4 effector reporter cells. The plates were then incubated at 37°C. After 5 hours, Bright-Glo luciferase reagent was added, and luminescence was measured using a microplate reader. Data from each well were analyzed using Prism GraphPad software.

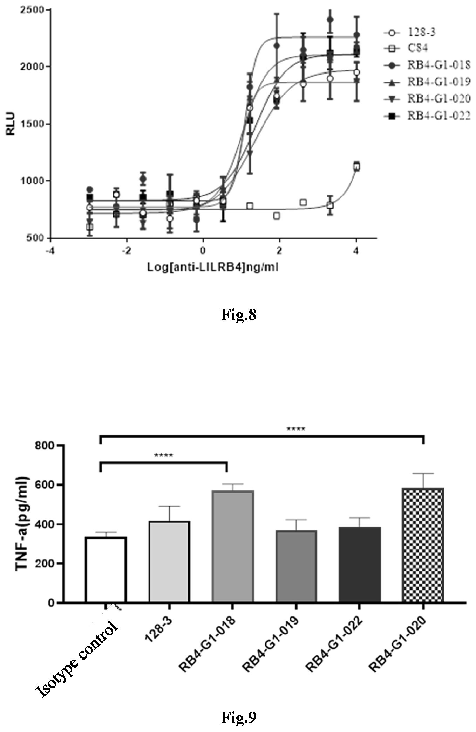

As shown in Figure 8, the humanized antibodies effectively blocked APOE binding to LILRB4, exhibiting stronger blocking activity than the positive control antibodies 128-3 and C84.

### Example 10 Stimulation of CD8+ T cell activation by humanized antibodies

PBMCs were isolated from whole blood of healthy donors, and CD8⁺ T cells were purified using a CD8+ T Cell Isolation Kit (Miltenyi). The purified CD8⁺ T cells were seeded into 96-well clear round-bottom TC-treated microplates (Corning) at 5×10⁴ cells/well. T cell activation beads (Dynabeads^{®} Human T-Activator CD3/CD28/CD137, GIBCO) were added, and the plates were incubated at 37°C in 5% CO₂ for 2 days. Humanized antibodies at a final concentration of 200 µg/mL, positive control antibody 128-3, and isotype control were then added, followed by the addition of human monocytic leukemia cells THP-1 at 5×10⁴ cells/well. The co-cultures were further incubated at 37°C in 5% CO₂ for 5 days. Supernatants were collected and analyzed for TNF-α levels using a Human TNF-α ELISA Kit (ExCell Bio) to evaluate the ability of humanized antibodies to activate CD8⁺ T cells in THP-1/CD8⁺ T cell co-culture assays.

As shown in Figure 9, humanized antibodies RB4-G1-018 and RB4-G1-020 demonstrated stronger CD8⁺ T cell activation capabilities in THP-1/CD8⁺ T cell co-cultures compared to the positive control antibody 128-3.

### Example 11 Tissue distribution of SG2919 in LILRB4-humanized MC38-LILRB4 tumor-bearing mice

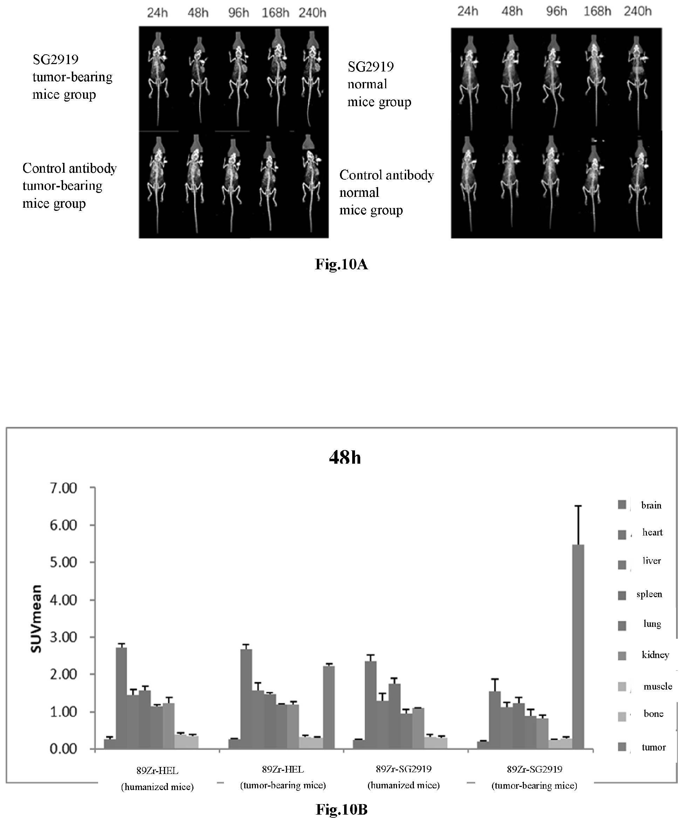

Humanized antibodies were labeled with 89Zr. After a single intravenous injection of 89Zr-SG2919 (corresponding to the humanized antibody RB4-G1-019) or 89Zr-control antibody, in vivo PET/CT imaging was performed at multiple time points to assess tissue distribution of SG2919 and the control antibody in humanized tumor-bearing mice and humanized mice. Group G1-1: Three humanized mice bearing LILRB4-positive tumors received intravenous injection of 89Zr-SG2919 and underwent static Micro PET/CT scans at 24 h, 48 h, 96 h, and 168 h. Group G1-2: Three humanized mice bearing LILRB4-positive tumors received intravenous injection of 89Zr-control antibody and underwent static Micro PET/CT scans at 24 h, 48 h, 96 h, and 168 h. Group G2-1: Three humanized mice (without tumor) received intravenous injection of 89Zr-SG2919 and underwent static Micro PET/CT scans at 24 h, 48 h, 96 h, and 168 h. Group G2-2: Three humanized mice (without tumor) received intravenous injection of 89Zr-control antibody and underwent static Micro PET/CT scans at 24 h, 48 h, 96 h, and 168 h. As shown in Figures 10A and 10B, the radiotracer accumulation of the test article 89Zr-SG2919 in tumors of humanized mice bearing LILRB4-positive tumors was higher than that of the control antibody 89Zr-HEL in the same model. Both 89Zr-SG2919 and 89Zr-HEL reached peak tumor accumulation at 48 h, after which the radioactivity in tumors gradually decreased over time. These results indicate that radiolabeled (e.g., 89Zr-labeled) antibodies of the present application can be used for the diagnosis of LILRB4-positive tumors.

### Example 12 Preparation of SG2919-ADC conjugated with different toxins

The humanized antibody RB4-G1-019, designated SG2919, was conjugated with various small-molecule compounds using standard antibody-drug conjugate (ADC) coupling techniques (e.g., as disclosed in CN107921030A) to generate the following ADCs: SG2919-MC-MK4827&MMAF, SG2919-MC-MMAF&SN38, SG2919-MC-2MMAF, SG2919-MC-MC-VC-PAB-MMAE, and SG2919-PY-VC-PAB-MMAE. Among these, SG2919-MC-MK4827&MMAF is a conjugate simultaneously linked to both MK4827 and MMAF toxins on the SG2919 antibody, while SG2919-MC-MMAF&SN38 is a conjugate simultaneously linked to MMAF and SN38. Selected samples were named as follows: SG2919 antibody intermediate (i.e., naked antibody); SG2919 antibody-drug conjugates: SG2919-MC-MK4827&MMAF (abbreviated as SG2919-MC4827AF), SG2919-MC-MMAF&SN38 (abbreviated as SG2919-MC38AF), SG2919-MC-2MMAF (abbreviated as SG2919-MC2AF), SG2919-MC-MC-VC-PAB-MMAE (abbreviated as SG2919-MACE), and SG2919-PY-VC-PAB-MMAE (abbreviated as SG2919-PYAE).

The humanized antibody RB4-G1-018, designated SG2918, was conjugated with various small-molecule compounds using standard antibody-drug conjugate (ADC) coupling techniques (e.g., as disclosed in CN107921030A) to generate the following ADCs: SG2918-MC-MK4827&MMAF, SG2918-MC-MMAF&SN38, SG2918-MC-2MMAF, SG2918-MC-MC-VC-PAB-MMAE, SG2918-MC-VC-PAB-MMAE, and SG2918-PY-VC-PAB-MMAE. Among these, SG2918-MC-MK4827&MMAF is a conjugate simultaneously linked to both MK4827 and MMAF toxins on the SG2918 antibody, while SG2918-MC-MMAF&SN38 is a conjugate simultaneously linked to MMAF and SN38. Selected samples were abbreviated as follows: SG2918 antibody intermediate (i.e., naked antibody); SG2918 antibody-drug conjugates: SG2918-MC-MK4827&MMAF (abbreviated as SG2918-MC4827AF), SG2918-MC-MMAF&SN38 (abbreviated as SG2918-MC38AF), SG2918-MC-2MMAF (abbreviated as SG2918-MC2AF), SG2918-MC-MC-VC-PAB-MMAE (abbreviated as SG2918-MACE), and SG2918-PY-VC-PAB-MMAE (abbreviated as SG2918-PYAE).

### Example 13 Cytotoxicity of SG2919-ADC conjugated with different toxins against THP-1 cells (AML model)

THP-1 cells in logarithmic growth phase were harvested and seeded into clear 96-well plates (Nunc^{™} Edge^{™} 96-well, Thermo) at 1.5×10⁴ cells/well. SG2919-ADCs conjugated with different toxins at varying concentrations were added to the cells. The plates were incubated at 37°C in 5% CO₂ for 3 days. Then, CCK-8 detection reagent (Cell Counting Kit-8, Dojindo) was added to each well to a final concentration of 10% (v/v), followed by incubation at 37°C for 1-2 hours. Absorbance was measured at 450 nm/600 nm using a multifunctional microplate reader. The cytotoxic activity of SG2919-ADCs conjugated with different toxins against THP-1 cells was analyzed.

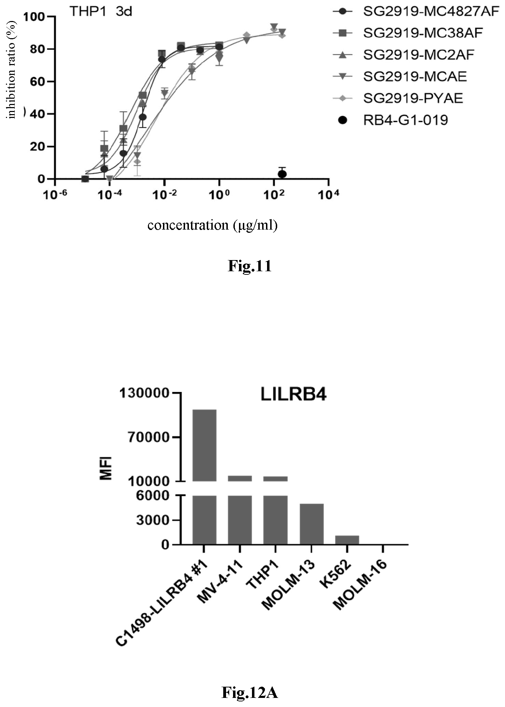

The results are shown in Figure 11. Figure 11 shows that SG2919-ADCs conjugated with different toxins exhibited clear dose-dependent cytotoxic activity against THP-1 cells. SG2919-MC4827AF (i.e., SG2919-MC-MK4827&MMAF), SG2919-MC38AF (i.e., SG2919-MC-MMAF&SN38), and SG2919-MC2AF (i.e., SG2919-MC-2MMAF) showed the strongest cytotoxic activity against THP-1 cells; SG2919-MACE (i.e., SG2919-MC-MC-VC-PAB-MMAE) and SG2919-PYAE (i.e., SG2919-PY-VC-PAB-MMAE) also exhibited strong activity. The unconjugated naked antibody RB4-G1-019 showed no cytotoxic activity.

### Example 14 Cytotoxicity of SG2918-ADC conjugated with different toxins against AML cells with varying LILRB4 expression levels

THP-1, MOLM-13, and MOLM-16 cells in logarithmic growth phase were harvested and seeded into clear 96-well plates (Nunc^{™} Edge^{™} 96-well, Thermo) at 1.5×10⁴ cells/well. SG2918-ADCs conjugated with different toxins at varying concentrations were added to the cells. The plates were incubated at 37°C in 5% CO₂ for 3 days. Then, CCK-8 detection reagent (Cell Counting Kit-8, Dojindo) was added to each well to a final concentration of 10% (v/v), followed by incubation at 37°C for 1-2 hours. Absorbance was measured at 450 nm/600 nm using a multifunctional microplate reader. The cytotoxic activity of SG2918-ADCs conjugated with different toxins was analyzed against AML cells with varying LILRB4 expression levels (THP-1 > MOLM-13 > MOLM-16, Figure 12A).

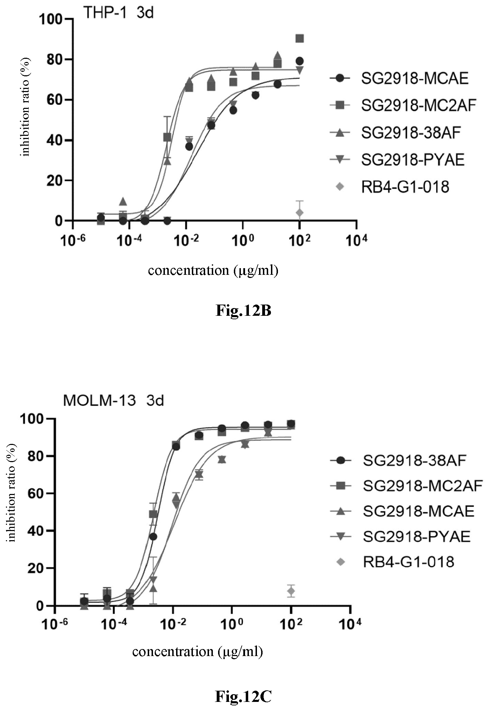

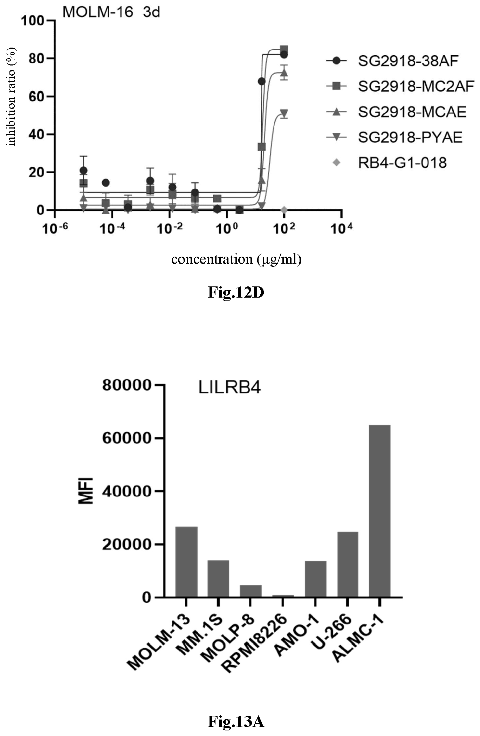

The results are shown in Figures 12B-12D. Figures 12B, 12C, and 12D show that SG2918-ADCs conjugated with different toxins exhibited clear dose-dependent cytotoxic activity against LILRB4-expressing THP-1 and MOLM-13 cells. SG2918-MC2AF (SG2918-MC-2MMAF) and SG2918-38AF (SG2918-MC-MMAF&SN38) showed the strongest and comparable cytotoxic activity; SG2918-MCAE (SG2918-MC-VC-PAB-MMAE) and SG2918-PYAE (SG2918-PY-VC-PAB-MMAE) also exhibited strong and comparable activity. In contrast, on LILRB4-negative MOLM-16 cells, SG2918-ADCs conjugated with different toxins only showed cytotoxic activity at higher concentrations (>10 µg/mL), with effective concentrations significantly higher than those observed for THP-1 and MOLM-13 cells. The unconjugated naked antibody RB4-G1-018 showed no cytotoxic activity on any of the three cell lines.

### Example 15 Cytotoxicity of SG2918-PYAE against multiple myeloma (MM) cells with varying LILRB4 expression levels

U-266, MM.1S, and ALMC-1 cells in logarithmic growth phase were harvested and seeded into clear 96-well plates (Nunc^{™} Edge^{™} 96-well, Thermo) at 1.5×10⁴ cells/well. SG2918-PYAE at varying concentrations was added to the cells. The plates were incubated at 37°C in 5% CO₂ for 5 days. Then, CCK-8 detection reagent (Cell Counting Kit-8, Dojindo) was added to each well to a final concentration of 10% (v/v), followed by incubation at 37°C for 1-2 hours. Absorbance was measured at 450 nm/600 nm using a multifunctional microplate reader. The cytotoxic activity of SG2918-PYAE was analyzed against MM cells with varying LILRB4 expression levels (ALMC-1 > U-266 > MM.1S, Figure 13A).

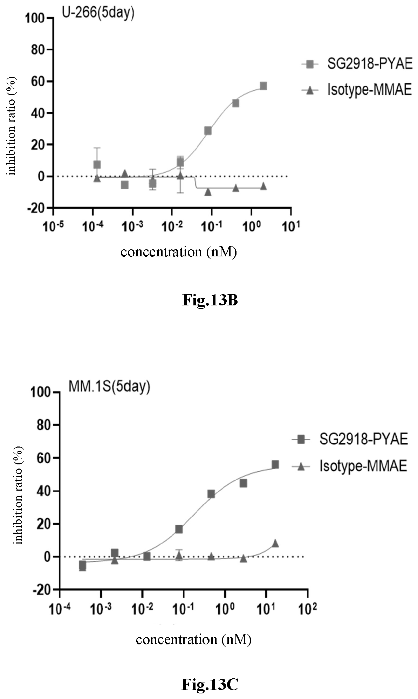

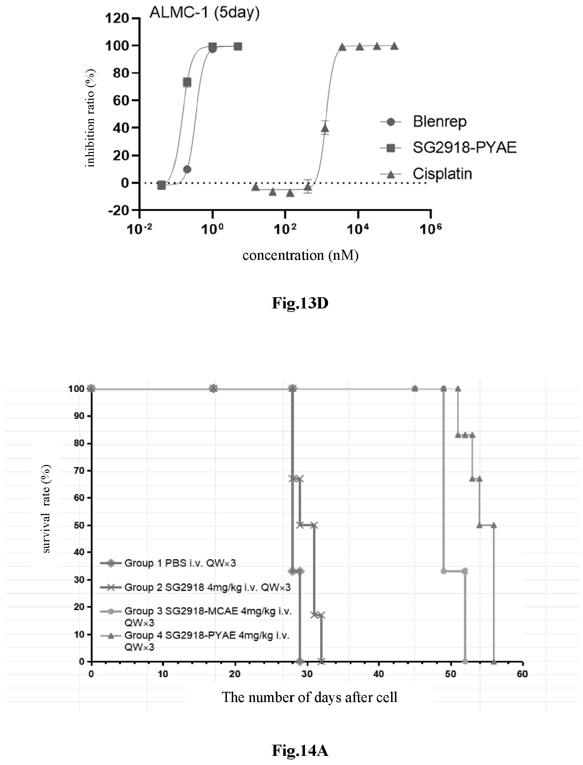

As shown in Figures 13B, 13C, and 13D, SG2918-PYAE exhibited clear dose-dependent cytotoxic activity against LILRB4-expressing U-266, MM.1S, and ALMC-1 cells. On ALMC-1 cells, SG2918-PYAE demonstrated stronger cytotoxic activity than Blenrep, an approved BCMA-targeting ADC drug.

### Example 16 In vivo efficacy of SG2918-ADC conjugated with different toxins (THP-1 model)

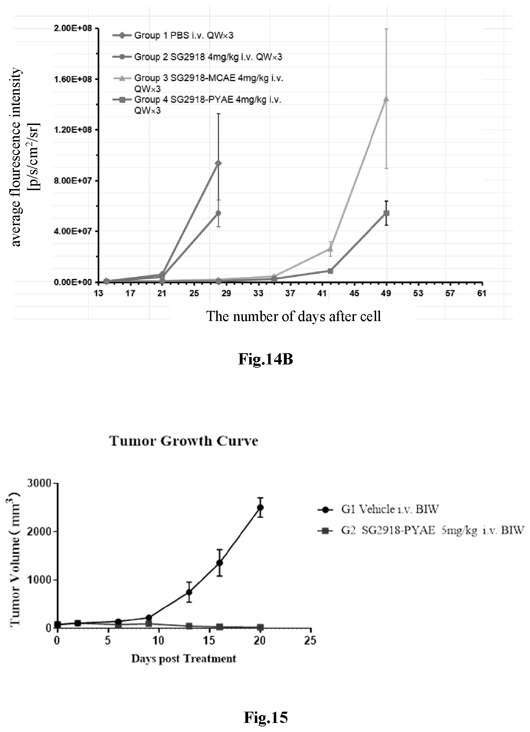

Luciferase-expressing human monocytic leukemia cells THP-1-Luc were injected intravenously via the tail vein into female NCG mice at 1×10⁷ cells/mouse. Fourteen days post-inoculation, mice were randomized into four groups (n=6 per group) based on in vivo imaging signals: PBS (iv, QW×3) control group, SG2918 (4 mg/kg, iv, QW×3) group, SG2918-MCAE (4 mg/kg, iv, QW×3) group, and SG2918-PYAE (4 mg/kg, iv, QW×3) group. Mouse body weights were measured weekly, and tumor bioluminescence signals were monitored using an in vivo imaging system to calculate bioluminescence values and T/C (%) for tumor-bearing mice in each group. Mouse survival times were recorded to calculate median survival and survival extension rates for each group, followed by statistical analysis. The results are shown in Figures 14A and 14B.

The SG2918-MCAE and SG2918-PYAE treatment groups showed significant early inhibition of tumor growth. On PG-D7 (i.e., day 21 post-tumor inoculation), the bioluminescence signal (p/s/cm²/sr) in the PBS control group was 5.72×10⁶ ± 8.80×10⁵; those in the SG2918, SG2918-MCAE, and SG2918-PYAE groups were 3.89×10⁶ ± 4.61×10⁵, 8.24×10⁵ ± 1.57×10⁵, and 4.50×10⁵ ± 8.64×10⁴, respectively. The relative tumor proliferation T/C (%) values for the treatment groups versus the PBS control group were 68%, 14%, and 8%, respectively. The bioluminescence signal intensities in the SG2918-MCAE and SG2918-PYAE groups were significantly lower than those in the PBS control group (p<0.05). Compared to the SG2918 group, both SG2918-MCAE and SG2918-PYAE groups showed significantly lower bioluminescence signals (p<0.05), whereas no significant difference was observed between the SG2918-MCAE and SG2918-PYAE groups (p>0.05).

By the end of the observation period (PG-D42), all mice in the PBS control, SG2918, SG2918-MCAE, and SG2918-PYAE groups had succumbed to disease, with median survival times of 14, 15, 35, and 40 days, respectively. The survival extension rates for the treatment groups were 7%, 150%, and 186%, respectively. Compared to the PBS control and SG2918 groups, the survival times in the SG2918-MCAE and SG2918-PYAE groups were significantly prolonged (p<0.05). Moreover, the SG2918-PYAE group exhibited significantly longer survival than the SG2918-MCAE group (p<0.05).

### Example 17 In vivo efficacy of SG2918-PYAE in multiple myeloma (OPM-2 model)

Human myeloma cells OPM-2 were implanted subcutaneously into the right flank of female NCG immunodeficient mice. When the average tumor volume reached 79 mm³, suitable mice were selected based on tumor volume and body weight and randomly assigned into two groups (n=6 per group): G1 (vehicle) and G2 (SG2918-PYAE). SG2918-PYAE was administered intravenously at 5 mg/kg twice weekly for a total of four doses. Mouse body weights were measured twice weekly during the dosing and observation period, and the experiment concluded on day 20 post-grouping. As shown in Figure 15, at the end of the experiment, the average tumor volume in the vehicle control group was 2503.3 ± 199.9 mm³, whereas that in the SG2918-PYAE group was 5.1 ± 0.7 mm³, yielding a tumor growth inhibition rate (TGI%) of 102% (*p*<0.01), demonstrating highly significant antitumor efficacy.

Throughout the experimental period, body weights in the SG2918-PYAE group steadily increased without any abnormalities, indicating good tolerability of the test article in this animal tumor model.

### Example 18 In vivo efficacy of SG2918-PYAE in solid tumors (LLC1 model)

LLC1 cells resuspended in PBS at 2×10⁵ cells/0.1 mL were implanted subcutaneously into the flanks of 18 B-Tg(hLILRB1-hLILRB4) mice at 0.1 mL/mouse. When tumor volumes reached approximately 80 mm³, 12 suitable animals were selected based on tumor volume and body weight and randomly assigned into two groups (n=6 per group): G1 (PBS) and G2 (SG2918-PYAE, i.e., SG2918-Py-VC-PAB-MMAE). All groups received intravenous administration via the tail vein twice weekly for a total of three doses. The experiment concluded on day 9 post-grouping. Mouse body weights and tumor volumes were measured twice weekly during the dosing and observation periods, and tumor volume growth inhibition (TGITV) was calculated. At the end of the experiment, tumors and spleens from animals in groups G1 and G2 were collected for FACS analysis.

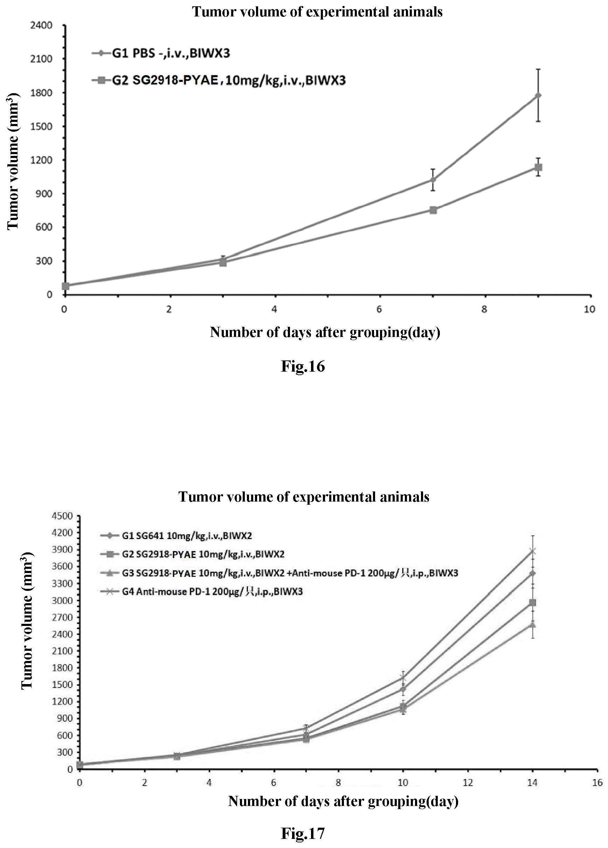

As shown in Figure 16, no obvious clinical symptoms were observed in mice across all groups during the experiment. Similar to the G1 control group, body weights in the G2 test article group gradually increased, indicating good tolerability of the test article in the animals. On day 9 after grouping, the mean tumor volume in the control group G1 was 1776 ± 232 mm³, and that in group G2 was 1137 ± 78 mm³. The corresponding TGITV is 37.7%.

### Example 19 In Vivo Efficacy of SG2918-PYAE in Combination with PD-1 Antibody for the Treatment of Solid Tumors

LLC1 cells resuspended in PBS were inoculated subcutaneously on the right flank of 34 B-Tg(hLILRB1-hLILRB4) mice at a volume of 5 × 10⁵ cells/0.1 mL/mouse. When the mean tumor volume reached 79 mm³, mice were selected based on tumor volume and body weight and randomly assigned to four experimental groups (n = 6 per group): G1: SG641 (negative control); G2: SG2918-PYAE (administered SG2918-Py-VC-PAB-MMAE); G3: SG2918-PYAE + Anti-mouse PD-1; and G4: Anti-mouse PD-1. Dose, route, frequency, and number of administrations for each group were performed as described in Section 6.2. The experiment was terminated on day 17 after grouping. Mouse body weight and tumor volume were measured twice weekly during the dosing and observation period. At the end of the experiment, mice were euthanized, tumors were excised, weighed, photographed, and tumor growth inhibition rate was calculated.

No obvious clinical signs were observed in any group during the experiment. Similar to the G1 control group, body weight gradually increased in the G2 test article group, indicating good tolerability of the test article in animals. On day 14 after grouping, the mean tumor volume in group G4 was 3868 ± 284 mm³, while those in groups G1-G3 were 3474 ± 257 mm³, 2961 ± 326 mm³, and 2573 ± 243 mm³, respectively, with corresponding tumor growth inhibition rates (TGITV) of 10.4%, 23.9%, and 34.2%, respectively. The results are shown in Figure 17. Under the experimental conditions, administration of SG2918-PYAE in combination with Anti-mouse PD-1 showed significantly greater inhibition of tumor growth in LLC1 cell-implanted B-Tg(hLILRB1-hLILRB4) mice compared to Anti-mouse PD-1 alone (p < 0.05). Animals exhibited good tolerability to the test article.

## Claims

1. An immunoconjugate comprising an antigen-binding protein targeting LILRB4 and a drug molecule, wherein said immunoconjugate has the following general formula:
Ab-(Q)ₙ (Formula 1)
wherein Ab represents said antigen-binding protein targeting LILRB4, Q represents a moiety connected to Ab and comprising said drug molecule, n is an integer from 1 to 10, and each Q is the same or different;
wherein said antigen-binding protein targeting LILRB4 possesses one or more of the following properties:
(1) capable of specifically recognizing leukocyte immunoglobulin-like receptor B4 (LILRB4);
(2) capable of binding to LILRB4 protein with a KD value of 2×10⁻⁷ M or lower;
(3) capable of blocking the activity of LILRB4/APOE binding;
(4) capable of stimulating activation of CD8⁺ T cells;
(5) capable of inhibiting migration of human monocytic leukemia cells THP-1;
(6) capable of inhibiting tumor cell growth and/or metastasis;
(7) capable of internalization activity upon binding to LILRB4 target; and
(8) capable of blocking the binding of LILRB4 to Fibronectin.

2. The immunoconjugate of claim 1, wherein said LILRB4 is human LILRB4.

3. The immunoconjugate of any one of claims 1-2, wherein said antigen-binding protein targeting LILRB4 comprises at least one CDR in the antibody heavy chain variable region VH, and said VH comprises an amino acid sequence as shown in any one of SEQ ID NO: 81, SEQ ID NO: 33, and SEQ ID NO: 22.

4. The immunoconjugate of any one of claims 1-3, wherein said antigen-binding protein targeting LILRB4 comprises HCDR1, HCDR2, and HCDR3, wherein said HCDR1 comprises an amino acid sequence as shown in SEQ ID NO: 69 (X₁X₂WMX₃, wherein X₁ is D or S; X₂ is A or Y; X₃ is D or H), said HCDR2 comprises an amino acid sequence as shown in SEQ ID NO: 2 or SEQ ID NO: 28, and said HCDR3 comprises an amino acid sequence shown in SEQ ID NO: 83 or SEQ ID NO: 29.

5. The immunoconjugate of any one of claims 1-4, wherein said antigen-binding protein targeting LILRB4 comprises HCDR1, HCDR2, and HCDR3, wherein said HCDR1 comprises an amino acid sequence as shown in SEQ ID NO: 1 or SEQ ID NO: 27, said HCDR2 comprises an amino acid sequence as shown in SEQ ID NO: 2 or SEQ ID NO: 28, and said HCDR3 comprises an amino acid sequence as shown in any one of SEQ ID NO: 3, SEQ ID NO: 20, and SEQ ID NO: 29.

6. The immunoconjugate of any one of claims 1-5, wherein said antigen-binding protein targeting LILRB4 comprises HCDR1, HCDR2, and HCDR3, and said HCDR1, HCDR2, and HCDR3 comprise amino acid sequences selected from the group consisting of:
(1) said HCDR1 comprises an amino acid sequence as shown in SEQ ID NO: 1, said HCDR2 comprises an amino acid sequence as shown in SEQ ID NO: 2, and said HCDR3 comprises an amino acid sequence as shown in SEQ ID NO: 3;
(2) said HCDR1 comprises an amino acid sequence as shown in SEQ ID NO: 1, said HCDR2 comprises an amino acid sequence as shown in SEQ ID NO: 2, and said HCDR3 comprises an amino acid sequence as shown in SEQ ID NO: 20; and
(3) said HCDR1 comprises an amino acid sequence as shown in SEQ ID NO: 27, said HCDR2 comprises an amino acid sequence as shown in SEQ ID NO: 28, and said HCDR3 comprises an amino acid sequence as shown in SEQ ID NO: 29.

7. The immunoconjugate of any one of claims 1-6, wherein said antigen-binding protein targeting LILRB4 comprises VH, and said VH comprises an amino acid sequence as shown in any one of SEQ ID NO: 81, SEQ ID NO: 33, and SEQ ID NO: 22.

8. The immunoconjugate of any one of claims 1-7, wherein said antigen-binding protein targeting LILRB4 comprises VH, and said VH comprises an amino acid sequence as shown in any one of SEQ ID NO: 8, SEQ ID NO: 19, SEQ ID NO: 22, SEQ ID NO: 33, SEQ ID NO: 45, SEQ ID NO: 55, and SEQ ID NO: 58.

9. The immunoconjugate of any one of claims 1-8, wherein said antigen-binding protein targeting LILRB4 comprises at least one CDR in the antibody light chain variable region VL, and said VL comprises an amino acid sequence as shown in SEQ ID NO: 82 or SEQ ID NO: 40.

10. The immunoconjugate of any one of claims 1-9, wherein said antigen-binding protein targeting LILRB4 comprises LCDR1, LCDR2, and LCDR3, wherein said LCDR1 comprises an amino acid sequence as shown in SEQ ID NO: 70 or SEQ ID NO: 34, said LCDR2 comprises an amino acid sequence as shown in SEQ ID NO: 71, and said LCDR3 comprises an amino acid sequence as shown in SEQ ID NO: 72 (QX₁X₂X₃X₄X₅PX₆T, wherein X₁ is H or Q; X₂ is G or S; X₃ is D, N, or W; X₄ is E or T; X₅ is I or L; X₆ is P or R).

11. The immunoconjugate of any one of claims 1-10, wherein said antigen-binding protein targeting LILRB4 comprises LCDR1, LCDR2, and LCDR3, wherein said LCDR1 comprises an amino acid sequence as shown in any one of SEQ ID NO: 9, SEQ ID NO: 23, and SEQ ID NO: 34, said LCDR2 comprises an amino acid sequence as shown in SEQ ID NO: 10 or SEQ ID NO: 35, and said LCDR3 comprises an amino acid sequence as shown in any one of SEQ ID NO: 11, SEQ ID NO: 24, and SEQ ID NO: 36.

12. The immunoconjugate of any one of claims 1-11, wherein said antigen-binding protein targeting LILRB4 comprises LCDR1, LCDR2, and LCDR3, and said LCDR1, LCDR2, and LCDR3 comprise amino acid sequences selected from the group consisting of:
(1) said LCDR1 comprises an amino acid sequence as shown in SEQ ID NO: 9, said LCDR2 comprises an amino acid sequence as shown in SEQ ID NO: 10, and said LCDR3 comprises an amino acid sequence as shown in SEQ ID NO: 11;
(2) said LCDR1 comprises an amino acid sequence as shown in SEQ ID NO: 23, said LCDR2 comprises an amino acid sequence as shown in SEQ ID NO: 10, and said LCDR3 comprises an amino acid sequence as shown in SEQ ID NO: 24; and
(3) said LCDR1 comprises an amino acid sequence as shown in SEQ ID NO: 34, said LCDR2 comprises an amino acid sequence as shown in SEQ ID NO: 35, and said LCDR3 comprises an amino acid sequence as shown in SEQ ID NO: 36.

13. The immunoconjugate of any one of claims 1-12, wherein said antigen-binding protein targeting LILRB4 comprises VL, and said VL comprises an amino acid sequence as shown in SEQ ID NO: 82 or SEQ ID NO: 40.

14. The immunoconjugate of any one of claims 1-13, wherein said antigen-binding protein targeting LILRB4 comprises VL, and said VL comprises an amino acid sequence as shown in any one of SEQ ID NO: 16, SEQ ID NO: 26, SEQ ID NO: 40, SEQ ID NO: 51, and SEQ ID NO: 61.

15. The immunoconjugate of any one of claims 1-14, wherein said antigen-binding protein targeting LILRB4 comprises HCDR1, HCDR2, HCDR3, LCDR1, LCDR2, and LCDR3, and said HCDR1, HCDR2, HCDR3, LCDR1, LCDR2, and LCDR3 comprise amino acid sequences selected from the group consisting of:
(1) said HCDR1 comprises an amino acid sequence as shown in SEQ ID NO: 1, said HCDR2 comprises an amino acid sequence as shown in SEQ ID NO: 2, said HCDR3 comprises an amino acid sequence as shown in SEQ ID NO: 3, said LCDR1 comprises an amino acid sequence as shown in SEQ ID NO: 9, said LCDR2 comprises an amino acid sequence as shown in SEQ ID NO: 10, and said LCDR3 comprises an amino acid sequence as shown in SEQ ID NO: 11;
(2) said HCDR1 comprises an amino acid sequence as shown in SEQ ID NO: 1, said HCDR2 comprises an amino acid sequence as shown in SEQ ID NO: 2, said HCDR3 comprises an amino acid sequence as shown in SEQ ID NO: 20, said LCDR1 comprises an amino acid sequence as shown in SEQ ID NO: 23, said LCDR2 comprises an amino acid sequence as shown in SEQ ID NO: 10, and said LCDR3 comprises an amino acid sequence as shown in SEQ ID NO: 24;
(3) said HCDR1 comprises an amino acid sequence as shown in SEQ ID NO: 27, said HCDR2 comprises an amino acid sequence as shown in SEQ ID NO: 28, said HCDR3 comprises an amino acid sequence as shown in SEQ ID NO: 29, said LCDR1 comprises an amino acid sequence as shown in SEQ ID NO: 34, said LCDR2 comprises an amino acid sequence as shown in SEQ ID NO: 35, and said LCDR3 comprises an amino acid sequence as shown in SEQ ID NO: 36.

16. The immunoconjugate of any one of claims 1-15, wherein said antigen-binding protein targeting LILRB4 comprises VH and VL, and said VH and VL comprise amino acid sequences selected from any one of the following groups:
(1) said VH comprises an amino acid sequence as shown in SEQ ID NO: 8, and said VL comprises an amino acid sequence as shown in SEQ ID NO: 16;
(2) said VH comprises an amino acid sequence as shown in SEQ ID NO: 19, and said VL comprises an amino acid sequence as shown in SEQ ID NO: 16;
(3) said VH comprises an amino acid sequence as shown in SEQ ID NO: 22, and said VL comprises an amino acid sequence as shown in SEQ ID NO: 26;
(4) said VH comprises an amino acid sequence as shown in SEQ ID NO: 33, and said VL comprises an amino acid sequence as shown in SEQ ID NO: 40;
(5) said VH comprises an amino acid sequence as shown in SEQ ID NO: 45, and said VL comprises an amino acid sequence as shown in SEQ ID NO: 51;
(6) said VH comprises an amino acid sequence as shown in SEQ ID NO: 55, and said VL comprises an amino acid sequence as shown in SEQ ID NO: 51;
(7) said VH comprises an amino acid sequence as shown in SEQ ID NO: 58, and said VL comprises an amino acid sequence as shown in SEQ ID NO: 51; and
(8) said VH comprises an amino acid sequence as shown in SEQ ID NO: 45, and said VL comprises an amino acid sequence as shown in SEQ ID NO: 61.

17. The immunoconjugate of any one of claims 1-16, wherein said antigen-binding protein targeting LILRB4 comprises an antibody heavy chain constant region, and said antibody heavy chain constant region is derived from an IgG constant region.

18. The immunoconjugate of claim 17, wherein said antibody heavy chain constant region comprises an IgG1 constant region, an IgG4 constant region, and respective mutants thereof.

19. The immunoconjugate of any one of claims 17-18, wherein said antibody heavy chain constant region comprises an amino acid sequence as shown in SEQ ID NO: 63.

20. The immunoconjugate of any one of claims 1-19, wherein said antigen-binding protein targeting LILRB4 comprises an antibody light chain constant region, and said antibody light chain constant region is derived from a human Igκ constant region.

21. The immunoconjugate of claim 20, wherein said antibody light chain constant region comprises an amino acid sequence as shown in SEQ ID NO: 64.

22. The immunoconjugate of any one of claims 1-21, wherein said antigen-binding protein targeting LILRB4 comprises an antibody heavy chain and an antibody light chain, and said antibody heavy chain and antibody light chain comprise amino acid sequences selected from any one of the following groups:
(1) said antibody heavy chain comprises an amino acid sequence as shown in SEQ ID NO: 86, and said antibody light chain comprises an amino acid sequence as shown in SEQ ID NO: 87;
(2) said antibody heavy chain comprises an amino acid sequence as shown in SEQ ID NO: 88, and said antibody light chain comprises an amino acid sequence as shown in SEQ ID NO: 87;
(3) said antibody heavy chain comprises an amino acid sequence as shown in SEQ ID NO: 89, and said antibody light chain comprises an amino acid sequence as shown in SEQ ID NO: 90;
(4) said antibody heavy chain comprises an amino acid sequence as shown in SEQ ID NO: 91, and said antibody light chain comprises an amino acid sequence as shown in SEQ ID NO: 92;
(5) said antibody heavy chain comprises an amino acid sequence as shown in SEQ ID NO: 46, and said antibody light chain comprises an amino acid sequence as shown in SEQ ID NO: 52;
(6) said antibody heavy chain comprises an amino acid sequence as shown in SEQ ID NO: 56, and said antibody light chain comprises an amino acid sequence as shown in SEQ ID NO: 52;
(7) said antibody heavy chain comprises an amino acid sequence as shown in SEQ ID NO: 59, and said antibody light chain comprises an amino acid sequence as shown in SEQ ID NO: 52; and
(8) said antibody heavy chain comprises an amino acid sequence as shown in SEQ ID NO: 46, and said antibody light chain comprises an amino acid sequence as shown in SEQ ID NO: 62.

23. The immunoconjugate of any one of claims 1-22, wherein said drug molecule comprises a macromolecular drug and/or a small molecule drug.

24. The immunoconjugate of claim 23, wherein said macromolecular drug comprises other antibody drugs, nucleic acid drugs, molecular glues, and PROTAC; said other antibody drugs comprise antibodies binding to tumor therapy-related antigens; and said nucleic acid drugs comprise sgRNA, mRNA, siRNA, shRNA, and antisense RNA.

25. The immunoconjugate of any one of claims 1-24, wherein said drug molecule is selected from chemotherapeutic agents, radioactive elements, cell growth inhibitors, and cytotoxic agents.

26. The immunoconjugate of claim 25, wherein said radioactive elements comprise one or more of ¹⁷⁷Lu, ⁶⁸Ga, ⁸⁹Zr, ²²⁵Ac, ¹¹¹In, ⁹⁰Y, ²¹³Bi, ¹³¹I, ¹²⁵I, ⁹⁹mTc, ³H, ¹⁴C, ¹⁵N, ³⁵S, ¹⁸F, and ⁶⁴Cu.

27. The immunoconjugate of any one of claims 1-26, wherein said drug molecule is selected from the group consisting of: V-ATPase inhibitor, Bcl2 inhibitor, MCL1 inhibitor, HSP90 inhibitor, IAP inhibitor, mTOR inhibitor, microtubule stabilizer, microtubule destabilizer, auristatin, dolastatin, maytansinoid, MetAP (methionine aminopeptidase), nuclear export inhibitor of protein CRM1, DPPIV inhibitor, proteasome inhibitor, mitochondrial phosphate transfer reaction inhibitor, protein synthesis inhibitor, CDK2 inhibitor, CDK9 inhibitor, kinesin inhibitor, HDAC inhibitor, DNA damaging agent, DNA alkylating agent, DNA intercalating agent, DNA minor groove binder, DHFR inhibitor, nucleoside analog, HDAC inhibitor, anthracycline, NAMPT inhibitor, SN-38 or its derivatives, etoposide phosphate, nitrogen mustard, proteasome inhibitor, cytokine, Tubulysin B analog, and Toll-like receptor agonist.

28. The immunoconjugate of any one of claims 1-27, wherein said drug molecule is selected from antibiotics, microtubule disrupting agents, DNA damaging drugs, apoptosis inducers, telomestatin and its analogs, amatoxin, nicotinamide phosphoribosyltransferase, and calicheamicin.

29. The immunoconjugate of claim 28, wherein said microtubule disrupting agents is selected from: auristatins, maytansine derivatives, tubulysins, cryptophycins, and antimitotic EG5 inhibitors; wherein preferably, said auristatins comprise MMAE and/or MMAF, and said maytansine derivatives comprises DM1, DM2, DM3, and/or DM4.

30. The immunoconjugate of any one of claims 28-29, wherein said DNA damaging drugs are selected from: pyrrolobenzodiazepine drugs, indolinobenzodiazepine drugs, duocarmycins, camptothecin and its derivatives, and calicheamicin; wherein preferably, said pyrrolobenzodiazepine drugs comprise pyrrolo[2,1-c][1,4]benzodiazepine (PBD), said camptothecin and its derivatives comprise SN-38 and/or DXd/DX8951, and said apoptosis inhibitors comprise a Bcl-xL inhibitor.

31. The immunoconjugate of any one of claims 1-30, further comprising a linker, wherein said linker is capable of connecting said antigen-binding protein targeting LILRB4 and said drug molecule.

32. The immunoconjugate of claim 31, wherein said linker is selected from non-cleavable linker and cleavable linker; preferably, said cleavable linker comprises chemically reactive linker, acid-cleavable linker, reduction-cleavable linker, and/or enzyme-cleavable linker.

33. The immunoconjugate of any one of claims 31-32, wherein said linker is connected to said LILRB4 antigen-binding protein via a thiol group, azide group, or amide group on said antigen-binding protein targeting LILRB4.

34. The immunoconjugate of any one of claims 31-33, wherein said linker comprises one or more combinations selected from the group consisting of: Acetyl Butyrate, MC-Val-Cit-PABC, SMCC, maleimide, CL2A, MC, MC-VC-PAB, PY-VC-PAB, PEG8-VA-PABC, MC-GGFG, MC-Val-Ala-PAB, MC-Val-Lys(Ac)-PAB, MC-Phe-Lys-PAB, D-Val-Leu-Lys, Gly-Gly-Arg, Ala-Ala-Asn-PAB, Ala-PAB, disulfide bond, and acid-labile hydrazone bond.

35. The immunoconjugate of any one of claims 1-34, comprising a structure represented by Formula 2:
Ab-(L1)ₐ-(L2)_{b}-(D)ₙ (Formula 2),
wherein Ab represents an antigen-binding protein targeting LILRB4;
L1 represents a linker connected to Ab, and L2 represents a linker connected to D,
a and b are each independently selected from 0-10,
D represents a drug molecule,
n is selected from 1-10,
wherein preferably, L1 is a group or molecule capable of reacting with a thiol group, azide group, or amide group, preferably Py-MAA;
L2 comprises one or more combinations selected from the group consisting of: C1-C9 alkyl, C2-C9 alkenyl, C2-C9 alkynyl, C6-C24 aryl, C6-C15 heteroaryl, C3-C9 cycloalkyl, C3-C9 heterocyclyl, polyethylene glycol, O, S, alkylamino, carbonyl, carboxyl, sulfonic acid group, Val-Cit-PAB, VA-PAB, GGFG, Val-Lys(Ac)-PAB, Phe-Lys-PAB, D-Val-Leu-Lys, Gly-Gly-Arg, Ala-Ala-Asn-PAB, and Ala-PAB.

36. The immunoconjugate of any one of claims 1-35, wherein Q in said Formula 1 is selected from one or more combinations of the following groups: 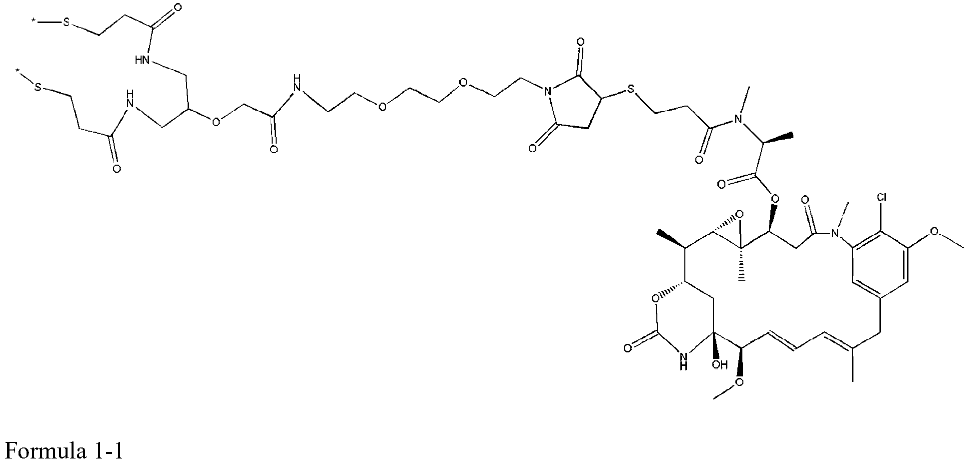 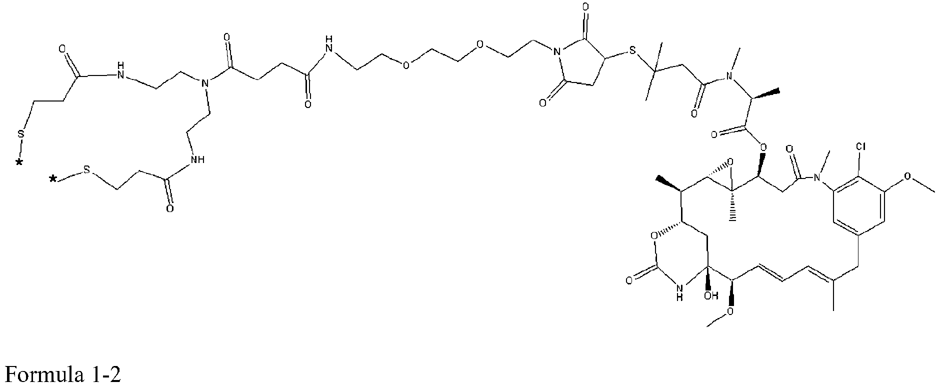 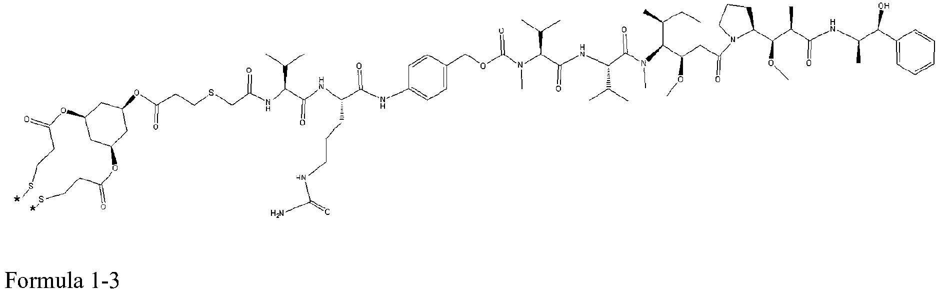 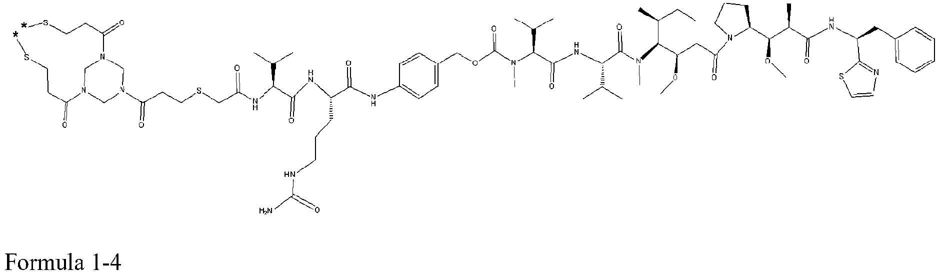 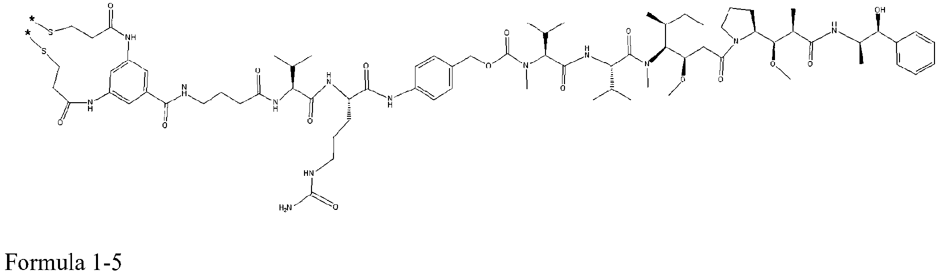 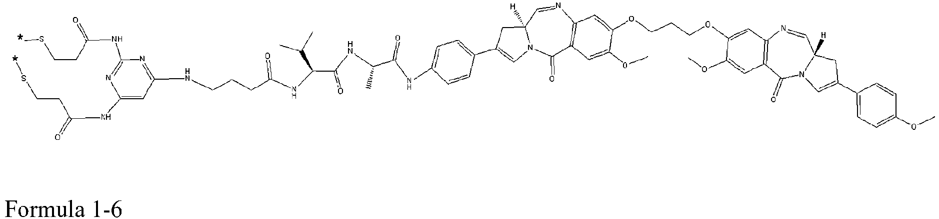 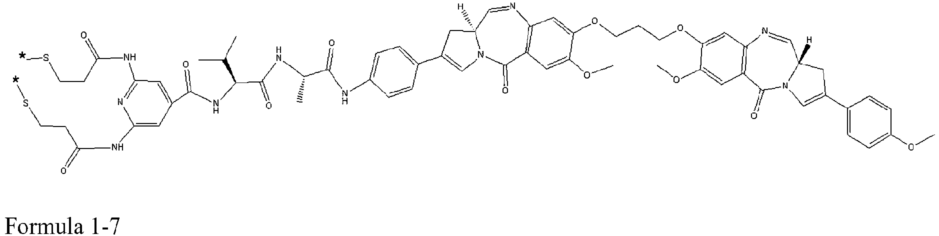 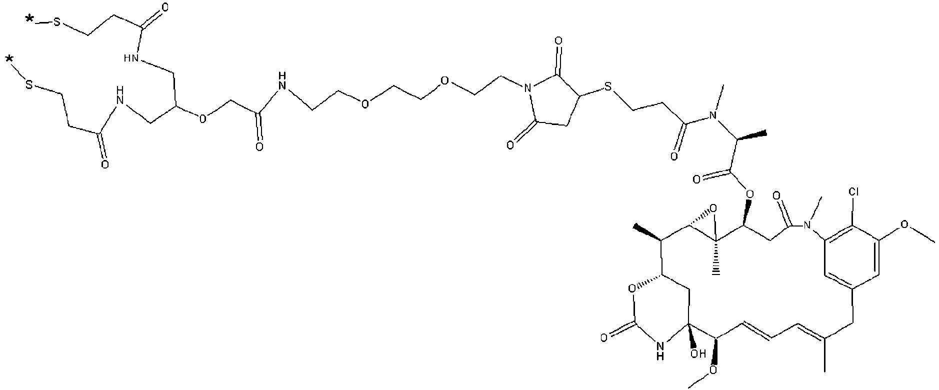 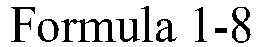 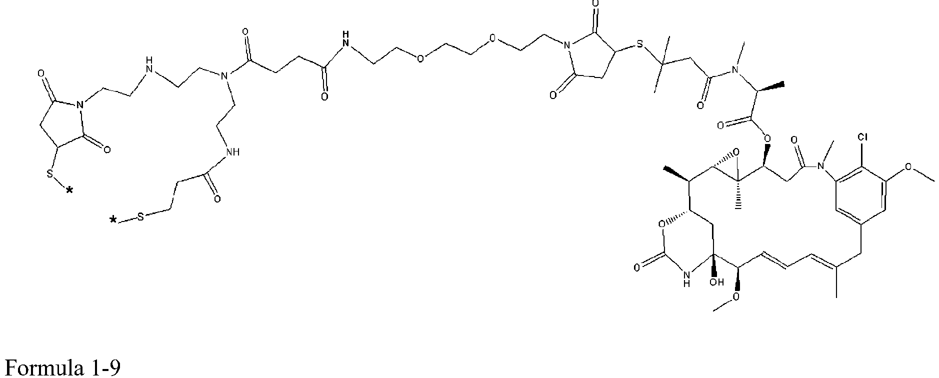 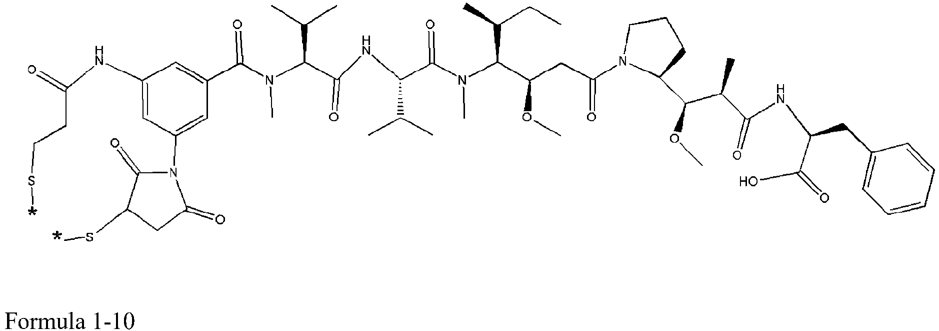 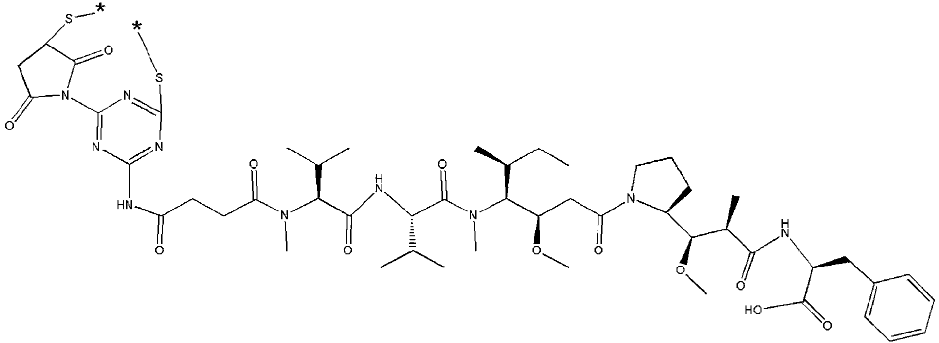 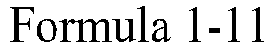 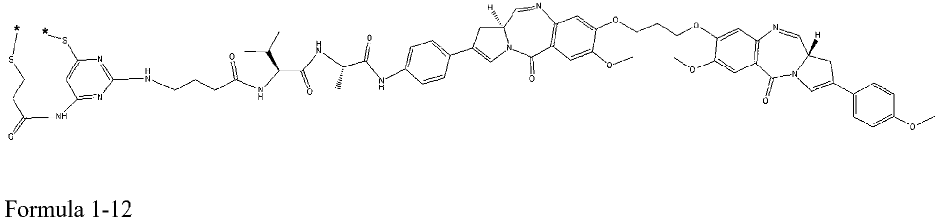 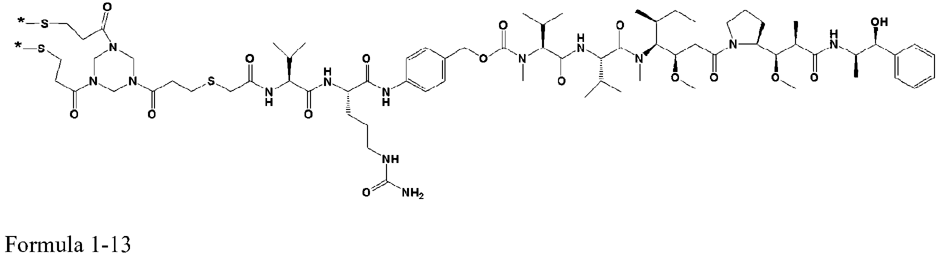 wherein * is the attachment site of Q to the antibody molecule.

37. The immunoconjugate of any one of claims 1-36, wherein said Q in Formula 1 is selected from the group consisting of: -MC-MK4827, -MC-MK4827&MMAF, -MC-MMAF, -MC-MMAF, -MC-MMAF&SN38, -MC-SN38, -MC-2MMAF, -MC-MC-VC-PAB-MMAE, and -PY-VC-PAB-MMAE.

38. The immunoconjugate of any one of claims 1-37, having the following molecular formulas: 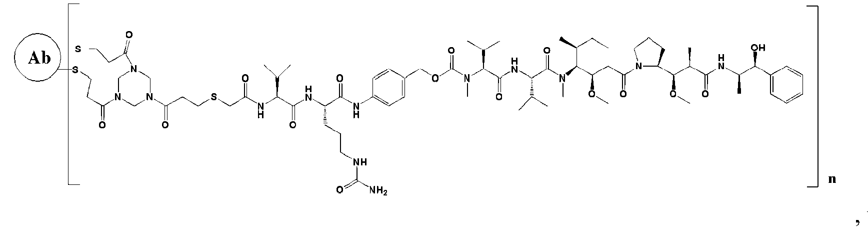 wherein n is selected from 1-8.

39. A pharmaceutical composition comprising the immunoconjugate of any one of claims 1-38, and optionally a pharmaceutically acceptable carrier.

40. A pharmaceutical combination comprising the immunoconjugate of any one of claims 1-38, and an immune checkpoint inhibitor; preferably, said immune checkpoint comprises PD-1, PD-L1, GITR, and/or CTLA-4; more preferably, said immune checkpoint inhibitor comprises one or more selected from the group consisting of: PD-1 antibody, PD-L1 antibody, CTLA-4 antibody, and GITR antibody.

41. A method for preparing the immunoconjugate of any one of claims 1-38, comprising connecting said antigen-binding protein targeting LILRB4 of any one of claims 1-38 and said drug molecule of any one of claims 1-38 under suitable conditions.

42. A method for preventing, diagnosing, and/or treating diseases and/or disorders, comprising administering to a subject in need thereof the immunoconjugate of any one of claims 1-38, the pharmaceutical composition of claim 39, or the pharmaceutical combination of claim 40.

43. The method of claim 42, wherein said diseases and/or disorders comprise disease or disorders associated with LILRB4 signal transduction and/or LILRB4 expression.

44. The method of any one of claims 42-43, wherein said diseases and/or disorders comprise tumors preferably comprising hematological tumors and/or solid tumors.

45. The method of claim 44, wherein said solid tumors are selected from one or more of the following: breast cancer, melanoma, colon cancer, lung cancer, renal cancer, head and neck cancer, gastric cancer, pancreatic cancer, lymphoma, non-small cell lung cancer, leukemia, ovarian cancer, nasopharyngeal carcinoma, breast cancer, endometrial cancer, colon cancer, rectal cancer, gastric cancer, bladder cancer, lung cancer, bronchial cancer, bone cancer, prostate cancer, pancreatic cancer, liver and bile duct cancer, esophageal cancer, renal cancer, thyroid cancer, head and neck cancer, testicular cancer, glioblastoma, astrocytoma, melanoma, myelodysplastic syndrome, and sarcoma.

46. The method of claim 44, wherein said hematological tumors are selected from multiple myeloma, leukemia, lymphoma, myelodysplastic syndrome, myelodysplastic syndrome, and myeloproliferative neoplasm.

47. The method of claim 46, wherein said leukemia is selected from acute lymphoblastic (lymphoblastic) leukemia, acute myeloid leukemia (AML), myeloid leukemia, chronic lymphocytic leukemia, multiple myeloma, plasma cell leukemia, chronic myeloid leukemia, acute promyelocytic leukemia (APL) or M3 AML, acute myelomonocytic leukemia or M4 AML, and acute monocytic leukemia or M5 AML; wherein preferably said acute myeloid leukemia is M4 or M5 type acute myeloid leukemia; and preferably said chronic myeloid leukemia is chronic myelomonocytic leukemia; and said lymphoma is selected from Hodgkin lymphoma and non-Hodgkin lymphoma, including B-cell lymphoma, diffuse large B-cell lymphoma, follicular lymphoma, mantle cell lymphoma, marginal zone B-cell lymphoma, T-cell lymphoma, and Waldenström macroglobulinemia.

48. The method of claim 45, wherein said sarcoma is selected from osteosarcoma, Ewing sarcoma, leiomyosarcoma, synovial sarcoma, soft tissue sarcoma, angiosarcoma, liposarcoma, fibrosarcoma, rhabdomyosarcoma, and chondrosarcoma.

49. Use of the immunoconjugate of any one of claims 1-38, the pharmaceutical composition of claim 39, or the pharmaceutical combination of claim 40 in the preparation of a medicament for preventing, diagnosing, and/or treating diseases and/or disorders.

50. The use of claim 49, wherein said diseases and/or disorders comprise tumors, preferably selected from hematological tumors and/or solid tumors.

51. The use of claim 50, wherein said solid tumors are selected from one or more of the following: breast cancer, melanoma, colon cancer, lung cancer, renal cancer, head and neck cancer, gastric cancer, pancreatic cancer, lymphoma, non-small cell lung cancer, leukemia, ovarian cancer, nasopharyngeal carcinoma, breast cancer, endometrial cancer, colon cancer, rectal cancer, gastric cancer, bladder cancer, lung cancer, bronchial cancer, bone cancer, prostate cancer, pancreatic cancer, liver and bile duct cancer, esophageal cancer, renal cancer, thyroid cancer, head and neck cancer, testicular cancer, glioblastoma, astrocytoma, melanoma, myelodysplastic syndrome, and sarcoma; preferably, said sarcoma is selected from osteosarcoma, Ewing sarcoma, leiomyosarcoma, synovial sarcoma, soft tissue sarcoma, angiosarcoma, liposarcoma, fibrosarcoma, rhabdomyosarcoma, and chondrosarcoma.

52. The use of claim 49, wherein said diseases and/or disorders comprise diseases or disorders associated with LILRB4 signal transduction and/or LILRB4 expression.

53. The use of claim 50, wherein said hematological tumors are selected from multiple myeloma, leukemia, lymphoma, myelodysplastic syndrome, myelodysplastic syndrome, and myeloproliferative neoplasm.

54. The use of claim 53, wherein said leukemia is selected from acute lymphoblastic (lymphoblastic) leukemia, acute myeloid leukemia (AML), myeloid leukemia, chronic lymphocytic leukemia, multiple myeloma, plasma cell leukemia, and chronic myeloid leukemia, acute promyelocytic leukemia (APL) or M3 AML, acute myelomonocytic leukemia or M4 AML, acute monocytic leukemia or M5 AML, wherein preferably said acute myeloid leukemia is M4 or M5 type acute myeloid leukemia; preferably, said chronic myeloid leukemia is chronic myelomonocytic leukemia.

55. The use of claim 50, wherein said lymphoma is selected from Hodgkin lymphoma and non-Hodgkin lymphoma, including B-cell lymphoma, diffuse large B-cell lymphoma, follicular lymphoma, mantle cell lymphoma, marginal zone B-cell lymphoma, T-cell lymphoma, and Waldenström macroglobulinemia.

56. The immunoconjugate of any one of claims 1-38, the pharmaceutical composition of claim 39, or the pharmaceutical combination of claim 40 for use in preventing, diagnosing, and/or treating diseases and/or disorders.

57. The immunoconjugate, pharmaceutical composition, or pharmaceutical combination of claim 56, wherein said diseases and/or disorders comprise diseases or disorders associated with LILRB4 signal transduction and/or LILRB4 expression.

58. The immunoconjugate, pharmaceutical composition, or pharmaceutical combination of claim 56, wherein said diseases and/or disorders comprise tumors, preferably selected from hematological tumors and/or solid tumors.

59. The immunoconjugate, pharmaceutical composition, or pharmaceutical combination of claim 58, wherein said solid tumors are selected from one or more of the following: breast cancer, melanoma, colon cancer, lung cancer, renal cancer, head and neck cancer, gastric cancer, pancreatic cancer, lymphoma, non-small cell lung cancer, leukemia, ovarian cancer, nasopharyngeal carcinoma, breast cancer, endometrial cancer, colon cancer, rectal cancer, gastric cancer, bladder cancer, lung cancer, bronchial cancer, bone cancer, prostate cancer, pancreatic cancer, liver and bile duct cancer, esophageal cancer, renal cancer, thyroid cancer, head and neck cancer, testicular cancer, glioblastoma, astrocytoma, melanoma, myelodysplastic syndrome, and sarcoma; and said hematological tumorsare selected from multiple myeloma, leukemia, lymphoma, myelodysplastic syndrome, myelodysplastic syndrome, and myeloproliferative neoplasm.

60. The immunoconjugate, pharmaceutical composition, or pharmaceutical combination of claim 59, wherein said leukemia is selected from acute lymphoblastic (lymphoblastic) leukemia, acute myeloid leukemia (AML), myeloid leukemia, chronic lymphocytic leukemia, multiple myeloma, plasma cell leukemia, and chronic myeloid leukemia, acute promyelocytic leukemia (APL) or M3 AML, acute myelomonocytic leukemia or M4 AML, acute monocytic leukemia or M5 AML, wherein preferably said acute myeloid leukemia is M4 or M5 type acute myeloid leukemia; preferably, said chronic myeloid leukemia is chronic myelomonocytic leukemia.

61. The immunoconjugate, pharmaceutical composition, or pharmaceutical combination of claim 59, wherein said lymphoma is selected from Hodgkin lymphoma and non-Hodgkin lymphoma, including B-cell lymphoma, diffuse large B-cell lymphoma, follicular lymphoma, mantle cell lymphoma, marginal zone B-cell lymphoma, T-cell lymphoma, and Waldenström macroglobulinemia.

62. The immunoconjugate, pharmaceutical composition, or pharmaceutical combination of claim 59, wherein said sarcoma is selected from osteosarcoma, Ewing sarcoma, leiomyosarcoma, synovial sarcoma, soft tissue sarcoma, angiosarcoma, liposarcoma, fibrosarcoma, rhabdomyosarcoma, and chondrosarcoma.
